# EUROPEAN PATENT SPECIFICATION

(11) **EP 3 077 419 B1**
(45) Date of publication and mention of the grant of the patent: **14.08.2019**
(21) Application number: 14868266.9
(22) Date of filing: 04.12.2014
(51) Int. Cl.: C07K 14/575, C07K 14/605, C12N 15/62, C12N 15/13, G01N 33/50

(54) **PEPTIDE-GRAFTED ANTIBODIES**
ANTIKÖRPER MIT GEPFROPFTEN PEPTIDEN
ANTICORPS À PEPTIDES GREFFÉS

(30) Priority: 04.12.2013 US 201361911829 P; 29.08.2014 US 201462043874 P
(43) Date of publication of application: 12.10.2016
(73) Proprietor: Innovative Targeting Solutions Inc., Vancouver, British Columbia V5M 4X7 (CA)
(72) Inventor: GALLO, Michael, North Vancouver, British Columbia V7R 2R1 (CA); KANG, Jaspal Singh, Surrey, British Columbia V4N 5H6 (CA); PIGOTT, Craig Robin, Vancouver, British Columbia V6J 2C1 (CA); LIN, Abby Yu Chen, Surrey, British Columbia V4N 1T8 (CA)
(74) Representative: Grund, Martin
(86) International application number: PCT/CA2014/051167
(87) International publication number: WO 2015/081440

(56) References cited:
- WO-A1-2013/134881
- WO-A2-2005/060642

### SEQUENCE LISTING

<110> INNOVATIVE TARGETING SOLUTIONS INC.
<120> G-PROTEIN COUPLED RECEPTOR AGONISTS AND METHODS
<130> V86880WO
<140> n/a
   <141> 2014-12-04
<150> 61/911,829
   <151> 2013-12-04
<150> 62/043,874
   <151> 2014-08-29
<160> 114
<170> PatentIn version 3.5
<210> 1
   <211> 7855
   <212> DNA
   <213> Artificial sequence
<220>
   <223> vector E425
<400> 1
   tcgacggggt ctgacgctca gtggaacgaa aactcacgtt aagggatttt ggtcatgaga      60
   ttatcaaaaa ggatcttcac ctagatcctt ttaaattaaa aatgaagttt taaatcaatc     120
   taaagtatat atgagtaaac ttggtctgac agttattatt tacccaccac tttggtaatt     180
   tcgcctttca cgtaatgcac aaattcttcc agctgatccg cacggctggc cagacgatct     240
   tcttcctggc ccagatacgc ctgacgcgct tccagaatca ccggctgata ctgcgccggc     300
   agacgttcca tggcccaatc cgccgccaca tctttcggcg caattttgcc ggtcaccgcg     360
   ctataccaaa tacggctcag ggtcagcacc acgttgcgtt catcacccgc ccaatccggc     420
   gggctgttcc acagggtcag ggtttcgttc agcgcttcaa acaggtcctg ttccggcacc     480
   ggatcaaaca gttcttccgc tgccggacca accagcgcca cgctatgttc acgcgctttg     540
   gtcagcagaa tggccagatc gatatcaatg gtcgccggtt caaaaatgcc ggccagaata     600
   tcgttacgct gccattcgcc aaactgcagc tcacgtttcg ccggataacg ccacggaata     660
   atgtcatcat gcaccacgat ggtcacttcc accgcacgca gaatttcgct ttcacccggg     720
   ctcgcgctgg tttccagcag atcgttaatc agcgcacgac gggtggtttc atccagacgc     780
   acggtcacgg tcaccagcag atcaatatcg ctatgcggtt tcaggccgcc atccaccgcg     840
   ctgccataca gatgcacggc cagcagggtc ggttccagat gacgttcaat cacgcccacc     900
   acttcagaca gctgggtgct cacttccgca atcaccgctt cacgcataat gttcagtttg     960
   gttttcggac ggctgccctg ctgggtcaca tcgttgctgc tccacagcat caggcaacgg    1020
   cccaccgcat aacgcgcctg ctgtttgctc gcacgcggca tgctctgcac gccaaaaaag 1080
   caatcatagc acgccataaa caccgccacc gcaccattac cgccgctacg ttcggtcagg 1140
   gtacggctcc aattacggct acgcatactc ttcctttttc aatattattg aagcatttat 1200
   cagggttatt gtctcatgag cggatacata tttgaatgta tttagaaaaa taaacaaata 1260
   ggggttccgc gcacatttcc ccgaaaagtg ccacctaaat tgtaagcgtt aatattttgt 1320
   taaaattcgc gttaaatttt tgttaaatca gctcattttt taaccaatag gccgaaatcg 1380
   gcaaaatccc ttataaatca aaagaataga ccgagatagg gttgagtggc cgctacaggg 1440
   cgctcccatt cgccattcag gctgcgcaac tgttgggaag ggcgtttcgg tgcgggcctc 1500
   ttcgctatta cgccagctgg cgaaaggggg atgtgctgca aggcgattaa gttgggtaac 1560
   gccagggttt tcccagtcac gacgttgtaa aacgacggcc agtgagcgcg acgtaatacg 1620
   actcactata gggcgaattg agtgaaggcc gtcaaggcct attgctcgat gagacgtctt 1680
   aattaactgg cctcatgggc cttcacttca ctgcccgctt tccagtcggg aaacctgtcg 1740
   tgccagctgc attaacatgg tcatagctgt ttccttgcgt attgggcgct ctccgcttcc 1800
   tcgctcactg actcgctgcg ctcggtcgtt cgggtaaagc ctggggtgcc taatgagcaa 1860
   aaggccagca aaaggccagg aaccgtaaaa aggccgcgtt gctggcgttt ttccataggc 1920
   tccgcccccc tgacgagcat cacaaaaatc gacgctcaag tcagaggtgg cgaaacccga 1980
   caggactata aagataccag gcgtttcccc ctggaagctc cctcgtgcgc tctcctgttc 2040
   cgaccctgcc gcttaccgga tacctgtccg cctttctccc ttcgggaagc gtggcgcttt 2100
   ctcatagctc acgctgtagg tatctcagtt cggtgtaggt cgttcgctcc aagctgggct 2160
   gtgtgcacga accccccgtt cagcccgacc gctgcgcctt atccggtaac tatcgtcttg 2220
   agtccaaccc ggtaagacac gacttatcgc cactggcagc agccactggt aacaggatta 2280
   gcagagcgag gtatgtaggc ggtgctacag agttcttgaa gtggtggcct aactacggct 2340
   acactagaag aacagtattt ggtatctgcg ctctgctgaa gccagttacc ttcggaaaaa 2400
   gagttggtag ctcttgatcc ggcaaacaaa ccaccgctgg tagcggtggt ttttttgttt 2460
   gcaagcagca gattacgcgc agaaaaaaag gatctcaaga agatcctttg atcttttcta 2520
   cggcgcgcct gaataacttc gtatagcata cattatagca atttatcgaa aaagcctgaa 2580
   ctcaccgcga catccgtgga gaaattcctc atcgaaaaat tcgactccgt gtccgatctc 2640
   atgcagctgt ccgagggcga ggagagtaga gcattctcat tcgatgtggg cgggagaggc 2700
   tacgtgctga gagtgaactc ttgtgccgac ggcttctaca aggaccgata cgtctaccgg 2760
   cattttgctt ccgccgctct gcctattcca gaagtcctgg acattgggga gtttagcgag 2820
   tccctcactt actgtattag ccggcgagcc cagggagtga cactccagga tctgcctgaa 2880
   actgaactgc ctgctgtgct ccagcctgtc gctgaggcaa tggatgctat tgctgctgcc 2940
   gatctgagtc agactagcgg attcggccca tttggacccc agggcattgg ccagtacaca 3000
   acatggcgag acttcatctg tgctatcgcc gatcctcacg tgtaccattg gcagactgtg 3060
   atggacgata ctgtgtctgc ttctgtggca caggcactcg acgaactcat gctgtgggct 3120
   gaggactgtc ctgaagtgag acatctggtc catgccgatt ttggctccaa caatgtgctc 3180
   accgataacg ggagaatcac tgccgtgatc gactggagcg aggcaatgtt tggcgattcc 3240
   cagtacgaag tggccaacat cttcttttgg cggccttggc tggcttgtat ggaacagcag 3300
   acccggtact ttgaacggcg ccaccctgag ctggctggga gtcctagact gagagcctac 3360
   atgctccgaa ttggcctgga tcagctctac cagtcactgg tggatggcaa tttcgacgat 3420
   gctgcttggg cacaggggcg ctgtgatgct attgtccgat ccggcgctgg aactgtgggg 3480
   agaacacaga tcgctaggag atccgctgct gtctggaccg atggatgtgt ggaagtgctg 3540
   gccgatagtg gaaaccggag gccttcaacc cgaccccggg caaaggagta atgaccgttt 3600
   aaacccgctg atcagcctcg actgtgcctt ctagttgcca gccatctgtt gtttgcccct 3660
   cccccgtgcc ttccttgacc ctggaaggtg ccactcccac tgtcctttcc taataaaatg 3720
   aggaaattgc atcgcattgt ctgagtaggt gtcattctat tctggggggt ggggtggggc 3780
   aggacagcaa gggggaggat tgggaagaca atagcaggca tgctggggat gcggtgggct 3840
   ctatggggat cccgcgttga cattgattat tgactagtta ttaatagtaa tcaattacgg 3900
   ggtcattagt tcatagccca tatatggagt tccgcgttac ataacttacg gtaaatggcc 3960
   cgcctggctg accgcccaac gacccccgcc cattgacgtc aataatgacg tatgttccca 4020
   tagtaacgcc aatagggact ttccattgac gtcaatgggt ggagtattta cggtaaactg 4080
   cccacttggc agtacatcaa gtgtatcata tgccaagtac gccccctatt gacgtcaatg 4140
   acggtaaatg gcccgcctgg cattatgccc agtacatgac cttatgggac tttcctactt 4200
   ggcagtacat ctacgtatta gtcatcgcta ttaccatggt gatgcggttt tggcagtaca 4260
   tcaatgggcg tggatagcgg tttgactcac ggggatttcc aagtctccac cccattgacg 4320
   tcaatgggag tttgttttgg caccaaaatc aacgggactt tccaaaatgt cgtaacaact 4380
   ccgccccatt gacgcaaatg ggcggtaggc gtgtacggtg ggaggtctat ataagcagag 4440
   ctctctggct aactagagaa cccactgctt actggcttat cgaaattaat acgactcact 4500
   atagggagac acaagctggc ggccgctctc ggaaggtctc tatgttgatc ctttttaacc 4560
   catcacatat acctgccgtt cactattatt tagtgaaatg agatattatg atattttctg 4620
   aattgtgatt aaaaaggcaa ctttatgccc atgcaacaga aactataaaa aatacagaga 4680
   atgaaaagaa acagatagat tttttagttc tttaggcccg tagtctgcaa atccttttat 4740
   gattttctat caaacaaaag aggaaaatag accagttgca atccaaacga gagtctaata 4800
   gaatgaggtc gaaaagtaaa tcgcgcgggt ttgttactga taaagcaggc aagacctaaa 4860
   atgtgtaaag ggcaaagtgt atactttggc gtcacccctt acatatttta ggtctttttt 4920
   tattgtgcgt aactaacttg ccatcttcaa acaggagggc tggaagaagc agaccgctaa 4980
   cacagtacat aaaaaaggag acatgaacga tgaacatcaa aaagtttgca aaacaagcaa 5040
   cagtattaac ctttactacc gcactgctgg caggaggcgc aactcaagcg tttgcgaaag 5100
   aaacgaacca aaagccatat aaggaaacat acggcatttc ccatattaca cgccatgata 5160
   tgctgcaaat ccctgaacag caaaaaaatg aaaaatatca agttcctgag ttcgattcgt 5220
   ccacaattaa aaatatctct tctgcaaaag gcctggacgt ttgggacagc tggccattac 5280
   aaaacgctga cggcactgtc gcaaactatc acggctacca catcgtcttt gcattagccg 5340
   gagatcctaa aaatgcggat gacacatcga tttacatgtt ctatcaaaaa gtcggcgaaa 5400
   cttctattga cagctggaaa aacgctggcc gcgtctttaa agacagcgac aaattcgatg 5460
   caaatgattc tatcctaaaa gaccaaacac aagaatggtc aggttcagcc acatttacat 5520
   ctgacggaaa aatccgttta ttctacactg atttctccgg taaacattac ggcaaacaaa 5580
   cactgacaac tgcacaagtt aacgtatcag catcagacag ctctttgaac atcaacggtg 5640
   tagaggatta taaatcaatc tttgacggtg acggaaaaac gtatcaaaat gtacagcagt 5700
   tcatcgatga aggcaactac agctcaggcg acaaccatac gctgagagat cctcactacg 5760
   tagaagataa aggccacaaa tacttagtat ttgaagcaaa cactggaact gaagatggct 5820
   accaaggcga agaatcttta tttaacaaag catactatgg caaaagcaca tcattcttcc 5880
   gtcaagaaag tcaaaaactt ctgcaaagcg ataaaaaacg cacggctgag ttagcaaacg 5940
   gcgctctcgg tatgattgag ctaaacgatg attacacact gaaaaaagtg atgaaaccgc 6000
   tgattgcatc taacacagta acagatgaaa ttgaacgcgc gaacgtcttt aaaatgaacg 6060
   gcaaatggta tctgttcact gactcccgcg gatcaaaaat gacgattgac ggcattacgt 6120
   ctaacgatat ttacatgctt ggttatgttt ctaattcttt aactggccca tacaagccgc 6180
   tgaacaaaac tggccttgtg ttaaaaatgg atcttgatcc taacgatgta acctttactt 6240
   actcacactt cgctgtacct caagcgaaag gaaacaatgt cgtgattaca agctatatga 6300
   caaacagagg attctacgca gacaaacaat caacgtttgc gcctagcttc ctgctgaaca 6360
   tcaaaggcaa gaaaacatct gttgtcaaag acagcatcct tgaacaagga caattaacag 6420
   ttaacaaata aaaacgcaaa agaaaatgcc gattatggtg cactctcagt acaatctggt 6480
   ctctcaggcg ccctgaccag cggcgtgcat accttcccgg ctgtcctaca gtcctcagga 6540
   ctctactccc tcagcagcgt ggtgaccgtg ccctccagca gcttgggcac ccagacctac 6600
   atctgcaacg tgaatcacaa gcccagcaac accaaggtgg acaagaaagt tgagcccaaa 6660
   tcttgtgaca aaactcacac atgcccaccg tgcccagcac ctgaactcct ggggggaccg     6720
   tcagtcttcc tcttcccccc aaaacccaag gacaccctca tgatctctag aacccctgag     6780
   gtcacatgcg tggtggtgga cgtgagccac gaagaccctg aggtcaagtt caactggtac     6840
   gtggacggcg tggaggtgca taatgccaag acaaagccgc gggaggagca gtacaacagc     6900
   acgtaccgtg tggtcagcgt cctcaccgtc ctgcaccagg actggctgaa tggcaaggag     6960
   tacaagtgca aggtgtccaa caaagccctc ccagccccca tcgagaaaac catctccaaa     7020
   gccaaaggtg ggacccgtgg ggtgcgaata gaagttccta ttccgaagtt cctattctct     7080
   agaaagtata ggaacttcgg gccacatgga attaattcag aggccggctc ggcccaccct     7140
   ctgccctgag agtgaccgct gtaccaacct ctgtccctac agggcagccc cgagaaccac     7200
   aggtgtacac cctgccccca tcccgggatg agctgaccaa gaaccaggtc agcctgacct     7260
   gcctggtcaa aggcttctat cccagcgaca tcgccgtgga gtgggagagc aatgggcagc     7320
   cggagaacaa ctacaagacc acgcctcccg tgctggactc cgacggctcc ttcttcctct     7380
   acagcaagct caccgtggac aagagcaggt ggcagcaggg gaacgtcttc tcatgctccg     7440
   tgatgcatga ggctctgcac aaccactaca cgcagaagag cctctccctg tctccgggca     7500
   aagctgtggg ccaggacacg caggaggtca tcgtggtgcc acactccttg ccctttaagg     7560
   tggtggtgat ctcagccatc ctggccctgg tggtgctcac catcatctcc cttatcatcc     7620
   tcatcatgct ttggcagaag aagccacgtt aggttttccg ggacgccggc tggatgatcc     7680
   tccagcgcgg ggatctcatg ctggagttct tcgcccaccc caacttgttt attgcagctt     7740
   ataatggtta caaataaagc aatagcatca caaatttcac aaataaagca tttttttcac     7800
   tgcattctag ttgtggtttg tccaaactca tcaatgtatc ttatcatgtc tgacc          7855
<210> 2
   <211> 1704
   <212> DNA
   <213> Artificial sequence
<220>
   <223> PCR product
<220>
   <221> misc_feature
   <222> (118)..(125)
   <223> n is a, c, g, or t
<400> 2
   catggcgagg gcaccttcac ctccgacctg tccaaacaaa tggaagaaga agccgtccgg      60
   ctgttcatcg aatggctgaa aaatggcggc ccttcctctg gcgcccctcc tccttctnnn     120
   nnnnnctggt tcgacccctg gggccaggga accctggtca ccgtgtcctc agcctccacc     180
   aagggcccat cggtcttccc cctggcaccc tcctccaaga gcacctctgg gggcacagcg     240
   gccctgggct gcctggtcaa ggactacttc cccgaaccgg tgacggtgtc gtggaactca     300
   ggcgccctga ccagcggcgt gcataccttc ccggctgtcc tacagtcctc aggactctac     360
   tccctcagca gcgtggtgac cgtgccctcc agcagcttgg gcacccagac ctacatctgc     420
   aacgtgaatc acaagcccag caacaccaag gtggacaaga aagttgagcc caaatcttgt     480
   gacaaaactc acacatgccc accgtgccca gcacctgaac tcctgggggg accgtcagtc     540
   ttcctcttcc ccccaaaacc caaggacacc ctcatgatct ctagaacccc tgaggtcaca     600
   tgcgtggtgg tggacgtgag ccacgaagac cctgaggtca agttcaactg gtacgtggac     660
   ggcgtggagg tgcataatgc caagacaaag ccgcgggagg agcagtacaa cagcacgtac     720
   cgtgtggtca gcgtcctcac cgtcctgcac caggactggc tgaatggcaa ggagtacaag     780
   tgcaaggtgt ccaacaaagc cctcccagcc cccatcgaga aaaccatctc caaagccaaa     840
   ggtgggaccc gtggggtgcg aatagaagtt cctattccga agttcctatt ctctagaaag     900
   tataggaact tcgggccaca tggaattaat tcagaggccg gctcggccca ccctctgccc     960
   tgagagtgac cgctgtacca acctctgtcc ctacagggca gccccgagaa ccacaggtgt    1020
   acaccctgcc cccatcccgg gatgagctga ccaagaacca ggtcagcctg acctgcctgg    1080
   tcaaaggctt ctatcccagc gacatcgccg tggagtggga gagcaatggg cagccggaga    1140
   acaactacaa gaccacgcct cccgtgctgg actccgacgg ctccttcttc ctctacagca    1200
   agctcaccgt ggacaagagc aggtggcagc aggggaacgt cttctcatgc tccgtgatgc    1260
   atgaggctct gcacaaccac tacacgcaga agagcctctc cctgtctccg ggcaaagctg    1320
   tgggccagga cacgcaggag gtcatcgtgg tgccacactc cttgcccttt aaggtggtgg    1380
   tgatctcagc catcctggcc ctggtggtgc tcaccatcat ctcccttatc atcctcatca    1440
   tgctttggca gaagaagcca cgttaggttt tccgggacgc cggctggatg atcctccagc    1500
   gcggggatct catgctggag ttcttcgccc accccaactt gtttattgca gcttataatg    1560
   gttacaaata aagcaatagc atcacaaatt tcacaaataa agcatttttt tcactgcatt    1620
   ctagttgtgg tttgtccaaa ctcatcaatg tatcttatca tgtctgacct cgacggggtc    1680
   tgacgctcag tggaacgaaa actc                                           1704
<210> 3
   <211> 1630
   <212> DNA
   <213> Artificial sequence
<220>
   <223> fragment derived from P163
<220>
   <221> misc_feature
   <222> (727)..(1026)
   <223> n is a, c, g, or t
<400> 3
   ggatcccgcg ttgacattga ttattgacta gttattaata gtaatcaatt acggggtcat 60
   tagttcatag cccatatatg gagttccgcg ttacataact tacggtaaat ggcccgcctg 120
   gctgaccgcc caacgacccc cgcccattga cgtcaataat gacgtatgtt cccatagtaa 180
   cgccaatagg gactttccat tgacgtcaat gggtggagta tttacggtaa actgcccact 240
   tggcagtaca tcaagtgtat catatgccaa gtacgccccc tattgacgtc aatgacggta 300
   aatggcccgc ctggcattat gcccagtaca tgaccttatg ggactttcct acttggcagt 360
   acatctacgt attagtcatc gctattacca tggtgatgcg gttttggcag tacatcaatg 420
   ggcgtggata gcggtttgac tcacggggat ttccaagtct ccaccccatt gacgtcaatg 480
   ggagtttgtt ttggcaccaa aatcaacggg actttccaaa atgtcgtaac aactccgccc 540
   cattgacgca aatgggcggt aggcgtgtac ggtgggaggt ctatataagc agagctctct 600
   ggctaactag agaacccact gcttactgct cgacgatctg atcaagagac aggataagga 660
   gccgccacca tggagtttgg gctgagctgg ctttttcttg tggctatttt aaaaggtgtc 720
   cagtgtnnnn nnnnnnnnnn nnnnnnnnnn nnnnnnnnnn nnnnnnnnnn nnnnnnnnnn 780
   nnnnnnnnnn nnnnnnnnnn nnnnnnnnnn nnnnnnnnnn nnnnnnnnnn nnnnnnnnnn 840
   nnnnnnnnnn nnnnnnnnnn nnnnnnnnnn nnnnnnnnnn nnnnnnnnnn nnnnnnnnnn 900
   nnnnnnnnnn nnnnnnnnnn nnnnnnnnnn nnnnnnnnnn nnnnnnnnnn nnnnnnnnnn 960
   nnnnnnnnnn nnnnnnnnnn nnnnnnnnnn nnnnnnnnnn nnnnnnnnnn nnnnnnnnnn 1020
   nnnnnncaca gtggtagtac tccactgtct gggtgtacaa aaacctccct gcacgcctct 1080
   ctaacctcac aattctgtgg cggccgcgcc gccaccatga ttgaacaaga tggattgcac 1140
   gcaggttctc cggccgcttg ggtggagagg ctattcggct atgactgggc acaacagaca 1200
   atcggctgct ctgatgccgc cgtgttccgg ctgtcagcgc aggggcgccc ggttcttttt 1260
   gtcaagaccg acctgtccgg tgccctgaat gaactgcagg acgaggcagc gcggctatcg 1320
   tggctggcca cgacgggcgt tccttgcgca gctgtgctcg acgttgtcac tgaagcggga 1380
   agggactggc tgctattggg cgaagtgccg gggcaggatc tcctgtcatc tcaccttgct 1440
   cctgccgaga aagtatccat catggctgat gcaatgcggc ggctgcatac gcttgatccg 1500
   gctacctgcc cattcgacca ccaagcgaaa catcgcatcg agcgagcacg tactcggatg 1560
   gaagccggtc ttgtcgatca ggtgagtaca ggaggtggag agtacgcgta acacttaagc 1620
   gtctctccaa 1630
<210> 4
   <211> 2423
   <212> DNA
   <213> Artificial sequence
<220>
   <223> vector V610 digested with KpnI and BsaI
<400> 4
   ggtacctctt aattaactgg cctcatgggc cttccgctca ctgcccgctt tccagtcggg 60
   aaacctgtcg tgccagctgc attaacatgg tcatagctgt ttccttgcgt attgggcgct 120
   ctccgcttcc tcgctcactg actcgctgcg ctcggtcgtt cgggtaaagc ctggggtgcc 180
   taatgagcaa aaggccagca aaaggccagg aaccgtaaaa aggccgcgtt gctggcgttt 240
   ttccataggc tccgcccccc tgacgagcat cacaaaaatc gacgctcaag tcagaggtgg 300
   cgaaacccga caggactata aagataccag gcgtttcccc ctggaagctc cctcgtgcgc 360
   tctcctgttc cgaccctgcc gcttaccgga tacctgtccg cctttctccc ttcgggaagc 420
   gtggcgcttt ctcatagctc acgctgtagg tatctcagtt cggtgtaggt cgttcgctcc 480
   aagctgggct gtgtgcacga accccccgtt cagcccgacc gctgcgcctt atccggtaac 540
   tatcgtcttg agtccaaccc ggtaagacac gacttatcgc cactggcagc agccactggt 600
   aacaggatta gcagagcgag gtatgtaggc ggtgctacag agttcttgaa gtggtggcct 660
   aactacggct acactagaag aacagtattt ggtatctgcg ctctgctgaa gccagttacc 720
   ttcggaaaaa gagttggtag ctcttgatcc ggcaaacaaa ccaccgctgg tagcggtggt 780
   tttttgtttt gcaagcagca gattacgcgc agaaaaaaag gatctcaaga agatcctttg 840
   atcttttcta cggggtctga cgctcagtgg aacgaaaact cacgttaagg gattttggtc 900
   atgagattat caaaaaggat cttcacctag atccttttaa attaaaaatg aagttttaaa 960
   tcaatctaaa gtatatatga gtaaacttgg tctgacagtt accaatgctt aatcagtgag 1020
   gcacctatct cagcgatctg tctatttcgt tcatccatag ttgcctgact ccccgtcgtg 1080
   tagataacta cgatacggga gggcttacca tctggcccca gtgctgcaat gataccgcga 1140
   gaaccacgct caccggctcc agatttatca gcaataaacc agccagccgg aagggccgag 1200
   cgcagaagtg gtcctgcaac tttatccgcc tccatccagt ctattaattg ttgccgggaa 1260
   gctagagtaa gtagttcgcc agttaatagt ttgcgcaacg ttgttgccat tgctacaggc 1320
   atcgtggtgt cacgctcgtc gtttggtatg gcttcattca gctccggttc ccaacgatca 1380
   aggcgagtta catgatcccc catgttgtgc aaaaaagcgg ttagctcctt cggtcctccg 1440
   atcgttgtca gaagtaagtt ggccgcagtg ttatcactca tggttatggc agcactgcat 1500
   aattctctta ctgtcatgcc atccgtaaga tgcttttctg tgactggtga gtactcaacc 1560
   aagtcattct gagaatagtg tatgcggcga ccgagttgct cttgcccggc gtcaatacgg 1620
   gataataccg cgccacatag cagaacttta aaagtgctca tcattggaaa acgttcttcg 1680
   gggcgaaaac tctcaaggat cttaccgctg ttgagatcca gttcgatgta acccactcgt 1740
   gcacccaact gatcttcagc atcttttact ttcaccagcg tttctgggtg agcaaaaaca 1800
   ggaaggcaaa atgccgcaaa aaagggaata agggcgacac ggaaatgttg aatactcata 1860
   ctcttccttt ttcaatatta ttgaagcatt tatcagggtt attgtctcat gagcggatac 1920
   atatttgaat gtatttagaa aaataaacaa ataggggttc cgcgcacatt tccccgaaaa 1980
   gtgccaccta aattgtaagc gttaatattt tgttaaaatt cgcgttaaat ttttgttaaa 2040
   tcagctcatt ttttaaccaa taggccgaaa tcggcaaaat cccttataaa tcaaaagaat 2100
   agaccgagat agggttgagt ggccgctaca gggcgctccc attcgccatt caggctgcgc 2160
   aactgttggg aagggcgttt cggtgcgggc ctcttcgcta ttacgccagc tggcgaaagg 2220
   gggatgtgct gcaaggcgat taagttgggt aacgccaggg ttttcccagt cacgacgttg 2280
   taaaacgacg gccagtgagc gcgacgtaat acgactcact atagggcgaa ttggcggaag 2340
   gccgtcaagg cctaggcgcg cccgtctctc caagtgcaaa gggacaggag gtttttgtta 2400
   agggctgtat cactgtggag acc 2423
<210> 5
   <211> 8514
   <212> DNA
   <213> Artificial sequence
<220>
   <223> acceptor vector E501
<400> 5
   cagactgccc cggggcagtt aggagatccg ggggccgccg ggaactgccg ctggcccccc      60
   accgccccaa ggatctcccg gtccccgccc ggcgtgctga cgtcacggcg ctgccccagg     120
   gtgtgctggg caggtcgcgg ggagcgctgg gaaatggagt ccattagcag aagtggccct     180
   tggccacttc caggagtcgc tgtgccccga tgcacactgg gaagtccgca gctccgaggc     240
   gcccagtgga aatcgccaga tgagggcctc ctccggggaa tgctgggaaa tggagtctac     300
   aggccggagg ggtgccccac ggcatactag gaagtgtgta gcaccgggta aaggggatga     360
   atagcagact gccccggggc agttaggaga tccgggggcc gccgggaact gccgctggcc     420
   ccccaccgcc ccaaggatct cccggtcccc gcccggcgtg ctgacgtcac ggcgctgccc     480
   cagggtgtgc tgggcaggtc gcggggagcg ctgggaaatg gagtccatta gcagaagtgg     540
   cccttggcca cttccaggag tcgctgtgcc ccgatgcaca ctgggaagtc cgcagctccg     600
   aggcgcccag tggaaatcgc cagatgaggg cctcctccgg ggaatgctgg gaaatggagt     660
   ctacaggccg gaggggtgcc ccacggcata ctaggaagtg tgtagcaccg ggtaaagggg     720
   atgaatagca gactgccccg gggcagttag gagatccggg ggccgccggg aactgccgct     780
   ggccccccac cgccccaagg atctcccggt ccccgcccgg cgtgctgacg tcacggcgct     840
   gccccagggt gtgctgggca ggtcgcgggg agcgctggga aatggagtcc attagcagaa 900
   gtggcccttg gccacttcca ggagtcgctg tgccccgatg cacactggga agtccgcagc 960
   tccgaggcgc ccagtggaaa tcgccagatg agggcctcct ccggggaatg ctgggaaatg 1020
   gagtctacag gccggagggg tgccccacgg catactagga agtgtgtagc accgggtaaa 1080
   ggggatgaat agcagactgc cccggggcag ttaggagatc cctcatgggc cttcctttca 1140
   ctgcactagt tattaatagt aatcaattac ggggtcatta gttcatagcc catatatgga 1200
   gttccgcgtt acataactta cggtaaatgg cccgcctggc tgaccgccca acgacccccg 1260
   cccattgacg tcaataatga cgtatgttcc catagtaacg ccaataggga ctttccattg 1320
   acgtcaatgg gtggagtatt tacggtaaac tgcccacttg gcagtacatc aagtgtatca 1380
   tatgccaagt acgcccccta ttgacgtcaa tgacggtaaa tggcccgcct ggcattatgc 1440
   ccagtacatg accttatggg actttcctac ttggcagtac atctacgtat tagtcatcgc 1500
   tattaccatg gtgatgcggt tttggcagta catcaatggg cgtggatagc ggtttgactc 1560
   acggggattt ccaagtctcc accccattga cgtcaatggg agtttgtttt ggcaccaaaa 1620
   tcaacgggac tttccaaaat gtcgtaacaa ctccgcccca ttgacgcaaa tgggcggtag 1680
   gcgtgtacgg tgggaggtct atataagcag agctctctgg ctaactagag aacccactgc 1740
   ttactggctt atcgaaatta atacgactca ctatagggag acccaagctg gctagcgttt 1800
   aaacttaagc ttgaggagcc gccaccatgg acatgagggt ccccgctcag ctcctggggc 1860
   tcctgctact ctggctccga ggtaaggatg gagaacacta ggaatttact cagccagtgt 1920
   gctcagtact gactggaact tcagggaagt tctctgataa catgattaat agtaagaata 1980
   tttgttttta tgtttccaat ctcaggtgcc agatgtgaca tccagatgac ccagtctcca 2040
   tcctccctgt ctgcatctgt aggagacaga gtcaccatca cttgccgggc aagtcagagc 2100
   attagcagct atttaaattg gtatcagcag aaaccaggga aagcccctaa gctcctgatc 2160
   tatgctgcat ccagtttgca aagtggggtc ccatcaaggt tcagtggcag tggatctggg 2220
   acagatttca ctctcaccat cagcagtctg caacctgaag attttgcaac ttactactgt 2280
   caacagagtt acagtacccc tctcactttc ggcggcggaa caaaggtgga gatcaagcgg 2340
   accgtggccg ctcccagcgt gttcatcttc ccccccagcg acgagcagct taagagcggt 2400
   accgctagcg tggtgtgcct gctgaacaac ttctaccccc gggaggccaa ggtgcagtgg 2460
   aaggtggaca acgccctgca gagcggcaac agccaggaaa gcgtcaccga gcaggacagc 2520
   aaggactcca cctacagcct gagcagcacc ctgaccctga gcaaggccga ctacgagaag 2580
   cacaaggtgt acgcctgcga agtgacccac cagggcctgt ccagccccgt gaccaagagc 2640
   ttcaaccggg gcgagtgcta atctagaggg cccgtttaaa cccgctgatc agcctcgact 2700
   gtgccttcta gttgccagcc atctgttgtt tgcccctccc ccgtgccttc cttgaccctg 2760
   gaaggtgcca ctcccactgt cctttcctaa taaaatgagg aaattgcatc gcattgtctg 2820
   agtaggtgtc attctattct ggggggtggg gtggggcagg acagcaaggg ggaggattgg 2880
   gaagacaata gcaggcatgc tggggatgcg gtgggctcta tggcttctga ggcggaaaga 2940
   accagctggg gctctagggg gtatccccac gcgccctgta gcggcgcatt aagcgcggcg 3000
   ggtgtggtgg ttacgcgcag cgtgaccgct acacttgcca gcgccctagc gcccgctcct 3060
   ttcgctttct tcccttcctt tctcgccacg ttcgccggct ttccccgtca agctctaaat 3120
   cgggggctcc ctttagggtt ccgatttagt gctttacggc acctcgaccc caaaaaactt 3180
   gattagggtg atggttcacg tagtgggcca tcgccctgat agacggtttt tcgccctttg 3240
   acgttggagt ccacgttctt taatagtgga ctcttgttcc aaactggaac aacactcaac 3300
   cctatctcgg tctattcttt tgatttataa gggattttgc cgatttcggc ctattggtta 3360
   aaaaatgagc tgatttaaca aaaatttaac gcgaattaat tctgtggaat gtgtgtcagt 3420
   tagggtgtgg aaagtcccca ggctccccag caggcagaag tatgcaaagc acgcgcatgc 3480
   ccgacggcga ggatctcgtc gtgacccatg gcgatgcctg cttgccgaat atcatggtgg 3540
   aaaatggccg cttttctgga ttcatcgact gtggccggct gggtgtggcg gaccgctatc 3600
   aggacatagc gttggctacc cgtgatattg ctgaagagct tggcggcgaa tgggctgacc 3660
   gcttcctcgt gctttacggt atcgccgctc ccgattcgca gcgcatcgcc ttctatcgcc 3720
   ttcttgacga gttcttctga gcgggactct ggggttcgaa atgaccgacc aagcgacgcc 3780
   caacctgcca tcacgagatt tcgattccac cgccgccttc tatgaaaggt tgggcttcgg 3840
   aatcgttttc cgggacgccg gctggatgat cctccagcgc ggggatctca tgctggagtt 3900
   cttcgcccac cccaacttgt ttattgcagc ttataatggt tacaaataaa gcaatagcat 3960
   cacaaatttc acaaataaag catttttttc actgcattct agttgtggtt tgtccaaact 4020
   catcaatgta tcttatcatg tctgtatacc gtcgattcga gttaattaac tggcctcatg 4080
   ggccttccgc tcactgcccg ctttccagtc gggaaacctg tcgtgccagc tgcattaaca 4140
   tggtcatagc tgtttccttg cgtattgggc gctctccgct tcctcgctca ctgactcgct 4200
   gcgctcggtc gttcgggtaa agcctggggt gcctaatgag caaaaggcca gcaaaaggcc 4260
   aggaaccgta aaaaggccgc gttgctggcg tttttccata ggctccgccc ccctgacgag 4320
   catcacaaaa atcgacgctc aagtcagagg tggcgaaacc cgacaggact ataaagatac 4380
   caggcgtttc cccctggaag ctccctcgtg cgctctcctg ttccgaccct gccgcttacc 4440
   ggatacctgt ccgcctttct cccttcggga agcgtggcgc tttctcatag ctcacgctgt 4500
   aggtatctca gttcggtgta ggtcgttcgc tccaagctgg gctgtgtgca cgaacccccc 4560
   gttcagcccg accgctgcgc cttatccggt aactatcgtc ttgagtccaa cccggtaaga 4620
   cacgacttat cgccactggc agcagccact ggtaacagga ttagcagagc gaggtatgta 4680
   ggcggtgcta cagagttctt gaagtggtgg cctaactacg gctacactag aagaacagta 4740
   tttggtatct gcgctctgct gaagccagtt accttcggaa aaagagttgg tagctcttga 4800
   tccggcaaac aaaccaccgc tggtagcggt ggtttttttg tttgcaagca gcagattacg 4860
   cgcagaaaaa aaggatctca agaagatcct ttgatctttt ctacggggtc tgacgctcag 4920
   tggaacgaaa actcacgtta agggattttg gtcatgagat tatcaaaaag gatcttcacc 4980
   tagatccttt taaattaaaa atgaagtttt aaatcaatct aaagtatata tgagtaaact 5040
   tggtctgaca gttaccaatg cttaatcagt gaggcaccta tctcagcgat ctgtctattt 5100
   cgttcatcca tagttgcctg actccccgtc gtgtagataa ctacgatacg ggagggctta 5160
   ccatctggcc ccagtgctgc aatgataccg cgagaaccac gctcaccggc tccagattta 5220
   tcagcaataa accagccagc cggaagggcc gagcgcagaa gtggtcctgc aactttatcc 5280
   gcctccatcc agtctattaa ttgttgccgg gaagctagag taagtagttc gccagttaat 5340
   agtttgcgca acgttgttgc cattgctaca ggcatcgtgg tgtcacgctc gtcgtttggt 5400
   atggcttcat tcagctccgg ttcccaacga tcaaggcgag ttacatgatc ccccatgttg 5460
   tgcaaaaaag cggttagctc cttcggtcct ccgatcgttg tcagaagtaa gttggccgca 5520
   gtgttatcac tcatggttat ggcagcactg cataattctc ttactgtcat gccatccgta 5580
   agatgctttt ctgtgactgg tgagtactca accaagtcat tctgagaata gtgtatgcgg 5640
   cgaccgagtt gctcttgccc ggcgtcaata cgggataata ccgcgccaca tagcagaact 5700
   ttaaaagtgc tcatcattgg aaaacgttct tcggggcgaa aactctcaag gatcttaccg 5760
   ctgttgagat ccagttcgat gtaacccact cgtgcaccca actgatcttc agcatctttt 5820
   actttcacca gcgtttctgg gtgagcaaaa acaggaaggc aaaatgccgc aaaaaaggga 5880
   ataagggcga cacggaaatg ttgaatactc atactcttcc tttttcaata ttattgaagc 5940
   atttatcagg gttattgtct catgagcgga tacatatttg aatgtattta gaaaaataaa 6000
   caaatagggg ttccgcgcac atttccccga aaagtgccac ctaaattgta agcgttaata 6060
   ttttgttaaa attcgcgtta aatttttgtt aaatcagctc attttttaac caataggccg 6120
   aaatcggcaa aatcccttat aaatcaaaag aatagaccga gatagggttg agtggccgct 6180
   acagggcgct cccattcgcc attcaggctg cgcaactgtt gggaagggcg tttcggtgcg 6240
   ggcctcttcg ctattacgcc agctggcgaa agggggatgt gctgcaaggc gattaagttg 6300
   ggtaacgcca gggttttccc agtcacgacg ttgtaaaacg acggccagtg agcgcgacgt 6360
   aatacgactc actatagggc gaattggcgg aaggccgtca aggcctaggc gcgcctgaat 6420
   aacttcgtat agcatacatt atagcaattt atcgaaaaag cctgaactca ccgcgacatc 6480
   cgtggagaaa ttcctcatcg aaaaattcga ctccgtgtcc gatctcatgc agctgtccga 6540
   gggcgaggag agtagagcat tctcattcga tgtgggcggg agaggctacg tgctgagagt 6600
   gaactcttgt gccgacggct tctacaagga ccgatacgtc taccggcatt ttgcttccgc 6660
   cgctctgcct attccagaag tcctggacat tggggagttt agcgagtccc tcacttactg 6720
   tattagccgg cgagcccagg gagtgacact ccaggatctg cctgaaactg aactgcctgc 6780
   tgtgctccag cctgtcgctg aggcaatgga tgctattgct gctgccgatc tgagtcagac 6840
   tagcggattc ggcccatttg gaccccaggg cattggccag tacacaacat ggcgagactt 6900
   catctgtgct atcgccgatc ctcacgtgta ccattggcag actgtgatgg acgatactgt 6960
   gtctgcttct gtggcacagg cactcgacga actcatgctg tgggctgagg actgtcctga 7020
   agtgagacat ctggtccatg ccgattttgg ctccaacaat gtgctcaccg ataacgggag 7080
   aatcactgcc gtgatcgact ggagcgaggc aatgtttggc gattcccagt acgaagtggc 7140
   caacatcttc ttttggcggc cttggctggc ttgtatggaa cagcagaccc ggtactttga 7200
   acggcgccac cctgagctgg ctgggagtcc tagactgaga gcctacatgc tccgaattgg 7260
   cctggatcag ctctaccagt cactggtgga tggcaatttc gacgatgctg cttgggcaca 7320
   ggggcgctgt gatgctattg tccgatccgg cgctggaact gtggggagaa cacagatcgc 7380
   taggagatcc gctgctgtct ggaccgatgg atgtgtggaa gtgctggccg atagtggaaa 7440
   ccggaggcct tcaacccgac cccgggcaaa ggagtaatga ccgtttaaac ccgctgatca 7500
   gcctcgactg tgccttctag ttgccagcca tctgttgttt gcccctcccc cgtgccttcc 7560
   ttgaccctgg aaggtgccac tcccactgtc ctttcctaat aaaatgagga aattgcatcg 7620
   cattgtctga gtaggtgtca ttctattctg gggggtgggg tggggcagga cagcaagggg 7680
   gaggattggg aagacaatag caggcatgct ggggatgcgg tgggctctat ggggatcccg 7740
   cgttgacatt gattattgac tagcctcgga ttctcgaggc taggctagtg agacgatacg 7800
   tctcatcgac gatccggggg ccgccgggaa ctgccgctgg ccccccaccg ccccaaggat 7860
   ctcccggtcc ccgcccggcg tgctgacgtc acggcgctgc cccagggtgt gctgggcagg 7920
   tcgcggggag cgctgggaaa tggagtccat tagcagaagt ggcccttggc cacttccagg 7980
   agtcgctgtg ccccgatgca cactgggaag tccgcagctc cgaggcgccc agtggaaatc 8040
   gccagatgag ggcctcctcc ggggaatgct gggaaatgga gtctacaggc cggaggggtg 8100
   ccccacggca tactaggaag tgtgtagcac cgggtaaagg ggatgaatag cagactgccc 8160
   cggggcagtt aggagatccg gggccgccgg ggaactgccg ctggcccccc accgccccaa 8220
   ggatctcccg gtccccgccc ggcgtgctga cgtcacggcg ctgccccagg gtgtgctggg 8280
   caggtcgcgg ggagcgctgg gaaatggagt ccattagcag aagtggccct tggccacttc 8340
   caggagtcgc tgtgccccga tgcacactgg gaagtccgca gctccgaggc gcccagtgga 8400
   aatcgccaga tgagggcctc ctccggggaa tgctgggaaa tggagtctac aggccggagg 8460
   ggtgccccac ggcatactag gaagtgtgta gcaccgggta aaggggatga atag 8514
<210> 6
   <211> 7593
   <212> DNA
   <213> Artificial sequence
<220>
   <223> vector comprising V503 expression cassette containing coding sequence for CRE recombinase
<400> 6
   gacggatcgg gagatctcgt tgacattgat tattgactag ttattaatag taatcaatta 60
   cggggtcatt agttcatagc ccatatatgg agttccgcgt tacataactt acggtaaatg 120
   gcccgcctgg ctgaccgccc aacgaccccc gcccattgac gtcaataatg acgtatgttc 180
   ccatagtaac gccaataggg actttccatt gacgtcaatg ggtggagtat ttacggtaaa 240
   ctgcccactt ggcagtacat caagtgtatc atatgccaag tacgccccct attgacgtca 300
   atgacggtaa atggcccgcc tggcattatg cccagtacat gaccttatgg gactttccta 360
   cttggcagta catctacgta ttagtcatcg ctattaccat ggtgatgcgg ttttggcagt 420
   acatcaatgg gcgtggatag cggtttgact cacggggatt tccaagtctc caccccattg 480
   acgtcaatgg gagtttgttt tggcaccaaa atcaacggga ctttccaaaa tgtcgtaaca 540
   actccgcccc attgacgcaa atgggcggta ggcgtgtacg gtgggaggtc tatataagca 600
   gagctctctg gctaactaga gaacccactg cttactggct tatcgaaatt aatacgactc 660
   actataggga gacccaagct ggctagcgtt taaacttaag cttggtaccg agctcggatc 720
   cgccgccacc atgggcagca acaacgccct ccagaccatc atcaacgccc gcctgcccgg 780
   cgaagagggc ctgtggcaga tccatctcca ggacggcaag atcagcgcca tcgacgccca 840
   gagcggcgtg atgcccatca ccgagaacag cctggacgcc gagcagggcc tggtgatccc 900
   ccccttcgtg gaaccccaca tccacctgga caccacccag acagccggcc agcccaactg 960
   gaaccagtcc ggcaccctgt tcgagggcat cgagagatgg gccgagcgga aggccctgct 1020
   gacccacgac gacgtgaagc agcgggcctg gcagaccctg aagtggcaga tcgccaacgg 1080
   catccagcac gtgcggacac acgtggacgt gtccgatgcc accctgaccg ccctgaaggc 1140
   catgctggaa gtgaagcagg aagtggcccc ctggatcgac ctccagatcg tggccttccc 1200
   acaggaaggc atcctgagct accccaacgg cgaagccctg ctggaagagg ccctgagact 1260
   gggcgccgac gtggtcggag ccatccccca cttcgagttc acccgcgagt acggcgtgga 1320
   aagcctgcac aagaccttcg ccctggccca gaagtacgac cggctgatcg atgtgcattg 1380
   cgacgagatc gacgacgagc agagcagatt cgtggaaacc gtggccgccc tggctcacca 1440
   cgagggaatg ggagccagag tgaccgccag ccacaccacc gccatgcaca gctacaacgg 1500
   cgcctacacc tcccggctgt tccggctgct gaagatgagc ggcatcaact tcgtggccaa 1560
   ccccctggtg aacatccatc tgcaaggcag attcgacacc taccccaagc ggagaggcat 1620
   caccagagtg aaagagatgc tggaatccgg catcaacgtg tgcttcggcc acgacgatgt 1680
   gttcgacccc tggtatcccc tgggcaccgc caacatgctc caggtgctgc acatgggcct 1740
   gcacgtgtgc cagctgatgg gctacggcca gatcaacgac ggcctgaacc tgatcaccca 1800
   ccacagcgcc cggaccctga acctgcaaga ctacggcatt gccgccggaa acagcgccaa 1860
   cctgatcatc ctgcccgccg agaacggctt cgacgccctg cggagacagg tgccagtgcg 1920
   gtatagcgtg cggggaggca aagtgatcgc ctccacccag cccgcccaga ccaccgtgta 1980
   cctggaacag cccgaggcca tcgactacaa gagatgagcg gccctagagg gcccgtttaa 2040
   acccgctgat cagcctcgac tgtgccttct agttgccagc catctgttgt ttgcccctcc 2100
   cccgtgcctt ccttgaccct ggaaggtgcc actcccactg tcctttccta ataaaatgag 2160
   gaaattgcat cgcattgtct gagtaggtgt cattctattc tggggggtgg ggtggggcag 2220
   gacagcaagg gggaggattg ggaagacaat agcaggcatg ctggggatgc ggtgggctct 2280
   atggacgcgt tgacattgat tattgactag ttattaatag taatcaatta cggggtcatt 2340
   agttcatagc ccatatatgg agttccgcgt tacataactt acggtaaatg gcccgcctgg 2400
   ctgaccgccc aacgaccccc gcccattgac gtcaataatg acgtatgttc ccatagtaac 2460
   gccaataggg actttccatt gacgtcaatg ggtggagtat ttacggtaaa ctgcccactt 2520
   ggcagtacat caagtgtatc atatgccaag tacgccccct attgacgtca atgacggtaa 2580
   atggcccgcc tggcattatg cccagtacat gaccttatgg gactttccta cttggcagta 2640
   catctacgta ttagtcatcg ctattaccat ggtgatgcgg ttttggcagt acatcaatgg 2700
   gcgtggatag cggtttgact cacggggatt tccaagtctc caccccattg acgtcaatgg 2760
   gagtttgttt tggcaccaaa atcaacggga ctttccaaaa tgtcgtaaca actccgcccc 2820
   attgacgcaa atgggcggta ggcgtgtacg gtgggaggtc tatataagca gagctctctg 2880
   gctaactaga gaacccactg cttactggct tatcgaaatt aatacgactc actataggga 2940
   gacccaagct ggctagcgtt taaacttaag cttgctgtga aatgtccaat ttactgaccg 3000
   tacaccaaaa tttgcctgca ttaccggtcg atgcaacgag tgatgaggtt cgcaagaacc 3060
   tgatggacat gttcagggat cgccaggcgt tttctgagca tacctggaaa atgcttctgt 3120
   ccgtttgccg gtcgtgggcg gcatggtgca agttgaataa ccggaaatgg tttcccgcag 3180
   aacctgaaga tgttcgcgat tatcttctat atcttcaggc gcgcggtctg gcagtaaaaa 3240
   ctatccagca acatttgggc cagctaaaca tgcttcatcg tcggtccggg ctgccacgac 3300
   caagtgacag caatgctgtt tcactggtta tgcggcggat ccgaaaagaa aacgttgatg 3360
   ccggtgaacg tgcaaaacag gctctagcgt tcgaacgcac tgatttcgac caggttcgtt 3420
   cactcatgga aaatagcgat cgctgccagg atatacgtaa tctggcattt ctggggattg 3480
   cttataacac cctgttacgt atagccgaaa ttgccaggat cagggttaaa gatatctcac 3540
   gtactgacgg tgggagaatg ttaatccata ttggcagaac gaaaacgctg gttagcaccg 3600
   caggtgtaga gaaggcactt agcctggggg taactaaact ggtcgagcga tggatttccg 3660
   tctctggtgt agctgatgat ccgaataact acctgttttg ccgggtcaga aaaaatggtg 3720
   ttgccgcgcc atctgccacc agccagctat caactcgcgc cctggaaggg atttttgaag 3780
   caactcatcg attgatttac ggcgctaagg atgactctgg tcagagatac ctggcctggt 3840
   ctggacacag tgcccgtgtc ggagccgcgc gagatatggc ccgcgctgga gtttcaatac 3900
   cggagatcat gcaagctggt ggctggacca atgtaaatat tgtcatgaac tatatccgta 3960
   acctggatag tgaaacaggg gcaatggtgc gcctgctgga agatggcgat tagccatcta 4020
   gagggcccgt ttaaacccgc tgatcagcct cgactgtgcc ttctagttgc cagccatctg 4080
   ttgtttgccc ctcccccgtg ccttccttga ccctggaagg tgccactccc actgtccttt 4140
   cctaataaaa tgaggaaatt gcatcgcatt gtctgagtag gtgtcattct attctggggg 4200
   gtggggtggg gcaggacagc aagggggagg attgggaaga caatagcagg catgctgggg 4260
   atgcggtggg ctctatggct tctgaggcgg aaagaaccag ctggggctct agggggtatc 4320
   cccacgcgcc ctgtagcggc gcattaagcg cggcgggtgt ggtggttacg cgcagcgtga 4380
   ccgctacact tgccagcgcc ctagcgcccg ctcctttcgc tttcttccct tcctttctcg 4440
   ccacgttcgc cggctttccc cgtcaagctc taaatcgggg gctcccttta gggttccgat 4500
   ttagtgcttt acggcacctc gaccccaaaa aacttgatta gggtgatggt tcacgtagtg 4560
   ggccatcgcc ctgatagacg gtttttcgcc ctttgacgtt ggagtccacg ttctttaata 4620
   gtggactctt gttccaaact ggaacaacac tcaaccctat ctcggtctat tcttttgatt 4680
   tataagggat tttgccgatt tcggcctatt ggttaaaaaa tgagctgatt taacaaaaat 4740
   ttaacgcgaa ttaattctgt ggaatgtgtg tcagttaggg tgtggaaagt ccccaggctc 4800
   cccagcaggc agaagtatgc aaagcacgcg catgcccgac ggcgaggatc tcgtcgtgac 4860
   ccatggcgat gcctgcttgc cgaatatcat ggtggaaaat ggccgctttt ctggattcat 4920
   cgactgtggc cggctgggtg tggcggaccg ctatcaggac atagcgttgg ctacccgtga 4980
   tattgctgaa gagcttggcg gcgaatgggc tgaccgcttc ctcgtgcttt acggtatcgc 5040
   cgctcccgat tcgcagcgca tcgccttcta tcgccttctt gacgagttct tctgagcggg 5100
   actctggggt tcgaaatgac cgaccaagcg acgcccaacc tgccatcacg agatttcgat 5160
   tccaccgccg ccttctatga aaggttgggc ttcggaatcg ttttccggga cgccggctgg 5220
   atgatcctcc agcgcgggga tctcatgctg gagttcttcg cccaccccaa cttgtttatt 5280
   gcagcttata atggttacaa ataaagcaat agcatcacaa atttcacaaa taaagcattt 5340
   ttttcactgc attctagttg tggtttgtcc aaactcatca atgtatctta tcatgtctgt 5400
   ataccgtcga cctctagcta gagcttggcg taatcatggt catagctgtt tcctgtgtga 5460
   aattgttatc cgctcacaat tccacacaac atacgagccg gaagcataaa gtgtaaagcc 5520
   tggggtgcct aatgagtgag ctaactcaca ttaattgcgt tgcgctcact gcccgctttc 5580
   cagtcgggaa acctgtcgtg ccagctgcat taatgaatcg gccaacgcgc ggggagaggc 5640
   ggtttgcgta ttgggcgctc ttccgcttcc tcgctcactg actcgctgcg ctcggtcgtt 5700
   cggctgcggc gagcggtatc agctcactca aaggcggtaa tacggttatc cacagaatca 5760
   ggggataacg caggaaagaa catgtgagca aaaggccagc aaaaggccag gaaccgtaaa 5820
   aaggccgcgt tgctggcgtt tttccatagg ctccgccccc ctgacgagca tcacaaaaat 5880
   cgacgctcaa gtcagaggtg gcgaaacccg acaggactat aaagatacca ggcgtttccc 5940
   cctggaagct ccctcgtgcg ctctcctgtt ccgaccctgc cgcttaccgg atacctgtcc 6000
   gcctttctcc cttcgggaag cgtggcgctt tctcatagct cacgctgtag gtatctcagt 6060
   tcggtgtagg tcgttcgctc caagctgggc tgtgtgcacg aaccccccgt tcagcccgac 6120
   cgctgcgcct tatccggtaa ctatcgtctt gagtccaacc cggtaagaca cgacttatcg 6180
   ccactggcag cagccactgg taacaggatt agcagagcga ggtatgtagg cggtgctaca 6240
   gagttcttga agtggtggcc taactacggc tacactagaa gaacagtatt tggtatctgc 6300
   gctctgctga agccagttac cttcggaaaa agagttggta gctcttgatc cggcaaacaa 6360
   accaccgctg gtagcggttt ttttgtttgc aagcagcaga ttacgcgcag aaaaaaagga 6420
   tctcaagaag atcctttgat cttttctacg gggtctgacg ctcagtggaa cgaaaactca 6480
   cgttaaggga ttttggtcat gagattatca aaaaggatct tcacctagat ccttttaaat 6540
   taaaaatgaa gttttaaatc aatctaaagt atatatgagt aaacttggtc tgacagttac 6600
   caatgcttaa tcagtgaggc acctatctca gcgatctgtc tatttcgttc atccatagtt 6660
   gcctgactcc ccgtcgtgta gataactacg atacgggagg gcttaccatc tggccccagt 6720
   gctgcaatga taccgcgaga cccacgctca ccggctccag atttatcagc aataaaccag 6780
   ccagccggaa gggccgagcg cagaagtggt cctgcaactt tatccgcctc catccagtct 6840
   attaattgtt gccgggaagc tagagtaagt agttcgccag ttaatagttt gcgcaacgtt 6900
   gttgccattg ctacaggcat cgtggtgtca cgctcgtcgt ttggtatggc ttcattcagc 6960
   tccggttccc aacgatcaag gcgagttaca tgatccccca tgttgtgcaa aaaagcggtt 7020
   agctccttcg gtcctccgat cgttgtcaga agtaagttgg ccgcagtgtt atcactcatg 7080
   gttatggcag cactgcataa ttctcttact gtcatgccat ccgtaagatg cttttctgtg     7140
   actggtgagt actcaaccaa gtcattctga gaatagtgta tgcggcgacc gagttgctct     7200
   tgcccggcgt caatacggga taataccgcg ccacatagca gaactttaaa agtgctcatc     7260
   attggaaaac gttcttcggg gcgaaaactc tcaaggatct taccgctgtt gagatccagt     7320
   tcgatgtaac ccactcgtgc acccaactga tcttcagcat cttttacttt caccagcgtt     7380
   tctgggtgag caaaaacagg aaggcaaaat gccgcaaaaa agggaataag ggcgacacgg     7440
   aaatgttgaa tactcatact cttccttttt caatattatt gaagcattta tcagggttat     7500
   tgtctcatga gcggatacat atttgaatgt atttagaaaa ataaacaaat aggggttccg     7560
   cgcacatttc cccgaaaagt gccacctgac gtc                                  7593
<210> 7
   <211> 39
   <212> PRT
   <213> Heloderma suspectum
<220>
   <221> misc_feature
   <223> Exendin-4
<400> 7
   His Gly Glu Gly Arg Phe Thr Ser Asp Leu Ser Lys Gln Met Glu Glu
   1               5                   10                  15
   Glu Ala Val Arg Leu Phe Ile Glu Trp Leu Lys Asn Gly Gly Pro Ser
               20                  25                  30
   Ser Gly Ala Pro Pro Pro Ser
           35
<210> 8
   <211> 30
   <212> PRT
   <213> Homo sapiens
<220>
   <221> misc_feature
   <223> GLP-1
<400> 8
   His Ala Glu Gly Thr Phe Thr Ser Asp Val Ser Ser Tyr Leu Glu Gly
   1               5                   10                  15
   Gln Ala Ala Lys Glu Phe Ile Ala Trp Leu Val Lys Gly Arg
               20                  25                  30
<210> 9
   <211> 34
   <212> PRT
   <213> Homo sapiens
<220>
   <221> misc_feature
   <223> GLP-2
<400> 9
   His Ala Asp Gly Ser Phe Ser Asp Glu Met Asn Thr Ile Leu Asp Asn
   1               5                   10                  15
   Leu Ala Ala Arg Asp Phe Ile Asn Trp Leu Ile Gln Thr Lys Ile Thr
               20                  25                  30
   Asp Arg
<210> 10
   <211> 29
   <212> PRT
   <213> Homo sapiens
<220>
   <221> misc_feature
   <223> Glucagon
<400> 10
   His Ser Gln Gly Thr Phe Thr Ser Asp Tyr Ser Lys Tyr Leu Asp Ser
   1               5                   10                  15
   Arg Arg Ala Gln Asp Arg Val Gln Trp Leu Met Asn Thr
               20                  25
<210> 11
   <211> 38
   <212> PRT
   <213> Homo sapiens
<220>
   <221> misc_feature
   <223> PACAP-38
<400> 11
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu Gly Lys Arg Tyr Lys
               20                  25                  30
   Gln Arg Val Lys Asn Lys
   35
<210> 12
   <211> 115
   <212> PRT
   <213> Homo sapiens
<220>
   <221> misc_feature
   <223> parathyroid hormone
<400> 12
<210> 13
   <211> 12
   <212> **DNA**
   <213> Artificial sequence
<220>
   <223> RSS spacer
<400> 13
   atacagacct ta 12
<210> 14
   <211> 12
   <212> DNA
   <213> Artificial sequence
<220>
   <223> RSS spacer
<400> 14
   ctacagactg ga 12
<210> 15
   <211> 12
   <212> DNA
   <213> Artificial sequence
<220>
   <223> RSS spacer
<400> 15
   ctccagggct ga 12
<210> 16
   <211> 12
   <212> DNA
   <213> Artificial sequence
<220>
   <223> RSS spacer
<400> 16
   gtacagacca at 12
<210> 17
   <211> 23
   <212> DNA
   <213> Artificial sequence
<220>
   <223> RSS spacer
<400> 17
   gtagtactcc actgtctggc tgt 23
<210> 18
   <211> 23
   <212> DNA
   <213> Artificial sequence
<220>
   <223> RSS spacer
<400> 18
   ttgcaaccac atcctgagtg tgt 23
<210> 19
   <211> 23
   <212> DNA
   <213> Artificial sequence
<220>
   <223> RSS spacer
<400> 19
   acggagataa aggaggaagc agg 23
<210> 20
   <211> 23
   <212> DNA
   <213> Artificial sequence
<220>
   <223> RSS spacer
<400> 20
   gccgggcccc gcggcccggc ggc 23
<210> 21
   <211> 5986
   <212> DNA
   <213> Artificial sequence
<220>
   <223> vector V525 expressing GLP1 receptor
<400> 21
   aacaaaatat taacgcttac aatttccatt cgccattcag gctgcgcaac tgttgggaag      60
   ggcgatcggt gcgggcctct tcgctattac gccagctggc gaaaggggga tgtgctgcaa     120
   ggcgattaag ttgggtaacg ccagggtttt cccagtcacg acgttgtaaa acgacggcca     180
   gtgccaagct gatctataca ttgaatcaat attggcaatt agccatatta gtcattggtt     240
   atatagcata aatcaatatt ggctattggc cattgcatac gttgtatcta tatcataata     300
   tgtacattta tattggctca tgtccaatat gaccgccatg ttgacattga ttattgacta     360
   gttattaata gtaatcaatt acggggtcat tagttcatag cccatatatg gagttccgcg     420
   ttacataact tacggtaaat ggcccgcctg gctgaccgcc caacgacccc cgcccattga     480
   cgtcaataat gacgtatgtt cccatagtaa cgccaatagg gactttccat tgacgtcaat     540
   gggtggagta tttacggtaa actgcccact tggcagtaca tcaagtgtat catatgccaa     600
   gtccgccccc tattgacgtc aatgacggta aatggcccgc ctggcattat gcccagtaca     660
   tgaccttacg ggactttcct acttggcagt acatctacgt attagtcatc gctattacca     720
   tggtgatgcg gttttggcag tacaccaatg ggcgtggata gcggtttgac tcacggggat     780
   ttccaagtct ccaccccatt gacgtcaatg ggagtttgtt ttggcaccaa aatcaacggg     840
   actttccaaa atgtcgtaat aaccccgccc cgttgacgca aatgggcggt aggcgtgtac     900
   ggtgggaggt ctatataagc agagctcgtt tagtgaaccg tcagaatttt gtaatacgac     960
   tcactatagg gcggccgcga attcgccctt gatggcccag tcctgaactc cccgccatgg    1020
   ccggcgcccc cggcccgctg cgccttgcgc tgctgctgct cgggatggtg ggcagggccg    1080
   gccccgcccc ccagggtgcc actgtgtccc tctgggagac ggtgcagaaa tggcgagaat 1140
   accgacgcca gtgccagcgc tccctgactg aggatccacc tcctgccaca gacttgttct 1200
   gcaaccggac cttcgatgaa tacgcctgct ggccagatgg ggagccaggc tcgttcgtga 1260
   atgtcagctg cccctggtac ctgccctggg ccagcagtgt gccgcagggc cacgtgtacc 1320
   ggttctgcac agctgaaggc ctctggctgc agaaggacaa ctccagcctg ccctggaggg 1380
   acttgtcgga gtgcgaggag tccaagcgag gggagagaag ctccccggag gagcagctcc 1440
   tgttcctcta catcatctac acggtgggct acgcactctc cttctctgct ctggttatcg 1500
   cctctgcgat cctcctcggc ttcagacacc tgcactgcac caggaactac atccacctga 1560
   acctgtttgc atccttcatc ctgcgagcat tgtccgtctt catcaaggac gcagccctga 1620
   agtggatgta tagcacagcc gcccagcagc accagtggga tgggctcctc tcctaccagg 1680
   actctctgag ctgccgcctg gtgtttctgc tcatgcagta ctgtgtggcg gccaattact 1740
   actggctctt ggtggagggc gtgtacctgt acacactgct ggccttctcg gtcttctctg 1800
   agcaatggat cttcaggctc tacgtgagca taggctgggg tgttcccctg ctgtttgttg 1860
   tcccctgggg cattgtcaag tacctctatg aggacgaggg ctgctggacc aggaactcca 1920
   acatgaacta ctggctcatt atccggctgc ccattctctt tggcattggg gtgaacttcc 1980
   tcatctttgt tcgggtcatc tgcatcgtgg tatccaaact gaaggccaat ctcatgtgca 2040
   agacagacat caaatgcaga cttgccaagt ccacgctgac actcatcccc ctgctgggga 2100
   ctcatgaggt catctttgcc tttgtgatgg acgagcacgc ccgggggacc ctgcgcttca 2160
   tcaagctgtt tacagagctc tccttcacct ccttccaggg gctgatggtg gccatattat 2220
   actgctttgt caacaatgag gtccagctgg aatttcggaa gagctgggag cgctggcggc 2280
   ttgagcactt gcacatccag agggacagca gcatgaagcc cctcaagtgt cccaccagca 2340
   gcctgagcag tggagccacg gcgggcagca gcatgtacac agccacttgc caggcctcct 2400
   gcagctgaga ctccagcgcc tgccctccct ggggtccttg ctgcaggcct ggtggccaat 2460
   ccaggtggga gagacactca agggcgaatt cagatctggt accgatatca agcttgtcga 2520
   ctctagattg cggccgcggt catagctgtt tcctgaacag atcccgggtg gcatccctgt 2580
   gacccctccc cagtgcctct cctggccctg gaagttgcca ctccagtgcc caccagcctt 2640
   gtcctaataa aattaagttg catcattttg tctgactagg tgtccttcta taatattatg 2700
   gggtggaggg gggtggtatg gagcaagggg caagttggga agacaacctg tagggcctgc 2760
   ggggtctatt gggaaccaag ctggagtgca gtggcacaat cttggctcac tgcaatctcc 2820
   gcctcctggg ttcaagcgat tctcctgcct cagcctcccg agttgttggg attccaggca 2880
   tgcatgacca ggctcagcta atttttgttt ttttggtaga gacggggttt caccatattg 2940
   gccaggctgg tctccaactc ctaatctcag gtgatctacc caccttggcc tcccaaattg 3000
   ctgggattac aggcgtgaac cactgctccc ttccctgtcc ttctgatttt aaaataacta 3060
   taccagcagg aggacgtcca gacacagcat aggctacctg gccatgccca accggtggga 3120
   catttgagtt gcttgcttgg cactgtcctc tcatgcgttg ggtccactca gtagatgcct 3180
   gttgaattgg gtacgcggcc agcttggctg tggaatgtgt gtcagttagg gtgtggaaag 3240
   tccccaggct ccccagcagg cagaagtatg caaagcatgc atctcaatta gtcagcaacc 3300
   aggtgtggaa agtccccagg ctccccagca ggcagaagta tgcaaagcat gcatctcaat 3360
   tagtcagcaa ccatagtccc gcccctaact ccgcccatcc cgcccctaac tccgcccagt 3420
   tccgcccatt ctccgcccca tggctgacta atttttttta tttatgcaga ggccgaggcc 3480
   gcctcggcct ctgagctatt ccagaagtag tgaggaggct tttttggagg cctaggcttt 3540
   tgcaaaaagc tcctcgactg cattaatgaa tcggccaacg cgcggggaga ggcggtttgc 3600
   gtattgggcg ctcttccgct tcctcgctca ctgactcgct gcgctcggtc gttcggctgc 3660
   ggcgagcggt atcagctcac tcaaaggcgg taatacggtt atccacagaa tcaggggata 3720
   acgcaggaaa gaacatgtga gcaaaaggcc agcaaaaggc caggaaccgt aaaaaggccg 3780
   cgttgctggc gtttttccat aggctccgcc cccctgacga gcatcacaaa aatcgacgct 3840
   caagtcagag gtggcgaaac ccgacaggac tataaagata ccaggcgttt ccccctggaa 3900
   gctccctcgt gcgctctcct gttccgaccc tgccgcttac cggatacctg tccgcctttc 3960
   tcccttcggg aagcgtggcg ctttctcata gctcacgctg taggtatctc agttcggtgt 4020
   aggtcgttcg ctccaagctg ggctgtgtgc acgaaccccc cgttcagccc gaccgctgcg 4080
   ccttatccgg taactatcgt cttgagtcca acccggtaag acacgactta tcgccactgg 4140
   cagcagccac tggtaacagg attagcagag cgaggtatgt aggcggtgct acagagttct 4200
   tgaagtggtg gcctaactac ggctacacta gaagaacagt atttggtatc tgcgctctgc 4260
   tgaagccagt taccttcgga aaaagagttg gtagctcttg atccggcaaa caaaccaccg 4320
   ctggtagcgg tggttttttt gtttgcaagc agcagattac gcgcagaaaa aaaggatctc 4380
   aagaagatcc tttgatcttt tctacggggt ctgacgctca gtggaacgaa aactcacgtt 4440
   aagggatttt ggtcatgaga ttatcaaaaa ggatcttcac ctagatcctt ttaaattaaa 4500
   aatgaagttt taaatcaatc taaagtatat atgagtaaac ttggtctgac agttaccaat 4560
   gcttaatcag tgaggcacct atctcagcga tctgtctatt tcgttcatcc atagttgcct 4620
   gactccccgt cgtgtagata actacgatac gggagggctt accatctggc cccagtgctg 4680
   caatgatacc gcgagaccca cgctcaccgg ctccagattt atcagcaata aaccagccag 4740
   ccggaagggc cgagcgcaga agtggtcctg caactttatc cgcctccatc cagtctatta 4800
   attgttgccg ggaagctaga gtaagtagtt cgccagttaa tagtttgcgc aacgttgttg 4860
   ccattgctac aggcatcgtg gtgtcacgct cgtcgtttgg tatggcttca ttcagctccg 4920
   gttcccaacg atcaaggcga gttacatgat cccccatgtt gtgcaaaaaa gcggttagct 4980
   ccttcggtcc tccgatcgtt gtcagaagta agttggccgc agtgttatca ctcatggtta 5040
   tggcagcact gcataattct cttactgtca tgccatccgt aagatgcttt tctgtgactg 5100
   gtgagtactc aaccaagtca ttctgagaat agtgtatgcg gcgaccgagt tgctcttgcc 5160
   cggcgtcaat acgggataat accgcgccac atagcagaac tttaaaagtg ctcatcattg 5220
   gaaaacgttc ttcggggcga aaactctcaa ggatcttacc gctgttgaga tccagttcga 5280
   tgtaacccac tcgtgcaccc aactgatctt cagcatcttt tactttcacc agcgtttctg 5340
   ggtgagcaaa aacaggaagg caaaatgccg caaaaaaggg aataagggcg acacggaaat 5400
   gttgaatact catactcttc ctttttcaat attattgaag catttatcag ggttattgtc 5460
   tcatgagcgg atacatattt gaatgtattt agaaaaataa acaaataggg gttccgcgca 5520
   catttccccg aaaagtgcca cctgacgcgc cctgtagcgg cgcattaagc gcggcgggtg 5580
   tggtggttac gcgcagcgtg accgctacac ttgccagcgc cctagcgccc gctcctttcg 5640
   ctttcttccc ttcctttctc gccacgttcg ccggctttcc ccgtcaagct ctaaatcggg 5700
   ggctcccttt agggttccga tttagtgctt tacggcacct cgaccccaaa aaacttgatt 5760
   agggtgatgg ttcacgtagt gggccatcgc cctgatagac ggtttttcgc cctttgacgt 5820
   tggagtccac gttctttaat agtggactct tgttccaaac tggaacaaca ctcaacccta 5880
   tctcggtcta ttcttttgat ttataaggga ttttgccgat ttcggcctat tggttaaaaa 5940
   atgagctgat ttaacaaaaa tttaacgcga attttaacaa aatatt 5986
<210> 22
   <211> 6723
   <212> DNA
   <213> Artificial sequence
<220>
   <223> vector E518 with cAMP response element and minimal promoter driving CD19
<400> 22
   gacggatcgg gagatctccc gatcccctat ggtgcactct cagtacaatc tgctctgatg       60
   ccgcatagtt aagccagtat ctgctccctg cttgtgtgtt ggaggtcgct gagtagtgcg      120
   cgagcaaaat ttaagctaca acaaggcaag gcttgaccga caattgcatg aagaatctgc      180
   ttagggttag gcgttttgcg ctgcttcgcg atgtacgggc cagatatacg cgtaataaaa      240
   tatctttatt ttcattacat ctgtgtgttg gttttttgtg tgaatcgata gtactaacat     300
   acgctctcca tcaaaacaaa acgaaacaaa acaaactagc aaaataggct gtccccagtg      360
   caagtgcagg tgccagaaca tttctctggc ctaactggcc ggtacctgag ctcgctagcg      420
   caccagacag tgacgtcagc tgccagatcc catggccgtc atactgtgac gtctttcaga      480
   caccccattg acgtcaatgg gagaacagat ctggcctcgg cggccaagct tagacactag      540
   agggtatata atggaagctc gacttccagc ttggcaatcc ggtactacta gcgccgccac      600
   catgccacct cctcgcctcc tcttcttcct cctcttcctc acccccatgg aagtcaggcc      660
   cgaggaacct ctagtggtga aggtggaaga gggagataac gctgtgctgc agtgcctcaa      720
   ggggacctca gatggcccca ctcagcagct gacctggtct cgggagtccc cgcttaaacc      780
   cttcttaaaa ctcagcctgg ggctgccagg cctgggaatc cacatgaggc ccctggccat      840
   ctggcttttc atcttcaacg tctctcaaca gatggggggc ttctacctgt gccagccggg      900
   gcccccctct gagaaggcct ggcagcctgg ctggacagtc aatgtggagg gcagcgggga      960
   gctgttccgg tggaatgttt cggacctagg tggcctgggc tgtggcctga agaacaggtc     1020
   ctcagagggc cccagctccc cttccgggaa gctcatgagc cccaagctgt atgtgtgggc     1080
   caaagaccgc cctgagatct gggagggaga gcctccgtgt ctcccaccga gggacagcct     1140
   gaaccagagc ctcagccagg acctcaccat ggcccctggc tccacactct ggctgtcctg     1200
   tggggtaccc cctgactctg tgtccagggg ccccctctcc tggacccatg tgcaccccaa      1260
   ggggcctaag tcattgctga gcctagagct gaaggacgat cgcccggcca gagatatgtg     1320
   ggtaatggag acgggtctgt tgttgccccg ggccacagct caagacgctg gaaagtatta     1380
   ttgtcaccgt ggcaacctga ccatgtcatt ccacctggag atcactgctc ggccagtact     1440
   atggcactgg ctgctgagga ctggtggctg gaaggtctca gctgtgactt tggcttatct     1500
   gatcttctgc ctgtgttccc ttgtgggcat tcttcatctt caaagagccc tggtcctgag     1560
   gaggaaaaga aagcgaatga ctgaccccac caggagattc ttcaaagtga cgcctccccc     1620
   aggaagcggg ccccagaacc agtacgggaa cgtgctgtct ctccccacac ccacctcagg     1680
   cctcggacgc gcccagcgtt gggccgcagg cctggggggc actgccccgt cttatggaaa      1740
   cccgagcagc gacgtccagg cggatggagc cttggggtcc cggagcccgc cgggagtggg     1800
   cccagaagaa gaggaagggg agggctatga ggaacctgac agtgaggagg actccgagtt     1860
   ctatgagaac gactccaacc ttgggcagga ccagctctcc caggatggca gcggctacga     1920
   gaaccctgag gatgagcccc tgggtcctga ggatgaagac tccttctcca acgctgagtc     1980
   ttatgagaac gaggatgaag agctgaccca gccggtcgcc aggacaatgg acttcctgag     2040
   ccctcatggg tcagcctggg accccagccg ggaagcaacc tccctggggt cccagtccta     2100
   tgaggatatg agaggaatcc tgtatgcagc cccccagctc cgctccattc ggggccagcc      2160
   tggacccaat catgaggaag atgcagactc ttatgagaac atggataatc ccgatgggcc     2220
   agacccagcc tggggaggag gggccgcatg gggcacctgg agcaccaggt gagcggccgc      2280
   tcgagtctag agggcccgtt taaacccgct gatcagcctc gactgtgcct tctagttgcc 2340
   agccatctgt tgtttgcccc tcccccgtgc cttccttgac cctggaaggt gccactccca 2400
   ctgtcctttc ctaataaaat gaggaaattg catcgcattg tctgagtagg tgtcattcta 2460
   ttctgggggg tggggtgggg caggacagca agggggagga ttgggaagac aatagcaggc 2520
   atgctgggga tgcggtgggc tctatggctt ctgaggcgga aagaaccagc tggggctcta 2580
   gggggtatcc ccacgcgccc tgtagcggcg cattaagcgc ggcgggtgtg gtggttacgc 2640
   gcagcgtgac cgctacactt gccagcgccc tagcgcccgc tcctttcgct ttcttccctt 2700
   cctttctcgc cacgttcgcc ggctttcccc gtcaagctct aaatcggggg ctccctttag 2760
   ggttccgatt tagtgcttta cggcacctcg accccaaaaa acttgattag ggtgatggtt 2820
   cacgtagtgg gccatcgccc tgatagacgg tttttcgccc tttgacgttg gagtccacgt 2880
   tctttaatag tggactcttg ttccaaactg gaacaacact caaccctatc tcggtctatt 2940
   cttttgattt ataagggatt ttgccgattt cggcctattg gttaaaaaat gagctgattt 3000
   aacaaaaatt taacgcgaat taattctgtg gaatgtgtgt cagttagggt gtggaaagtc 3060
   cccaggctcc ccagcaggca gaagtatgca aagcatgcat ctcaattagt cagcaaccag 3120
   gtgtggaaag tccccaggct ccccagcagg cagaagtatg caaagcatgc atctcaatta 3180
   gtcagcaacc atagtcccgc ccctaactcc gcccatcccg cccctaactc cgcccagttc 3240
   cgcccattct ccgccccatg gctgactaat tttttttatt tatgcagagg ccgaggccgc 3300
   ctctgcctct gagctattcc agaagtagtg aggaggcttt tttggaggcc taggcttttg 3360
   caaaaagctc ccgggagctt gtatatccat tttcggatct gatcaagaga caggatgagg 3420
   atcgtttcgc atgattgaac aagatggatt gcacgcaggt tctccggccg cttgggtgga 3480
   gaggctattc ggctatgact gggcacaaca gacaatcggc tgctctgatg ccgccgtgtt 3540
   ccggctgtca gcgcaggggc gcccggttct ttttgtcaag accgacctgt ccggtgccct 3600
   gaatgaactg caggacgagg cagcgcggct atcgtggctg gccacgacgg gcgttccttg 3660
   cgcagctgtg ctcgacgttg tcactgaagc gggaagggac tggctgctat tgggcgaagt 3720
   gccggggcag gatctcctgt catctcacct tgctcctgcc gagaaagtat ccatcatggc 3780
   tgatgcaatg cggcggctgc atacgcttga tccggctacc tgcccattcg accaccaagc 3840
   gaaacatcgc atcgagcgag cacgtactcg gatggaagcc ggtcttgtcg atcaggatga 3900
   tctggacgaa gagcatcagg ggctcgcgcc agccgaactg ttcgccaggc tcaaggcgcg 3960
   catgcccgac ggcgaggatc tcgtcgtgac ccatggcgat gcctgcttgc cgaatatcat 4020
   ggtggaaaat ggccgctttt ctggattcat cgactgtggc cggctgggtg tggcggaccg 4080
   ctatcaggac atagcgttgg ctacccgtga tattgctgaa gagcttggcg gcgaatgggc 4140
   tgaccgcttc ctcgtgcttt acggtatcgc cgctcccgat tcgcagcgca tcgccttcta 4200
   tcgccttctt gacgagttct tctgagcggg actctggggt tcgaaatgac cgaccaagcg 4260
   acgcccaacc tgccatcacg agatttcgat tccaccgccg ccttctatga aaggttgggc 4320
   ttcggaatcg ttttccggga cgccggctgg atgatcctcc agcgcgggga tctcatgctg 4380
   gagttcttcg cccaccccaa cttgtttatt gcagcttata atggttacaa ataaagcaat 4440
   agcatcacaa atttcacaaa taaagcattt ttttcactgc attctagttg tggtttgtcc 4500
   aaactcatca atgtatctta tcatgtctgt ataccgtcga cctctagcta gagcttggcg 4560
   taatcatggt catagctgtt tcctgtgtga aattgttatc cgctcacaat tccacacaac 4620
   atacgagccg gaagcataaa gtgtaaagcc tggggtgcct aatgagtgag ctaactcaca 4680
   ttaattgcgt tgcgctcact gcccgctttc cagtcgggaa acctgtcgtg ccagctgcat 4740
   taatgaatcg gccaacgcgc ggggagaggc ggtttgcgta ttgggcgctc ttccgcttcc 4800
   tcgctcactg actcgctgcg ctcggtcgtt cggctgcggc gagcggtatc agctcactca 4860
   aaggcggtaa tacggttatc cacagaatca ggggataacg caggaaagaa catgtgagca 4920
   aaaggccagc aaaaggccag gaaccgtaaa aaggccgcgt tgctggcgtt tttccatagg 4980
   ctccgccccc ctgacgagca tcacaaaaat cgacgctcaa gtcagaggtg gcgaaacccg 5040
   acaggactat aaagatacca ggcgtttccc cctggaagct ccctcgtgcg ctctcctgtt 5100
   ccgaccctgc cgcttaccgg atacctgtcc gcctttctcc cttcgggaag cgtggcgctt 5160
   tctcatagct cacgctgtag gtatctcagt tcggtgtagg tcgttcgctc caagctgggc 5220
   tgtgtgcacg aaccccccgt tcagcccgac cgctgcgcct tatccggtaa ctatcgtctt 5280
   gagtccaacc cggtaagaca cgacttatcg ccactggcag cagccactgg taacaggatt 5340
   agcagagcga ggtatgtagg cggtgctaca gagttcttga agtggtggcc taactacggc 5400
   tacactagaa gaacagtatt tggtatctgc gctctgctga agccagttac cttcggaaaa 5460
   agagttggta gctcttgatc cggcaaacaa accaccgctg gtagcggttt ttttgtttgc 5520
   aagcagcaga ttacgcgcag aaaaaaagga tctcaagaag atcctttgat cttttctacg 5580
   gggtctgacg ctcagtggaa cgaaaactca cgttaaggga ttttggtcat gagattatca 5640
   aaaaggatct tcacctagat ccttttaaat taaaaatgaa gttttaaatc aatctaaagt 5700
   atatatgagt aaacttggtc tgacagttac caatgcttaa tcagtgaggc acctatctca 5760
   gcgatctgtc tatttcgttc atccatagtt gcctgactcc ccgtcgtgta gataactacg 5820
   atacgggagg gcttaccatc tggccccagt gctgcaatga taccgcgaga cccacgctca 5880
   ccggctccag atttatcagc aataaaccag ccagccggaa gggccgagcg cagaagtggt 5940
   cctgcaactt tatccgcctc catccagtct attaattgtt gccgggaagc tagagtaagt 6000
   agttcgccag ttaatagttt gcgcaacgtt gttgccattg ctacaggcat cgtggtgtca 6060
   cgctcgtcgt ttggtatggc ttcattcagc tccggttccc aacgatcaag gcgagttaca      6120
   tgatccccca tgttgtgcaa aaaagcggtt agctccttcg gtcctccgat cgttgtcaga      6180
   agtaagttgg ccgcagtgtt atcactcatg gttatggcag cactgcataa ttctcttact      6240
   gtcatgccat ccgtaagatg cttttctgtg actggtgagt actcaaccaa gtcattctga      6300
   gaatagtgta tgcggcgacc gagttgctct tgcccggcgt caatacggga taataccgcg      6360
   ccacatagca gaactttaaa agtgctcatc attggaaaac gttcttcggg gcgaaaactc      6420
   tcaaggatct taccgctgtt gagatccagt tcgatgtaac ccactcgtgc acccaactga      6480
   tcttcagcat cttttacttt caccagcgtt tctgggtgag caaaaacagg aaggcaaaat      6540
   gccgcaaaaa agggaataag ggcgacacgg aaatgttgaa tactcatact cttccttttt      6600
   caatattatt gaagcattta tcagggttat tgtctcatga gcggatacat atttgaatgt      6660
   atttagaaaa ataaacaaat aggggttccg cgcacatttc cccgaaaagt gccacctgac      6720
   gtc                                                                    6723
<210> 23
   <211> 475
   <212> DNA
   <213> Artificial sequence
<220>
   <223> ITS007-V105 (VH sequence through the end of the JH)
<400> 23
   caggtcacac tgagagagtc cggccctgcc ctggtgaaac ccacccagac cctgaccctg        60
   acatgcacct tcagcggctt cagcctgagc accagcggga tgtgcgtgtc ctggattcga       120
   cagccccctg gcaaggccct ggaatggctg gccctgattg actgggacga cgacaagtac      180
   tacagcacca gcctgaaaac ccggctgacc atcagcaagg acaccagcaa gaaccaggtg       240
   gtgctgacca tgaccaacat ggaccccgtg gacaccgcca cgtattactg tgcacggcat       300
   ggcgagggca ccttcacctc cgacctgtcc aaacaaatgg aagaagaagc cgtccggctg       360
   ttcatcgaat ggctgaaaaa tggcggccct tcctctggcg cccctcctcc ttcttcgctg       420
   caggatgatg cttttgatat ctggggccaa gggacaatgg tcaccgtgtc ctcag            475
<210> 24
   <211> 158
   <212> PRT
   <213> Artificial sequence
<220>
   <223> ITS007-V105 (VH sequence through the end of the JH)
<400> 24
   Gln Val Thr Leu Arg Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Met Cys Val Ser Trp Ile Arg Gln Pro Pro Gly Lys Ala Leu Glu
           35                  40                  45
   Trp Leu Ala Leu Ile Asp Trp Asp Asp Asp Lys Tyr Tyr Ser Thr Ser
       50                  55                  60
   Leu Lys Thr Arg Leu Thr Ile Ser Lys Asp Thr Ser Lys Asn Gln Val
   65                  70                  75                  80
   Val Leu Thr Met Thr Asn Met Asp Pro Val Asp Thr Ala Thr Tyr Tyr
                   85                  90                  95
   Cys Ala Arg His Gly Glu Gly Thr Phe Thr Ser Asp Leu Ser Lys Gln
               100                 105                 110
   Met Glu Glu Glu Ala Val Arg Leu Phe Ile Glu Trp Leu Lys Asn Gly
           115                 120                 125
   Gly Pro Ser Ser Gly Ala Pro Pro Pro Ser Ser Leu Gln Asp Asp Ala
       130                 135                 140
   Phe Asp Ile Trp Gly Gln Gly Thr Met Val Thr Val Ser Ser
   145                 150                 155
<210> 25
   <211> 472
   <212> DNA
   <213> Artificial sequence
<220>
   <223> ITS007-V107 (VH sequence through the end of the JH)
<400> 25
   gaagtgcagc tggtggaaag cggcggaggc ctggtgcagc ccggtcgaag cctgagactg        60
   agctgcgccg ccagcggctt cacctttgac gactacgcca tgcactgggt ccgccaggcc       120
   cctggcaagg gactggaatg ggtgtccggc atcagctgga acagcggcag catcggctac       180
   gccgacagcg tgaagggccg gttcaccatc agccgggaca acgccaagaa cagcctgtac       240
   ctgcagatga acagcctgcg ggccgaggac accgccttgt attactgtgc gagagatgag       300
   cggcatggcg agggcacctt cacctccgac ctgtccaaac aaatggaaga agaagccgtc       360
   cggctgttca tcgaatggct gaaaaatggc ggcccttcct ctggcgcccc tcctccttct       420
   gtggctgctt ttgatatctg gggccaaggg acaatggtca ccgtgtcctc ag               472
<210> 26
   <211> 157
   <212> PRT
   <213> Artificial sequence
<220>
   <223> ITS007-V107 (VH sequence through the end of the JH)
<400> 26
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ser Gly Ala Pro Pro Pro Ser Val Ala Ala Phe
       130                 135                 140
   Asp Ile Trp Gly Gln Gly Thr Met Val Thr Val Ser Ser
   145                 150                 155
<210> 27
   <211> 41
   <212> DNA
   <213> Artificial sequence
<220>
   <223> primer
<400> 27
   gagagtttgg atcccaactt tcttgtccac cttggtgttg c 41
<210> 28
   <211> 50
   <212> DNA
   <213> Artificial sequence
<220>
   <223> primer
<400> 28
   gagagatttg gtctctatgt cgatctgatc aagagacagg ataaggagcc 50
<210> 29
   <211> 41
   <212> DNA
   <213> Artificial sequence
<220>
   <223> primer
<400> 29
   gagagagatt ggtctcgcct gagttccacg acaccgtcac c 41
<210> 30
   <211> 58
   <212> DNA
   <213> Artificial sequence
<220>
   <223> primer
<220>
   <221> misc_feature
   <222> (19)..(20)
   <223> n is a, c, g, or t
<400> 30
   ctacgaggtc tcactgtgnn kcatggcgag ggcaccttca cctccgacct gtccaaac 58
<210> 31
   <211> 55
   <212> DNA
   <213> Artificial sequence
<220>
   <223> primer
<400> 31
   ttaagaggta ccctcgagca gacatgataa gatacattga tgagtttgga caaac 55
<210> 32
   <211> 62
   <212> PRT
   <213> Artificial sequence
<220>
   <223> ITS007-V105 exendin junction
<400> 32
   Cys Ala Arg His Gly Glu Gly Thr Phe Thr Ser Asp Leu Ser Lys Gln
   1               5                   10                  15
   Met Glu Glu Glu Ala Val Arg Leu Phe Ile Glu Trp Leu Lys Asn Gly
               20                  25                  30
   Gly Pro Ser Ser Gly Ala Pro Pro Pro Ser Ser Leu Gln Asp Asp Ala
           35                  40                  45
   Phe Asp Ile Trp Gly Gln Gly Thr Met Val Thr Val Ser Ser
       50                  55                  60
<210> 33
   <211> 62
   <212> PRT
   <213> Artificial sequence
<220>
   <223> ITS007-V107 exendin junction
<400> 33
   Cys Ala Arg Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala Ala
           35                  40                  45
   Phe Asp Ile Trp Gly Gln Gly Thr Met Val Thr Val Ser Ser
       50                  55                  60
<210> 34
   <211> 37
   <212> PRT
   <213> Homo sapiens
<220>
   <221> misc_feature
   <223> Oxyntomodulin
<400> 34
   His Ser Gln Gly Thr Phe Thr Ser Asp Tyr Ser Lys Tyr Leu Asp Ser
   1               5                   10                  15
   Arg Arg Ala Gln Asp Phe Val Gln Trp Leu Met Asn Thr Lys Arg Asn
               20                  25                  30
   Arg Asn Asn Ile Ala
   35
<210> 35
   <211> 87
   <212> DNA
   <213> Artificial sequence
<220>
   <223> sequence encoding glucagon
<400> 35
   cattcacagg gcacattcac cagtgactac agcaagtatc tggactccag gcgtgcccaa 60
   gattttgtgc agtggttgat gaatacc 87
<210> 36
   <211> 111
   <212> DNA
   <213> Artificial sequence
<220>
   <223> sequence encoding oxyntomodulin
<400> 36
   cattcacagg gcacattcac cagtgactac agcaagtatc tggactccag gcgtgcccaa 60
   gattttgtgc agtggttgat gaataccaag aggaacagga ataacattgc c 111
<210> 37
   <211> 42
   <212> PRT
   <213> Artificial sequence
<220>
   <223> ITS011-V36 glucagon junction
<400> 37
   Cys Thr Thr Asp Leu Ser Arg Arg Arg His Ser Gln Gly Thr Phe Thr
   1 5 10 15
   Ser Asp Tyr Ser Lys Tyr Leu Asp Ser Arg Arg Ala Gln Asp Phe Val
   20 25 30
   Gln Trp Leu Met Asn Thr Leu Pro Met Pro
   35 40
<210> 38
   <211> 41
   <212> PRT
   <213> Artificial sequence
<220>
   <223> ITS011-V66 glucagon junction
<400> 38
   Cys Thr Thr Asp Pro Val Leu Lys His Ser Gln Gly Thr Phe Thr Ser
   1               5                   10                  15
   Asp Tyr Ser Lys Tyr Leu Asp Ser Arg Arg Ala Gln Asp Phe Val Gln
               20                  25                  30
   Trp Leu Met Asn Thr Leu Ala Pro Val
           35                  40
<210> 39
   <211> 43
   <212> PRT
   <213> Artificial sequence
<220>
   <223> ITS011-V69 glucagon junction
<400> 39
   Cys Ala Arg Ala Glu Ser Ala Gly Arg His Ser Gln Gly Thr Phe Thr
   1               5                   10                  15
   Ser Asp Tyr Ser Lys Tyr Leu Asp Ser Arg Arg Ala Gln Asp Phe Val
               20                  25                  30
   Gln Trp Leu Met Asn Thr Thr Ser Met Val Arg
           35                  40
<210> 40
   <211> 45
   <212> PRT
   <213> Artificial sequence
<220>
   <223> ITS011-V23 oxyntomodulin junction
<400> 40
   Cys Ala Ser Asn Arg His Ser Gln Gly Thr Phe Thr Ser Asp Tyr Ser
   1               5                   10                  15
   Lys Tyr Leu Asp Ser Arg Arg Ala Gln Asp Phe Val Gln Trp Leu Met
               20                  25                  30
   Asn Thr Lys Arg Asn Arg Asn Asn Ile Ala Met Leu Leu
           35                  40                  45
<210> 41
   <211> 50
   <212> PRT
   <213> Artificial sequence
<220>
   <223> ITS011-V74 oxyntomodulin junction
<400> 41
   Cys Ala Arg Asp Lys Gly Ile Lys His Ser Gln Gly Thr Phe Thr Ser
   1               5                   10                  15
   Asp Tyr Ser Lys Tyr Leu Asp Ser Arg Arg Ala Gln Asp Phe Val Gln
               20                  25                  30
   Trp Leu Met Asn Thr Lys Arg Asn Arg Asn Asn Ile Ala Ser Lys Gln
           35                  40                  45
   Val Arg
       50
<210> 42
   <211> 58
   <212> PRT
   <213> Artificial sequence
<220>
   <223> ITS011-V100 oxyntomodulin junction
<400> 42
   Cys Ala Arg Asp Leu Ser Asp Leu Ser His Ser Val Leu Leu Arg His
   1               5                   10                  15
   Ser Gln Gly Thr Phe Thr Ser Asp Tyr Ser Lys Tyr Leu Asp Ser Arg
               20                  25                  30
   Arg Ala Gln Asp Phe Val Gln Trp Leu Met Asn Thr Lys Arg Asn Arg
           35                  40                  45
   Asn Asn Ile Ala Arg Pro Leu Val Leu Ser
       50                  55
<210> 43
   <211> 28
   <212> PRT
   <213> Homo sapiens
<220>
   <221> misc_feature
   <223> Vasoactive intestinal peptide
<400> 43
   His Ser Asp Ala Val Phe Thr Asp Asn Tyr Thr Arg Leu Arg Lys Gln
   1               5                   10                  15
   Met Ala Val Lys Lys Tyr Leu Asn Ser Ile Leu Asn
                       20                  25
<210> 44
   <211> 27
   <212> PRT
   <213> Homo sapiens
<220>
   <221> misc_feature
   <223> PACAP-27
<400> 44
   His Ser Asp Gly Ile Phe Thr Asp Ser Tyr Ser Arg Tyr Arg Lys Gln
   1               5                   10                  15
   Met Ala Val Lys Lys Tyr Leu Ala Ala Val Leu
               20                  25
<210> 45
   <211> 35
   <212> PRT
   <213> Heloderma suspectum
<220>
   <221> misc_feature
   <223> helodermin
<400> 45
   His Ser Asp Ala Ile Phe Thr Glu Glu Tyr Ser Lys Leu Leu Ala Lys
   1               5                   10                  15
   Leu Ala Leu Gln Lys Tyr Leu Ala Ser Ile Leu Gly Ser Arg Thr Ser
               20                  25                  30
   Pro Pro Pro
           35
<210> 46
   <211> 27
   <212> PRT
   <213> Homo sapiens
<220>
   <221> misc_feature
   <223> Peptide histidine methionine
<400> 46
   His Ala Asp Gly Val Phe Thr Ser Asp Phe Ser Lys Leu Leu Gly Gln
   1               5                   10                  15
   Leu Ser Ala Lys Lys Tyr Leu Glu Ser Leu Met
               20                  25
<210> 47
   <211> 27
   <212> PRT
   <213> Homo sapiens
<220>
   <221> misc_feature
   <223> secretin
<400> 47
   His Ser Asp Gly Thr Phe Thr Ser Glu Leu Ser Arg Leu Arg Glu Gly
   1               5                   10                  15
   Ala Arg Leu Gln Arg Leu Leu Gln Gly Leu Val
               20                  25
<210> 48
   <211> 44
   <212> PRT
   <213> Homo sapiens
<220>
   <221> misc_feature
   <223> Growth hormone releasing factor
<400> 48
   Tyr Ala Asp Ala Ile Phe Thr Asn Ser Tyr Arg Lys Val Leu Gly Gln
   1               5                   10                  15
   Leu Ser Ala Arg Lys Leu Leu Gln Asp Ile Met Ser Arg Gln Gln Gly
               20                  25                  30
   Glu Ser Asn Gln Glu Arg Gly Ala Arg Ala Arg Leu
           35                  40
<210> 49
   <211> 42
   <212> PRT
   <213> Homo sapiens
<220>
   <221> misc_feature
   <223> Gastric inhibitory peptide
<400> 49
   Tyr Ala Glu Gly Thr Phe Ile Ser Asp Tyr Ser Ile Ala Met Asp Lys
   1               5                   10                  15
   Ile His Gln Gln Asp Phe Val Asn Trp Leu Leu Ala Gln Lys Gly Lys
               20                  25                  30
   Lys Asn Asp Trp Lys His Asn Ile Thr Gln
           35                  40
<210> 50
   <211> 1224
   <212> DNA
   <213> Artificial sequence
<220>
   <223> ITS007-V125
<400> 50
   atggagtttg ggctgagctg gctttttctt gtggctattt taaaaggtgt ccagtgtgaa      60
   gtgcagctgg tggaaagcgg cggaggcctg gtgcagcccg gtcgaagcct gagactgagc     120
   tgcgccgcca gcggcttcac ctttgacgac tacgccatgc actgggtccg ccaggcccct     180
   ggcaagggac tggaatgggt gtccggcatc agctggaaca gcggcagcat cggctacgcc     240
   gacagcgtga agggccggtt caccatcagc cgggacaacg ccaagaacag cctgtacctg     300
   cagatgaaca gcctgcgggc cgaggacacc gccttgtatt actgtgcgag agatgagcgg     360
   catggcgagg gcaccttcac ctccgacctg tccaaacaaa tggaagaaga agccgtccgg     420
   ctgttcatcg aatggctgaa aaatggcggc ccttcctctg gcgcccctcc tccttctgtg     480
   gctgcttttg atatctgggg ccaagggaca atggtcaccg tgtcctcaga gcccaaatct     540
   tgtgacaaaa ctcacacatg cccaccgtgc ccagcacctg aactcctggg gggaccgtca     600
   gtcttcctct tccccccaaa acccaaggac accctcatga tctctagaac ccctgaggtc     660
   acatgcgtgg tggtggacgt gagccacgaa gaccctgagg tcaagttcaa ctggtacgtg     720
   gacggcgtgg aggtgcataa tgccaagaca aagccgcggg aggagcagta caacagcacg     780
   taccgtgtgg tcagcgtcct caccgtcctg caccaggact ggctgaatgg caaggagtac     840
   aagtgcaagg tgtccaacaa agccctccca gcccccatcg agaaaaccat ctccaaagcc     900
   aaagggcagc cccgagaacc acaggtgtac accctgcccc catcccggga tgagctgacc     960
   aagaaccagg tcagcctgac ctgcctggtc aaaggcttct atcccagcga catcgccgtg    1020
   gagtgggaga gcaatgggca gccggagaac aactacaaga ccacgcctcc cgtgctggac    1080
   tccgacggct ccttcttcct ctacagcaag ctcaccgtgg acaagagcag gtggcagcag    1140
   gggaacgtct tctcatgctc cgtgatgcat gaggctctgc acaaccacta cacgcagaag    1200
   agcctctccc tgtctccggg caaa                                           1224
<210> 51
   <211> 408
   <212> PRT
   <213> Artificial sequence
<220>
   <223> ITS007-V125
<400> 51
   Met Glu Phe Gly Leu Ser Trp Leu Phe Leu Val Ala Ile Leu Lys Gly
   1               5                   10                  15
   Val Gln Cys Glu Val Gln Leu Val Glu Ser Gly Gly Gly Leu Val Gln
               20                  25                  30
   Pro Gly Arg Ser Leu Arg Leu Ser Cys Ala Ala Ser Gly Phe Thr Phe
           35                  40                  45
   Asp Asp Tyr Ala Met His Trp Val Arg Gln Ala Pro Gly Lys Gly Leu
       50                  55                  60
   Glu Trp Val Ser Gly Ile Ser Trp Asn Ser Gly Ser Ile Gly Tyr Ala
   65                  70                  75                  80
   Asp Ser Val Lys Gly Arg Phe Thr Ile Ser Arg Asp Asn Ala Lys Asn
                   85                  90                  95
   Ser Leu Tyr Leu Gln Met Asn Ser Leu Arg Ala Glu Asp Thr Ala Leu
               100                 105                 110
   Tyr Tyr Cys Ala Arg Asp Glu Arg His Gly Glu Gly Thr Phe Thr Ser
           115                 120                 125
   Asp Leu Ser Lys Gln Met Glu Glu Glu Ala Val Arg Leu Phe Ile Glu
       130                 135                 140
   Trp Leu Lys Asn Gly Gly Pro Ser Ser Gly Ala Pro Pro Pro Ser Val
   145                 150                 155                 160
   Ala Ala Phe Asp Ile Trp Gly Gln Gly Thr Met Val Thr Val Ser Ser
                   165                 170                 175
   Glu Pro Lys Ser Cys Asp Lys Thr His Thr Cys Pro Pro Cys Pro Ala
               180                 185                 190
   Pro Glu Leu Leu Gly Gly Pro Ser Val Phe Leu Phe Pro Pro Lys Pro
           195                 200                 205
   Lys Asp Thr Leu Met Ile Ser Arg Thr Pro Glu Val Thr Cys Val Val
       210                 215                 220
   Val Asp Val Ser His Glu Asp Pro Glu Val Lys Phe Asn Trp Tyr Val
   225 230 235 240
   Asp Gly Val Glu Val His Asn Ala Lys Thr Lys Pro Arg Glu Glu Gln
   245 250 255
   Tyr Asn Ser Thr Tyr Arg Val Val Ser Val Leu Thr Val Leu His Gln
   260 265 270
   Asp Trp Leu Asn Gly Lys Glu Tyr Lys Cys Lys Val Ser Asn Lys Ala
   275 280 285
   Leu Pro Ala Pro Ile Glu Lys Thr Ile Ser Lys Ala Lys Gly Gln Pro
   290 295 300
   Arg Glu Pro Gln Val Tyr Thr Leu Pro Pro Ser Arg Asp Glu Leu Thr
   305 310 315 320
   Lys Asn Gln Val Ser Leu Thr Cys Leu Val Lys Gly Phe Tyr Pro Ser
   325 330 335
   Asp Ile Ala Val Glu Trp Glu Ser Asn Gly Gln Pro Glu Asn Asn Tyr
   340 345 350
   Lys Thr Thr Pro Pro Val Leu Asp Ser Asp Gly Ser Phe Phe Leu Tyr
   355 360 365
   Ser Lys Leu Thr Val Asp Lys Ser Arg Trp Gln Gln Gly Asn Val Phe
   370 375 380
   Ser Cys Ser Val Met His Glu Ala Leu His Asn His Tyr Thr Gln Lys
   385 390 395 400
   Ser Leu Ser Leu Ser Pro Gly Lys
   405
<210> 52
   <211> 4917
   <212> DNA
   <213> Artificial sequence
<220>
   <223> ITS007-V248
<400> 52
   tcgagttaat taactggcct catgggcctt ccgctcactg cccgctttcc agtcgggaaa      60
   cctgtcgtgc cagctgcatt aacatggtca tagctgtttc cttgcgtatt gggcgctctc     120
   cgcttcctcg ctcactgact cgctgcgctc ggtcgttcgg gtaaagcctg gggtgcctaa     180
   tgagcaaaag gccagcaaaa ggccaggaac cgtaaaaagg ccgcgttgct ggcgtttttc     240
   cataggctcc gcccccctga cgagcatcac aaaaatcgac gctcaagtca gaggtggcga     300
   aacccgacag gactataaag ataccaggcg tttccccctg gaagctccct cgtgcgctct     360
   cctgttccga ccctgccgct taccggatac ctgtccgcct ttctcccttc gggaagcgtg     420
   gcgctttctc atagctcacg ctgtaggtat ctcagttcgg tgtaggtcgt tcgctccaag     480
   ctgggctgtg tgcacgaacc ccccgttcag cccgaccgct gcgccttatc cggtaactat     540
   cgtcttgagt ccaacccggt aagacacgac ttatcgccac tggcagcagc cactggtaac     600
   aggattagca gagcgaggta tgtaggcggt gctacagagt tcttgaagtg gtggcctaac     660
   tacggctaca ctagaagaac agtatttggt atctgcgctc tgctgaagcc agttaccttc     720
   ggaaaaagag ttggtagctc ttgatccggc aaacaaacca ccgctggtag cggtggtttt     780
   tttgtttgca agcagcagat tacgcgcaga aaaaaaggat ctcaagaaga tcctttgatc     840
   ttttctacgg ggtctgacgc tcagtggaac gaaaactcac gttaagggat tttggtcatg     900
   agattatcaa aaaggatctt cacctagatc cttttaaatt aaaaatgaag ttttaaatca     960
   atctaaagta tatatgagta aacttggtct gacagttacc aatgcttaat cagtgaggca    1020
   cctatctcag cgatctgtct atttcgttca tccatagttg cctgactccc cgtcgtgtag    1080
   ataactacga tacgggaggg cttaccatct ggccccagtg ctgcaatgat accgcgagaa    1140
   ccacgctcac cggctccaga tttatcagca ataaaccagc cagccggaag ggccgagcgc    1200
   agaagtggtc ctgcaacttt atccgcctcc atccagtcta ttaattgttg ccgggaagct    1260
   agagtaagta gttcgccagt taatagtttg cgcaacgttg ttgccattgc tacaggcatc    1320
   gtggtgtcac gctcgtcgtt tggtatggct tcattcagct ccggttccca acgatcaagg    1380
   cgagttacat gatcccccat gttgtgcaaa aaagcggtta gctccttcgg tcctccgatc    1440
   gttgtcagaa gtaagttggc cgcagtgtta tcactcatgg ttatggcagc actgcataat    1500
   tctcttactg tcatgccatc cgtaagatgc ttttctgtga ctggtgagta ctcaaccaag    1560
   tcattctgag aatagtgtat gcggcgaccg agttgctctt gcccggcgtc aatacgggat    1620
   aataccgcgc cacatagcag aactttaaaa gtgctcatca ttggaaaacg ttcttcgggg    1680
   cgaaaactct caaggatctt accgctgttg agatccagtt cgatgtaacc cactcgtgca    1740
   cccaactgat cttcagcatc ttttactttc accagcgttt ctgggtgagc aaaaacagga    1800
   aggcaaaatg ccgcaaaaaa gggaataagg gcgacacgga aatgttgaat actcatactc    1860
   ttcctttttc aatattattg aagcatttat cagggttatt gtctcatgag cggatacata    1920
   tttgaatgta tttagaaaaa taaacaaata ggggttccgc gcacatttcc ccgaaaagtg    1980
   ccacctaaat tgtaagcgtt aatattttgt taaaattcgc gttaaatttt tgttaaatca    2040
   gctcattttt taaccaatag gccgaaatcg gcaaaatccc ttataaatca aaagaataga 2100
   ccgagatagg gttgagtggc cgctacaggg cgctcccatt cgccattcag gctgcgcaac 2160
   tgttgggaag ggcgtttcgg tgcgggcctc ttcgctatta cgccagctgg cgaaaggggg 2220
   atgtgctgca aggcgattaa gttgggtaac gccagggttt tcccagtcac gacgttgtaa 2280
   aacgacggcc agtgagcgcg acgtaatacg actcactata gggcgaattg gcggaaggcc 2340
   gtcaaggcct acgcgccgac cgagtccact agttattaat agtaatcaat tacggggtca 2400
   ttagttcata gcccatatat ggagttccgc gttacataac ttacggtaaa tggcccgcct 2460
   ggctgaccgc ccaacgaccc ccgcccattg acgtcaataa tgacgtatgt tcccatagta 2520
   acgccaatag ggactttcca ttgacgtcaa tgggtggagt atttacggta aactgcccac 2580
   ttggcagtac atcaagtgta tcatatgcca agtacgcccc ctattgacgt caatgacggt 2640
   aaatggcccg cctggcatta tgcccagtac atgaccttat gggactttcc tacttggcag 2700
   tacatctacg tattagtcat cgctattacc atggtgatgc ggttttggca gtacatcaat 2760
   gggcgtggat agcggtttga ctcacgggga tttccaagtc tccaccccat tgacgtcaat 2820
   gggagtttgt tttggcacca aaatcaacgg gactttccaa aatgtcgtaa caactccgcc 2880
   ccattgacgc aaatgggcgg taggcgtgta cggtgggagg tctatataag cagagctctc 2940
   tggctaacta gagaacccac tgcttactgc tcgacgatct gatcaagaga caggataagg 3000
   agccgccacc atggagtttg ggctgagctg gctttttctt gtggctattt taaaaggtgt 3060
   ccagtgtgaa gtgcagctgg tggaaagcgg cggaggcctg gtgcagcccg gtcgaagcct 3120
   gagactgagc tgcgccgcca gcggcttcac ctttgacgac tacgccatgc actgggtccg 3180
   ccaggcccct ggcaagggac tggaatgggt gtccggcatc agctggaaca gcggcagcat 3240
   cggctacgcc gacagcgtga agggccggtt caccatcagc cgggacaacg ccaagaacag 3300
   cctgtacctg cagatgaaca gcctgcgggc cgaggacacc gccttgtatt actgtagcag 3360
   aaagaagcgg catggcgagg gcaccttcac ctccgacctg tccaaacaaa tggaagaaga 3420
   agccgtccgg ctgttcatcg aatggctgaa aaatggcggc ccttcctctg gcgcccctcc 3480
   tccttctgtg gctgcttttg atatctgggg ccaagggaca atggtcaccg tgtcctcaga 3540
   gcccaaatct tgtgacaaaa ctcacacatg cccaccgtgc ccagcacctg aactcctggg 3600
   gggaccgtca gtcttcctct tccccccaaa acccaaggac accctcatga tctctagaac 3660
   ccctgaggtc acatgcgtgg tggtggacgt gagccacgaa gaccctgagg tcaagttcaa 3720
   ctggtacgtg gacggcgtgg aggtgcataa tgccaagaca aagccgcggg aggagcagta 3780
   caacagcacg taccgtgtgg tcagcgtcct caccgtcctg caccaggact ggctgaatgg 3840
   caaggagtac aagtgcaagg tgtccaacaa agccctccca gcccccatcg agaaaaccat 3900
   ctccaaagcc aaagggcagc cccgagaacc acaggtgtac accctgcccc catcccggga 3960
   tgagctgacc aagaaccagg tcagcctgac ctgcctggtc aaaggcttct atcccagcga 4020
   catcgccgtg gagtgggaga gcaatgggca gccggagaac aactacaaga ccacgcctcc 4080
   cgtgctggac tccgacggct ccttcttcct ctacagcaag ctcaccgtgg acaagagcag 4140
   gtggcagcag gggaacgtct tctcatgctc cgtgatgcat gaggctctgc acaaccacta 4200
   cacgcagaag agcctctccc tgtctccggg caaatctggc gaagtgcagc tggtggaaag 4260
   cggcggaggc ctggtgcagc ccggtcgaag cctgagactg agctgcgccg ccagcggctt 4320
   cacctttgac gactacgcca tgcactgggt ccgccaggcc cctggcaagg gactggaatg 4380
   ggtgtccggc atcagctgga acagcggcag catcggctac gccgacagcg tgaagggccg 4440
   gttcaccatc agccgggaca acgccaagaa cagcctgtac ctgcagatga acagcctgcg 4500
   ggccgaggac accgccttgt attactgtag cagaaagaag cggcatggcg agggcacctt 4560
   cacctccgac ctgtccaaac aaatggaaga agaagccgtc cggctgttca tcgaatggct 4620
   gaaaaatggc ggcccttcct ctggcgcccc tcctccttct gtggctgctt ttgatatctg 4680
   gggccaaggg acaatggtca ccgtgtcctc ataggttttc cgggacgccg gctggatgat 4740
   cctccagcgc ggggatctca tgctggagtt cttcgcccac cccaacttgt ttattgcagc 4800
   ttataatggt tacaaataaa gcaatagcat cacaaatttc acaaataaag catttttttc 4860
   actgcattct agttgtggtt tgtccaaact catcaatgta tcttatcatg tctgacc 4917
<210> 53
   <211> 471
   <212> DNA
   <213> Artificial sequence
<220>
   <223> ITS007-V126 (from VH to the end of JH)
<400> 53
   gaagtgcagc tggtggaaag cggcggaggc ctggtgcagc ccggtcgaag cctgagactg 60
   agctgcgccg ccagcggctt cacctttgac gactacgcca tgcactgggt ccgccaggcc 120
   cctggcaagg gactggaatg ggtgtccggc atcagctgga acagcggcag catcggctac 180
   gccgacagcg tgaagggccg gttcaccatc agccgggaca acgccaagaa cagcctgtac 240
   ctgcagatga acagcctgcg ggccgaggac accgccttgt attactgtgc gaaggatgag 300
   cggcatggcg agggcacctt cacctccgac ctgtccaaac aaatggaaga agaagccgtc 360
   cggctgttca tcgaatggct gaaaaatggc ggcccttcct ctggcgcccc tcctccttct 420
   gtggctgctt ttgatatctg gggccaaggg acaatggtca ccgtgtcctc a 471
<210> 54
   <211> 157
   <212> PRT
   <213> Artificial sequence
<220>
   <223> ITS007-V126 (from VH to the end of JH)
<400> 54
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ser Gly Ala Pro Pro Pro Ser Val Ala Ala Phe
       130                 135                 140
   Asp Ile Trp Gly Gln Gly Thr Met Val Thr Val Ser Ser
   145                 150                 155
<210> 55
   <211> 471
   <212> DNA
   <213> Artificial sequence
<220>
   <223> ITS007-V127 (from VH to the end of JH)
<400> 55
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gagagataga     300
   cggcatggcg agggcacctt cacctccgac ctgtccaaac aaatggaaga agaagccgtc     360
   cggctgttca tcgaatggct gaaaaatggc ggcccttcct ctggcgcccc tcctccttct     420
   gtggctgctt ttgatatctg gggccaaggg acaatggtca ccgtgtcctc a              471
<210> 56
   <211> 157
   <212> PRT
   <213> Artificial sequence
<220>
   <223> ITS007-V127 (from VH to the end of JH)
<400> 56
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Arg Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ser Gly Ala Pro Pro Pro Ser Val Ala Ala Phe
       130                 135                 140
   Asp Ile Trp Gly Gln Gly Thr Met Val Thr Val Ser Ser
   145                 150                 155
<210> 57
   <211> 471
   <212> DNA
   <213> Artificial sequence
<220>
   <223> ITS007-V128 (from VH to the end of JH)
<400> 57
   gaagtgcagc tggtggaaag cggcggaggc ctggtgcagc ccggtcgaag cctgagactg        60
   agctgcgccg ccagcggctt cacctttgac gactacgcca tgcactgggt ccgccaggcc       120
   cctggcaagg gactggaatg ggtgtccggc atcagctgga acagcggcag catcggctac       180
   gccgacagcg tgaagggccg gttcaccatc agccgggaca acgccaagaa cagcctgtac       240
   ctgcagatga acagcctgcg ggccgaggac accgccttgt attactgtgc gagagatgag       300
   aagcatggcg agggcacctt cacctccgac ctgtccaaac aaatggaaga agaagccgtc       360
   cggctgttca tcgaatggct gaaaaatggc ggcccttcct ctggcgcccc tcctccttct       420
   gtggctgctt ttgatatctg gggccaaggg acaatggtca ccgtgtcctc a                471
<210> 58
   <211> 157
   <212> PRT
   <213> artificial sequence
<220>
   <223> ITS007-V128 (from VH to the end of JH)
<400> 58
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Glu Lys His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ser Gly Ala Pro Pro Pro Ser Val Ala Ala Phe
       130                 135                 140
   Asp Ile Trp Gly Gln Gly Thr Met Val Thr Val Ser Ser
   145                 150                 155
<210> 59
   <211> 471
   <212> DNA
   <213> Artificial sequence
<220>
   <223> ITS007-V130 (from VH to the end of JH)
<400> 59
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtag cagaaagaag     300
   cggcatggcg agggcacctt cacctccgac ctgtccaaac aaatggaaga agaagccgtc     360
   cggctgttca tcgaatggct gaaaaatggc ggcccttcct ctggcgcccc tcctccttct     420
   gtggctgctt ttgatatctg gggccaaggg acaatggtca ccgtgtcctc a              471
<210> 60
   <211> 157
   <212> PRT
   <213> Artificial sequence
<220>
   <223> ITS007-V130 (from VH to the end of JH)
<400> 60
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ser Arg Lys Lys Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ser Gly Ala Pro Pro Pro Ser Val Ala Ala Phe
       130                 135                 140
   Asp Ile Trp Gly Gln Gly Thr Met Val Thr Val Ser Ser
   145                 150                 155
<210> 61
   <211> 477
   <212> DNA
   <213> Artificial sequence
<220>
   <223> ITS007-V206 (from VH to the end of JH)
<400> 61
   gaagtgcagc tggtggaaag cggcggaggc ctggtgcagc ccggtcgaag cctgagactg        60
   agctgcgccg ccagcggctt cacctttgac gactacgcca tgcactgggt ccgccaggcc       120
   cctggcaagg gactggaatg ggtgtccggc atcagctgga acagcggcag catcggctac       180
   gccgacagcg tgaagggccg gttcaccatc agccgggaca acgccaagaa cagcctgtac       240
   ctgcagatga acagcctgcg ggccgaggac accgccttgt attactgtgc gagagatgag       300
   cggcatggcg agggcacctt cacctccgac ctgtccaaac aaatggaaga agaagccgtc       360
   cggctgttca tcgaatggct gaaaaatggc ggcccttcgg aggcctctgg cgcccctcct       420
   ccttctgtgg ctgcttttga tatctggggc caagggacaa tggtcaccgt gtcctca          477
<210> 62
   <211> 159
   <212> PRT
   <213> Artificial sequence
<220>
   <223> ITS007-V206 (from VH to the end of JH)
<400> 62
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Glu Ala Ser Gly Ala Pro Pro Pro Ser Val Ala
       130                 135                 140
   Ala Phe Asp Ile Trp Gly Gln Gly Thr Met Val Thr Val Ser Ser
   145                 150                 155
<210> 63
   <211> 480
   <212> DNA
   <213> Artificial sequence
<220>
   <223> ITS007-V209 (from VH to the end of JH)
<400> 63
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gagagatgag     300
   cggcatggcg agggcacctt cacctccgac ctgtccaaac aaatggaaga agaagccgtc     360
   cggctgttca tcgaatggct gaaaaatggc ggcccttccg cggagtcctc tggcgcccct     420
   cctccttctg tggctgcttt tgatatctgg ggccaaggga caatggtcac cgtgtcctca     480
<210> 64
   <211> 160
   <212> PRT
   <213> Artificial sequence
<220>
   <223> ITS007-V209 (from VH to the end of JH)
<400> 64
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ala Glu Ser Ser Gly Ala Pro Pro Pro Ser Val
       130                 135                 140
   Ala Ala Phe Asp Ile Trp Gly Gln Gly Thr Met Val Thr Val Ser Ser
   145                 150                 155                 160
<210> 65
   <211> 477
   <212> DNA
   <213> Artificial sequence
<220>
   <223> ITS007-V211 (from VH to the end of JH)
<400> 65
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gagagatgag     300
   cggcatggcg agggcacctt cacctccgac ctgtccaaac aaatggaaga agaagccgtc     360
   cggctgttca tcgaatggct gaaaaatggc ggcccttccg agtcctctgg cgcccctcct     420
   ccttctgtgg ctgcttttga tatctggggc caagggacaa tggtcaccgt gtcctca        477
<210> 66
   <211> 159
   <212> PRT
   <213> Artificial sequence
<220>
   <223> ITS007-V211 (from VH to the end of JH)
<400> 66
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Arg Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Glu Ser Ser Gly Ala Pro Pro Pro Ser Val Ala
       130                 135                 140
   Ala Phe Asp Ile Trp Gly Gln Gly Thr Met Val Thr Val Ser Ser
   145                 150                 155
<210> 67
   <211> 474
   <212> DNA
   <213> Artificial sequence
<220>
   <223> ITS007-V212 (from VH to the end of JH)
<400> 67
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gaaggatgag     300
   tggcggcatg gcgagggcac cttcacctcc gacctgtcca aacaaatgga agaagaagcc     360
   gtccggctgt tcatcgaatg gctgaaaaat ggcggccctt cctctggcgc ccctcctcct     420
   tctgtggctg cttttgatat ctggggccaa gggacaatgg tcaccgtgtc ctca           474
<210> 68
   <211> 158
   <212> PRT
   <213> Artificial sequence
<220>
   <223> ITS007-V212 (from VH to the end of JH)
<400> 68
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Glu Trp Arg His Gly Glu Gly Thr Phe Thr Ser Asp Leu
               100                 105                 110
   Ser Lys Gln Met Glu Glu Glu Ala Val Arg Leu Phe Ile Glu Trp Leu
           115                 120                 125
   Lys Asn Gly Gly Pro Ser Ser Gly Ala Pro Pro Pro Ser Val Ala Ala
       130                 135                 140
   Phe Asp Ile Trp Gly Gln Gly Thr Met Val Thr Val Ser Ser
   145                 150                 155
<210> 69
   <211> 592
   <212> DNA
   <213> Artificial sequence
<220>
   <223> ITS007-V152 (tet) tetracycline inducible CMV promoter
<400> 69
   tagttattaa tagtaatcaa ttacggggtc attagttcat agcccatata tggagttccg       60
   cgttacataa cttacggtaa atggcccgcc tggctgaccg cccaacgacc cccgcccatt      120
   gacgtcaata atgacgtatg ttcccatagt aacgccaata gggactttcc attgacgtca      180
   atgggtggag tatttacggt aaactgccca cttggcagta catcaagtgt atcatatgcc      240
   aagtacgccc cctattgacg tcaatgacgg taaatggccc gcctggcatt atgcccagta      300
   catgacctta tgggactttc ctacttggca gtacatctac gtattagtca tcgctattac      360
   catggtgatg cggttttggc agtacatcaa tgggcgtgga tagcggtttg actcacgggg      420
   atttccaagt ctccacccca ttgacgtcaa tgggagtttg ttttggcacc aaaatcaacg      480
   ggactttcca aaatgtcgta acaactccgc cccattgacg caaatgggcg gtaggcgtcc      540
   ctatcagtga tagagatcta tataagtccc tatcagtgat agagagagct ct              592
<210> 70
   <211> 477
   <212> DNA
   <213> Artificial sequence
<220>
   <223> ITS007-V241 (from VH to the end of JH)
<400> 70
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gaaggatgag     300
   cggcatggcg agggcacctt cacctccgac ctgtccaaac aaatggaaga agaagccgtc     360
   cggctgttca tcgaatggct gaaaaatggc ggcccttcgg aggcctctgg cgcccctcct     420
   ccttctgtgg ctgcttttga tatctggggc caagggacaa tggtcaccgt gtcctca        477
<210> 71
   <211> 159
   <212> PRT
   <213> Artificial sequence
<220>
   <223> ITS007-V241 (from VH to the end of JH)
<400> 71
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Glu Ala Ser Gly Ala Pro Pro Pro Ser Val Ala
       130                 135                 140
   Ala Phe Asp Ile Trp Gly Gln Gly Thr Met Val Thr Val Ser Ser
   145                 150                 155
<210> 72
   <211> 480
   <212> DNA
   <213> Artificial sequence
<220>
   <223> ITS007-V242 (from VH to the end of JH)
<400> 72
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gaaggatgag     300
   cggcatggcg agggcacctt cacctccgac ctgtccaaac aaatggaaga agaagccgtc     360
   cggctgttca tcgaatggct gaaaaatggc ggcccttccg cggagtcctc tggcgcccct     420
   cctccttctg tggctgcttt tgatatctgg ggccaaggga caatggtcac cgtgtcctca     480
<210> 73
   <211> 160
   <212> PRT
   <213> Artificial sequence
<220>
   <223> ITS007-V242 (from VH to the end of JH)
<400> 73
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ala Glu Ser Ser Gly Ala Pro Pro Pro Ser Val
       130                 135                 140
   Ala Ala Phe Asp Ile Trp Gly Gln Gly Thr Met Val Thr Val Ser Ser
   145                 150                 155                 160
<210> 74
   <211> 477
   <212> DNA
   <213> Artificial sequence
<220>
   <223> ITS007-V243 (from VH to the end of JH)
<400> 74
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtgc gaaggatgag     300
   cggcatggcg agggcacctt cacctccgac ctgtccaaac aaatggaaga agaagccgtc     360
   cggctgttca tcgaatggct gaaaaatggc ggcccttccg agtcctctgg cgcccctcct     420
   ccttctgtgg ctgcttttga tatctggggc caagggacaa tggtcaccgt gtcctca        477
<210> 75
   <211> 159
   <212> PRT
   <213> Artificial sequence
<220>
   <223> ITS007-V243 (from VH to the end of JH)
<400> 75
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ala Lys Asp Glu Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Glu Ser Ser Gly Ala Pro Pro Pro Ser Val Ala
       130                 135                 140
   Ala Phe Asp Ile Trp Gly Gln Gly Thr Met Val Thr Val Ser Ser
   145                 150                 155
<210> 76
   <211> 477
   <212> DNA
   <213> Artificial sequence
<220>
   <223> ITS007-V244 (from VH to the end of JH)
<400> 76
   gaagtgcagc tggtggaaag cggcggaggc ctggtgcagc ccggtcgaag cctgagactg      60
   agctgcgccg ccagcggctt cacctttgac gactacgcca tgcactgggt ccgccaggcc     120
   cctggcaagg gactggaatg ggtgtccggc atcagctgga acagcggcag catcggctac     180
   gccgacagcg tgaagggccg gttcaccatc agccgggaca acgccaagaa cagcctgtac     240
   ctgcagatga acagcctgcg ggccgaggac accgccttgt attactgtag cagaaagaag     300
   cggcatggcg agggcacctt cacctccgac ctgtccaaac aaatggaaga agaagccgtc     360
   cggctgttca tcgaatggct gaaaaatggc ggcccttcgg aggcctctgg cgcccctcct     420
   ccttctgtgg ctgcttttga tatctggggc caagggacaa tggtcaccgt gtcctca        477
<210> 77
   <211> 159
   <212> PRT
   <213> Artificial sequence
<220>
   <223> ITS007-V244 (from VH to the end of JH)
<400> 77
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
   35 40 45
   Ser Gly Ile Ser Trp Asn Ser Gly Ser Ile Gly Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Leu Tyr Tyr Cys
   85 90 95
   Ser Arg Lys Lys Arg His Gly Glu Gly Thr Phe Thr Ser Asp Leu Ser
   100 105 110
   Lys Gln Met Glu Glu Glu Ala Val Arg Leu Phe Ile Glu Trp Leu Lys
   115 120 125
   Asn Gly Gly Pro Ser Glu Ala Ser Gly Ala Pro Pro Pro Ser Val Ala
   130 135 140
   Ala Phe Asp Ile Trp Gly Gln Gly Thr Met Val Thr Val Ser Ser
   145 150 155
<210> 78
   <211> 480
   <212> DNA
   <213> Artificial sequence
<220>
   <223> ITS007-V245 (from VH to the end of JH)
<400> 78
   gaagtgcagc tggtggaaag cggcggaggc ctggtgcagc ccggtcgaag cctgagactg 60
   agctgcgccg ccagcggctt cacctttgac gactacgcca tgcactgggt ccgccaggcc 120
   cctggcaagg gactggaatg ggtgtccggc atcagctgga acagcggcag catcggctac 180
   gccgacagcg tgaagggccg gttcaccatc agccgggaca acgccaagaa cagcctgtac 240
   ctgcagatga acagcctgcg ggccgaggac accgccttgt attactgtag cagaaagaag 300
   cggcatggcg agggcacctt cacctccgac ctgtccaaac aaatggaaga agaagccgtc 360
   cggctgttca tcgaatggct gaaaaatggc ggcccttccg cggagtcctc tggcgcccct 420
   cctccttctg tggctgcttt tgatatctgg ggccaaggga caatggtcac cgtgtcctca 480
<210> 79
   <211> 160
   <212> PRT
   <213> Artificial sequence
<220>
   <223> ITS007-V245 (from VH to the end of JH)
<400> 79
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ser Arg Lys Lys Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Ala Glu Ser Ser Gly Ala Pro Pro Pro Ser Val
       130                 135                 140
   Ala Ala Phe Asp Ile Trp Gly Gln Gly Thr Met Val Thr Val Ser Ser
   145                 150                 155                 160
<210> 80
   <211> 477
   <212> DNA
   <213> Artificial sequence
<220>
   <223> ITS007-V246 (from VH to the end of JH)
<400> 80
   gaagtgcagc tggtggaaag cggcggaggc ctggtgcagc ccggtcgaag cctgagactg        60
   agctgcgccg ccagcggctt cacctttgac gactacgcca tgcactgggt ccgccaggcc       120
   cctggcaagg gactggaatg ggtgtccggc atcagctgga acagcggcag catcggctac       180
   gccgacagcg tgaagggccg gttcaccatc agccgggaca acgccaagaa cagcctgtac       240
   ctgcagatga acagcctgcg ggccgaggac accgccttgt attactgtag cagaaagaag       300
   cggcatggcg agggcacctt cacctccgac ctgtccaaac aaatggaaga agaagccgtc       360
   cggctgttca tcgaatggct gaaaaatggc ggcccttccg agtcctctgg cgcccctcct       420
   ccttctgtgg ctgcttttga tatctggggc caagggacaa tggtcaccgt gtcctca          477
<210> 81
   <211> 159
   <212> PRT
   <213> Artificial sequence
<220>
   <223> ITS007-V246 (from VH to the end of JH)
<400> 81
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Gly Ile Ser Trp Asn Ser Gly Ser Ile Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr Tyr Cys
                   85                  90                  95
   Ser Arg Lys Lys Arg His Gly Glu Gly Thr Phe Thr Ser Asp Leu Ser
               100                 105                 110
   Lys Gln Met Glu Glu Glu Ala Val Arg Leu Phe Ile Glu Trp Leu Lys
           115                 120                 125
   Asn Gly Gly Pro Ser Glu Ser Ser Gly Ala Pro Pro Pro Ser Val Ala
       130                 135                 140
   Ala Phe Asp Ile Trp Gly Gln Gly Thr Met Val Thr Val Ser Ser
   145                 150                 155
<210> 82
   <211> 47
   <212> PRT
   <213> Artificial sequence
<220>
   <223> ITS007-V125 exendin junction
<400> 82
   Cys Ala Arg Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala
           35                  40                  45
<210> 83
   <211> 47
   <212> PRT
   <213> Artificial sequence
<220>
   <223> ITS007-V126 exendin junction
<400> 83
   Cys Ala Lys Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala
           35                  40                  45
<210> 84
   <211> 47
   <212> PRT
   <213> Artificial sequence
<220>
   <223> ITS007-V127 exendin junction
<400> 84
   Cys Ala Arg Asp Arg Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala
           35                  40                  45
<210> 85
   <211> 47
   <212> PRT
   <213> Artificial sequence
<220>
   <223> ITS007-V128 exendin junction
<400> 85
   Cys Ala Arg Asp Glu Lys His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala
           35                  40                  45
<210> 86
   <211> 47
   <212> PRT
   <213> Artificial sequence
<220>
   <223> ITS007-V130 exendin junction
<400> 86
   Cys Ser Arg Lys Lys Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ser Gly Ala Pro Pro Pro Ser Val Ala
           35                  40                  45
<210> 87
   <211> 48
   <212> PRT
   <213> Artificial sequence
<220>
   <223> ITS007-V212 exendin junction
<400> 87
   Cys Ala Lys Asp Glu Trp Arg His Gly Glu Gly Thr Phe Thr Ser Asp
   1               5                   10                  15
   Leu Ser Lys Gln Met Glu Glu Glu Ala Val Arg Leu Phe Ile Glu Trp
               20                  25                  30
   Leu Lys Asn Gly Gly Pro Ser Ser Gly Ala Pro Pro Pro Ser Val Ala
           35                  40                  45
<210> 88
   <211> 49
   <212> PRT
   <213> Artificial sequence
<220>
   <223> ITS007-V206 exendin junction
<400> 88
   Cys Ala Arg Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Glu Ala Ser Gly Ala Pro Pro Pro Ser Val
           35                  40                  45
   Ala
<210> 89
   <211> 50
   <212> PRT
   <213> Artificial sequence
<220>
   <223> ITS007-V209 exendin junction
<400> 89
   Cys Ala Arg Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ala Glu Ser Ser Gly Ala Pro Pro Pro Ser
           35                  40                  45
   Val Ala
       50
<210> 90
   <211> 49
   <212> PRT
   <213> Artificial sequence
<220>
   <223> ITS007-V211 exendin junction
<400> 90
   Cys Ala Arg Asp Glu Arg His Gly Glu Gly Thr Phe Thr Ser Asp Leu
   1 5 10 15
   Ser Lys Gln Met Glu Glu Glu Ala Val Arg Leu Phe Ile Glu Trp Leu
   20 25 30
   Lys Asn Gly Gly Pro Ser Glu Ser Ser Gly Ala Pro Pro Pro Ser Val
   35 40 45
   Ala
<210> 91
   <211> 49
   <212> PRT
   <213> Artificial sequence
<220>
   <223> ITS007-V241 exendin junction
<400> 91
   Cys Ala Lys Asp Glu Arg His Gly Glu Gly Thr Phe Thr Ser Asp Leu
   1 5 10 15
   Ser Lys Gln Met Glu Glu Glu Ala Val Arg Leu Phe Ile Glu Trp Leu
   20 25 30
   Lys Asn Gly Gly Pro Ser Glu Ala Ser Gly Ala Pro Pro Pro Ser Val
   35 40 45
   Ala
<210> 92
   <211> 50
   <212> PRT
   <213> Artificial sequence
<220>
   <223> ITS007-V242 exendin junction
<400> 92
   Cys Ala Lys Asp Glu Arg His Gly Glu Gly Thr Phe Thr Ser Asp Leu
   1 5 10 15
   Ser Lys Gln Met Glu Glu Glu Ala Val Arg Leu Phe Ile Glu Trp Leu
   20 25 30
   Lys Asn Gly Gly Pro Ser Ala Glu Ser Ser Gly Ala Pro Pro Pro Ser
   35 40 45
   Val Ala
       50
<210> 93
   <211> 49
   <212> PRT
   <213> Artificial sequence
<220>
   <223> ITS007-V243 exendin junction
<400> 93
   Cys Ala Lys Asp Glu Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Glu Ser Ser Gly Ala Pro Pro Pro Ser Val
           35                  40                  45
   Ala
<210> 94
   <211> 49
   <212> PRT
   <213> Artificial sequence
<220>
   <223> ITS007-V244 exendin junction
<400> 94
   Cys Ser Arg Lys Lys Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Glu Ala Ser Gly Ala Pro Pro Pro Ser Val
           35                  40                  45
   Ala
<210> 95
   <211> 50
   <212> PRT
   <213> Artificial sequence
<220>
   <223> ITS007-V245 exendin junction
<400> 95
   Cys Ser Arg Lys Lys Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Ala Glu Ser Ser Gly Ala Pro Pro Pro Ser
           35                  40                  45
   Val Ala
       50
<210> 96
   <211> 49
   <212> PRT
   <213> Artificial sequence
<220>
   <223> ITS007-V246 exendin junction
<400> 96
   Cys Ser Arg Lys Lys Arg His Gly Glu Gly Thr Phe Thr Ser Asp Leu
   1               5                   10                  15
   Ser Lys Gln Met Glu Glu Glu Ala Val Arg Leu Phe Ile Glu Trp Leu
               20                  25                  30
   Lys Asn Gly Gly Pro Ser Glu Ser Ser Gly Ala Pro Pro Pro Ser Val
           35                  40                  45
   Ala
<210> 97
   <211> 465
   <212> DNA
   <213> Artificial sequence
<220>
   <223> ITS011-V36 (from VH to the end of JH)
<400> 97
   gaagtgcagc tggtggaaag cggcggaggc ctggtgaaac ctggcggcag cctgagactg    60
   agctgcgccg ccagcggctt caccttctcg aacgcctgga tgagctgggt ccgccaggcc   120
   cctggcaagg gactggaatg ggtcggacgg atcaagagca agaccgacgg cggcaccacc   180
   gactacgctg cccccgtgaa gggccggttc accatcagcc gggacgacag caagaacacc   240
   ctgtacctgc agatgaacag cctgaaaacc gaggacaccg ccgtgtatta ctgtaccaca   300
   gatctgtcca ggaggaggca ttcacagggc acattcacca gtgactacag caagtatctg   360
   gactccaggc gtgcccaaga ttttgtgcag tggttgatga ataccctgcc tatgcccgat   420
   gcttttgata tctggggcca agggacaatg gtcaccgtgt cctca                   465
<210> 98
   <211> 155
   <212> PRT
   <213> Artificial sequence
<220>
   <223> ITS011-V36 (from VH to the end of JH)
<400> 98
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Gly Arg Ile Lys Ser Lys Thr Asp Gly Gly Thr Thr Asp Tyr Ala Ala
       50                  55                  60
   Pro Val Lys Gly Arg Phe Thr Ile Ser Arg Asp Asp Ser Lys Asn Thr
   65                  70                  75                  80
   Leu Tyr Leu Gln Met Asn Ser Leu Lys Thr Glu Asp Thr Ala Val Tyr
                   85                  90                  95
   Tyr Cys Thr Thr Asp Leu Ser Arg Arg Arg His Ser Gln Gly Thr Phe
               100                 105                 110
   Thr Ser Asp Tyr Ser Lys Tyr Leu Asp Ser Arg Arg Ala Gln Asp Phe
           115                 120                 125
   Val Gln Trp Leu Met Asn Thr Leu Pro Met Pro Asp Ala Phe Asp Ile
       130                 135                 140
   Trp Gly Gln Gly Thr Met Val Thr Val Ser Ser
   145                 150                 155
<210> 99
   <211> 471
   <212> DNA
   <213> Artificial sequence
<220>
   <223> ITS011-V66 (from VH to the end of JH)
<400> 99
   gaagtgcagc tggtggaaag cggcggaggc ctggtgaaac ctggcggcag cctgagactg 60
   agctgcgccg ccagcggctt caccttctcg aacgcctgga tgagctgggt ccgccaggcc 120
   cctggcaagg gactggaatg ggtcggacgg atcaagagca agaccgacgg cggcaccacc 180
   gactacgctg cccccgtgaa gggccggttc accatcagcc gggacgacag caagaacacc 240
   ctgtacctgc agatgaacag cctgaaaacc gaggacaccg ccgtgtatta ctgtaccaca 300
   gatcccgtgc ttaagcattc acagggcaca ttcaccagtg actacagcaa gtatctggac 360
   tccaggcgtg cccaagattt tgtgcagtgg ttgatgaata ccctggcgcc tgtaaactac 420
   tactactaca tggacgtctg gggcaaaggg accacggtca ccgtgtcctc a 471
<210> 100
   <211> 157
   <212> PRT
   <213> Artificial sequence
<220>
   <223> ITS011-V66 (from VH to the end of JH)
<400> 100
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Thr Phe Ser Asn Ala
               20                  25                  30
   Trp Met Ser Trp Val Arg Gln Ala Pro Gly Lys Gly Leu Glu Trp Val
           35                  40                  45
   Gly Arg Ile Lys Ser Lys Thr Asp Gly Gly Thr Thr Asp Tyr Ala Ala
       50                  55                  60
   Pro Val Lys Gly Arg Phe Thr Ile Ser Arg Asp Asp Ser Lys Asn Thr
   65                  70                  75                  80
   Leu Tyr Leu Gln Met Asn Ser Leu Lys Thr Glu Asp Thr Ala Val Tyr
                   85                  90                  95
   Tyr Cys Thr Thr Asp Pro Val Leu Lys His Ser Gln Gly Thr Phe Thr
               100                 105                 110
   Ser Asp Tyr Ser Lys Tyr Leu Asp Ser Arg Arg Ala Gln Asp Phe Val
           115                 120                 125
   Gln Trp Leu Met Asn Thr Leu Ala Pro Val Asn Tyr Tyr Tyr Tyr Met
   130 135 140
   Asp Val Trp Gly Lys Gly Thr Thr Val Thr Val Ser Ser
   145 150 155
<210> 101
   <211> 459
   <212> DNA
   <213> Artificial sequence
<220>
   <223> ITS011-V69 (from VH to the end of JH)
<400> 101
   gaagtgcagc tggtggaaag cggaggcgga gtggttcgac ctggcggaag cctgagactg 60
   tcttgcgccg ccagcggctt cacctttgac gactacggca tgagctgggt ccgccaggcc 120
   cctggcaagg gactggaatg ggtgtccggc atcaactgga acggcggcag caccggctac 180
   gccgacagcg tgaagggccg gttcaccatc agccgggaca acgccaagaa cagcctgtac 240
   ctgcagatga acagcctgcg ggccgaggac accgccttgt atcactgtgc gagagcggaa 300
   agcgcgggga ggcattcaca gggcacattc accagtgact acagcaagta tctggactcc 360
   aggcgtgccc aagattttgt gcagtggttg atgaatacca cttcgatggt gcggtacttc 420
   cagcactggg gccagggcac cctggtcacc gtgtcctca 459
<210> 102
   <211> 153
   <212> PRT
   <213> Artificial sequence
<220>
   <223> ITS011-V69 (from VH to the end of JH)
<400> 102
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr His Cys
                   85                  90                  95
   Ala Arg Ala Glu Ser Ala Gly Arg His Ser Gln Gly Thr Phe Thr Ser
               100                 105                 110
   Asp Tyr Ser Lys Tyr Leu Asp Ser Arg Arg Ala Gln Asp Phe Val Gln
           115                 120                 125
   Trp Leu Met Asn Thr Thr Ser Met Val Arg Tyr Phe Gln His Trp Gly
       130                 135                 140
   Gln Gly Thr Leu Val Thr Val Ser Ser
   145                 150
<210> 103
   <211> 477
   <212> DNA
   <213> Artificial sequence
<220>
   <223> ITS011-V23 (from VH to the end of JH)
<400> 103
   caggtgcagc tggtggaaag cggcggaggc gtggtgcagc ccggcagaag cctgagactg        60
   agctgcgccg ccagcggctt caccttcagc agctacgcca tgcactgggt ccgccaggcc       120
   cctggcaagg gactggaatg ggtggccgtg atcagctacg acggcagcaa caagtactac       180
   gccgacagcg tgaagggccg gttcaccatc tctcgagaca acagcaagaa caccctgtac       240
   ctgcagatga acagcctgcg ggccgaggac accgccgtgt attactgtgc gagtaatagg       300
   cattcacagg gcacattcac cagtgactac agcaagtatc tggactccag gcgtgcccaa       360
   gattttgtgc agtggttgat gaataccaag aggaacagga ataacattgc catgcttctt       420
   gactactact actacatgga cgtctggggc aaagggacca cggtcaccgt gtcctca         477
<210> 104
   <211> 159
   <212> PRT
   <213> Artificial sequence
<220>
   <223> ITS011-V23 (from VH to the end of JH)
<400> 104
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
   35 40 45
   Ala Val Ile Ser Tyr Asp Gly Ser Asn Lys Tyr Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Ser Asn Arg His Ser Gln Gly Thr Phe Thr Ser Asp Tyr Ser Lys
   100 105 110
   Tyr Leu Asp Ser Arg Arg Ala Gln Asp Phe Val Gln Trp Leu Met Asn
   115 120 125
   Thr Lys Arg Asn Arg Asn Asn Ile Ala Met Leu Leu Asp Tyr Tyr Tyr
   130 135 140
   Tyr Met Asp Val Trp Gly Lys Gly Thr Thr Val Thr Val Ser Ser
   145 150 155
<210> 105
   <211> 483
   <212> DNA
   <213> Artificial sequence
<220>
   <223> ITS011-V74 (from VH to the end of JH)
<400> 105
   gaagtgcagc tggtggaaag cggaggcgga gtggttcgac ctggcggaag cctgagactg 60
   tcttgcgccg ccagcggctt cacctttgac gactacggca tgagctgggt ccgccaggcc 120
   cctggcaagg gactggaatg ggtgtccggc atcaactgga acggcggcag caccggctac 180
   gccgacagcg tgaagggccg gttcaccatc agccgggaca acgccaagaa cagcctgtac 240
   ctgcagatga acagcctgcg ggccgaggac accgccttgt atcactgtgc gagagataaa 300
   gggattaagc attcacaggg cacattcacc agtgactaca gcaagtatct ggactccagg 360
   cgtgcccaag attttgtgca gtggttgatg aataccaaga ggaacaggaa taacattgcc 420
   agtaagcagg tgcgatggta cttcgatctc tggggccgtg gcaccctggt cactgtgtcc 480
   tca 483
<210> 106
   <211> 161
   <212> PRT
   <213> Artificial sequence
<220>
   <223> ITS011-V74 (from VH to the end of JH)
<400> 106
   Glu Val Gln Leu Val Glu Ser Gly Gly Gly Val Val Arg Pro Gly Gly
   1               5                   10                  15
   Ser Leu Arg Leu Ser Cys Ala Ala Ser Gly Phe Thr Phe Asp Asp Tyr
               20                  25                  30
   Gly Met Ser Trp Val Arg Gln Ala Pro Gly Lys Gly Leu Glu Trp Val
           35                  40                  45
   Ser Gly Ile Asn Trp Asn Gly Gly Ser Thr Gly Tyr Ala Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Leu Tyr His Cys
                   85                  90                  95
   Ala Arg Asp Lys Gly Ile Lys His Ser Gln Gly Thr Phe Thr Ser Asp
               100                 105                 110
   Tyr Ser Lys Tyr Leu Asp Ser Arg Arg Ala Gln Asp Phe Val Gln Trp
           115                 120                 125
   Leu Met Asn Thr Lys Arg Asn Arg Asn Asn Ile Ala Ser Lys Gln Val
       130                 135                 140
   Arg Trp Tyr Phe Asp Leu Trp Gly Arg Gly Thr Leu Val Thr Val Ser
   145                 150                 155                 160
   Ser
<210> 107
   <211> 510
   <212> DNA
   <213> Artificial sequence
<220>
   <223> ITS011-V100 (from VH to the end of JH)
<400> 107
   caggtgcagc tggtggaaag cggcggaggc gtggtgcagc ccggcagaag cctgagactg        60
   agctgcgccg ccagcggctt caccttcagc agctacgcca tgcactgggt ccgccaggcc       120
   cctggcaagg gactggaatg ggtggccgtg atcagctacg acggcagcaa caagtactac       180
   gccgacagcg tgaagggccg gttcaccatc tctcgagaca acagcaagaa caccctgtac       240
   ctgcagatga acagcctgcg ggccgaggac accgccgtgt attactgtgc gagagatctc       300
   tcggatctct cgcacagtgt attactgagg cattcacagg gcacattcac cagtgactac       360
   agcaagtatc tggactccag gcgtgcccaa gattttgtgc agtggttgat gaataccaag       420
   aggaacagga ataacattgc ccggccgctt gtcctcagcg ctgaatactt ccagcactgg       480
   ggccagggca ccctggtcac cgtgtcctca                                        510
<210> 108
   <211> 170
   <212> PRT
   <213> Artificial sequence
<220>
   <223> ITS011-V100 (from VH to the end of JH)
<400> 108
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Leu Ser Asp Leu Ser His Ser Val Leu Leu Arg His Ser
               100                 105                 110
   Gln Gly Thr Phe Thr Ser Asp Tyr Ser Lys Tyr Leu Asp Ser Arg Arg
           115                 120                 125
   Ala Gln Asp Phe Val Gln Trp Leu Met Asn Thr Lys Arg Asn Arg Asn
       130                 135                 140
   Asn Ile Ala Arg Pro Leu Val Leu Ser Ala Glu Tyr Phe Gln His Trp
   145                 150                 155                 160
   Gly Gln Gly Thr Leu Val Thr Val Ser Ser
                   165                 170
<210> 109
   <211> 477
   <212> DNA
   <213> Artificial sequence
<220>
   <223> ITS011-V165 (from VH to the end of JH)
<400> 109
   caggtgcagc tgcagcagtg gggagccggc ctgctgaagc ccagcgagac actgagcctg        60
   acctgcgccg tgtacggcgg cagcttcagc ggctactact ggtcctggat cagacagccc       120
   cctggcaagg gcctggaatg gatcggcgag atcaaccaca gcggcagcac caactacaac       180
   cccagcctga agtccagagt gaccatcagc gtggacacct cgaagaacca gttcagcctg       240
   aagctgagca gcgtgacagc cgccgacacc gctgtgtatt actgtgcgag aggccccatc       300
   ctaaggcccc ggaggcattc acagggcaca ttcaccagtg actacagcaa gtatctggac       360
   tccaggcgtg cccaagattt tgtgcagtgg ttgatgaata ccaagaggaa caggaataac       420
   attgcccata gtattgtgga ctactggggc cagggaaccc tggtcaccgt gtcctca          477
<210> 110
   <211> 159
   <212> PRT
   <213> Artificial sequence
<220>
   <223> ITS011-V165 (from VH to the end of JH)
<400> 110
   Gln Val Gln Leu Gln Gln Trp Gly Ala Gly Leu Leu Lys Pro Ser Glu
   1               5                   10                  15
   Thr Leu Ser Leu Thr Cys Ala Val Tyr Gly Gly Ser Phe Ser Gly Tyr
               20                  25                  30
   Tyr Trp Ser Trp Ile Arg Gln Pro Pro Gly Lys Gly Leu Glu Trp Ile
           35                  40                  45
   Gly Glu Ile Asn His Ser Gly Ser Thr Asn Tyr Asn Pro Ser Leu Lys
       50                  55                  60
   Ser Arg Val Thr Ile Ser Val Asp Thr Ser Lys Asn Gln Phe Ser Leu
   65                  70                  75                  80
   Lys Leu Ser Ser Val Thr Ala Ala Asp Thr Ala Val Tyr Tyr Cys Ala
   85 90 95
   Arg Gly Pro Ile Leu Arg Pro Arg Arg His Ser Gln Gly Thr Phe Thr
   100 105 110
   Ser Asp Tyr Ser Lys Tyr Leu Asp Ser Arg Arg Ala Gln Asp Phe Val
   115 120 125
   Gln Trp Leu Met Asn Thr Lys Arg Asn Arg Asn Asn Ile Ala His Ser
   130 135 140
   Ile Val Asp Tyr Trp Gly Gln Gly Thr Leu Val Thr Val Ser Ser
   145 150 155
<210> 111
   <211> 468
   <212> DNA
   <213> Artificial sequence
<220>
   <223> ITS011-V171 (from VH to the end of JH)
<400> 111
   caggtgcagc tggtggaaag cggcggaggc gtggtgcagc ccggcagaag cctgagactg 60
   agctgcgccg ccagcggctt caccttcagc agctacgcca tgcactgggt ccgccaggcc 120
   cctggcaagg gactggaatg ggtggccgtg atcagctacg acggcagcaa caagtactac 180
   gccgacagcg tgaagggccg gttcaccatc tctcgagaca acagcaagaa caccctgtac 240
   ctgcagatga acagcctgcg ggccgaggac accgccgtgt attactgtgc gagagatcgg 300
   ttttcccgat ccttttctaa gcattcacag ggcacattca ccagtgacta cagcaagtat 360
   ctggactcca ggcgtgccca agattttgtg cagtggttga tgaataccaa gaggaacagg 420
   aataacattg ctaactgggg ccagggcacc ctggtcaccg tgtcctca 468
<210> 112
   <211> 156
   <212> PRT
   <213> Artificial sequence
<220>
   <223> ITS011-V171 (from VH to the end of JH)
<400> 112
   Gln Val Gln Leu Val Glu Ser Gly Gly Gly Val Val Gln Pro Gly Arg
   1               5                   10                  15
   Ser Leu Arg Leu Ser Cys Ala Ala Ser Gly Phe Thr Phe Ser Ser Tyr
               20                  25                  30
   Ala Met His Trp Val Arg Gln Ala Pro Gly Lys Gly Leu Glu Trp Val
           35                  40                  45
   Ala Val Ile Ser Tyr Asp Gly Ser Asn Lys Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Phe Ser Arg Ser Phe Ser Lys His Ser Gln Gly Thr
               100                 105                 110
   Phe Thr Ser Asp Tyr Ser Lys Tyr Leu Asp Ser Arg Arg Ala Gln Asp
           115                 120                 125
   Phe Val Gln Trp Leu Met Asn Thr Lys Arg Asn Arg Asn Asn Ile Ala
       130                 135                 140
   Asn Trp Gly Gln Gly Thr Leu Val Thr Val Ser Ser
   145                 150                 155
<210> 113
   <211> 52
   <212> PRT
   <213> Artificial sequence
<220>
   <223> ITS011-V165 oxyntomodulin junction
<400> 113
   Cys Ala Arg Gly Pro Ile Leu Arg Pro Arg Arg His Ser Gln Gly Thr
   1               5                   10                  15
   Phe Thr Ser Asp Tyr Ser Lys Tyr Leu Asp Ser Arg Arg Ala Gln Asp
               20                  25                  30
   Phe Val Gln Trp Leu Met Asn Thr Lys Arg Asn Arg Asn Asn Ile Ala
           35                  40                  45
   His Ser Ile Val
       50
<210> 114
   <211> 50
   <212> PRT
   <213> Artificial sequence
<220>
   <223> ITS011-V171 oxyntomodulin junction
<400> 114
   Cys Ala Arg Asp Arg Phe Ser Arg Ser Phe Ser Lys His Ser Gln Gly
   1               5                   10                  15
   Thr Phe Thr Ser Asp Tyr Ser Lys Tyr Leu Asp Ser Arg Arg Ala Gln
               20                  25                  30
   Asp Phe Val Gln Trp Leu Met Asn Thr Lys Arg Asn Arg Asn Asn Ile
           35                  40                  45
   Ala Asn
       50

## Description

### FIELD OF THE INVENTION

The present invention relates to the field of biologics and, in particular, to peptide-grafted antibodies capable of activating GPCRs and methods for identifying same.

### BACKGROUND OF THE INVENTION

G-protein coupled receptors (GPCRs) are a large family of transmembrane proteins that are involved in a wide variety of physiological processes. Class B GPCRs (the secretin receptor family) include a number of hormone receptors such as secretin receptors, vasoactive intestinal peptide receptors, calcitonin receptors and parathyroid hormone receptors.

The glucagon-like peptide 1 receptor (GLP1R) is a class B GPCR. The ligands appear to bind to GLP1R via a two step mechanism (Runge et al., 2008, J Biol. Chem., 283(17):11340-11347; Underwood et al., 2010, J Biol. Chem., 285(1):723-730). The first step is binding to the amino terminus of the receptor via the carboxy terminus of the ligand. A second step of binding results in activation of the receptor and is mediated by the amino terminus of the ligand. The carboxy terminus of the ligand appears to be able to accommodate modification and in fact is active as a fusion protein such as GLP1-Fc. The amino terminus of GLP1 receptor ligands, on the other hand, appears to be significantly more sensitive to modification (Kieffer & Habener, (1999), Endocrine Reviews, 20(6), 876-913). The removal of the N-terminal histidine of the mature GLP1 peptide has been shown to result in a loss of binding and activity. The addition of one amino acid to the amino terminus of GLP1 also reduces activity. Single histidine to alanine substitution of the terminal histidine has shown this residue to be important for receptor binding. Similarly, the removal of the terminal histidine from Exendin also results in reduced activity. A two amino-acid deletion from the N-terminus of Exendin results in an antagonist even though the binding affinity is not significantly decreased.

Cross-linking studies using another class B GPCR, the parathyroid hormone receptor (PTHR), have shown that the N-terminal serine of the PTH ligand interacts directly with the receptor (Rolz et al., 1999, Biochemistry, 38(20):6397-6405). The PTHrp peptide has a N-terminal alanine and is also capable of activating the PTH receptor indicating that the side chain may not be directly involved in the contact with the receptor.

Peptide agonists of class B GPCRs, such as the GLP1R agonist Exendin-4, have potential as therapeutic agents. One drawback to the use of peptides as therapeutic reagents, however, is that they are generally unstable *in vivo, i.e.* their clearance rates from serum may be quite rapid. Fusion of the peptide to another larger molecule is one approach often adopted in order to stabilize the peptide *in vivo,* however conformational changes and/or other molecular forces that occur in peptide fusions may interfere with or negate the activity of the peptide. Each such peptide fusion must therefore be tested empirically in order to determine whether it has retained sufficient activity to be therapeutically useful.

International Patent Application No. PCT/US2004/041946 (WO 2005/060642) describes a method of producing rationally designed antibodies that comprise a peptide in one or more CDRs. A library of peptide-grafted antibodies that comprised GLP1 fused into the heavy chain CDR of a human tetanus toxoid specific antibody is described, but was not tested for the ability of the antibodies to bind GLP1R.

International Patent Application No. PCT/CA2013/050204 (WO 2013/134881) describes methods of generating fusion protein variants, including immunoglobulins comprising a peptide in one or more CDR. Immunoglobulins comprising GLP1 or exendin fused into the CDR of the immunoglobulin are described that are capable of binding to GLP1R.

This background information is provided for the purpose of making known information believed by the applicant to be of possible relevance to the present invention. No admission is necessarily intended, nor should be construed, that any of the preceding information constitutes prior art against the present invention.

### SUMMARY OF THE INVENTION

The invention is defined by the appended claims.

The present invention relates generally to peptide-grafted antibodies having GPCR agonist activity and their uses, and methods for identifying same. In one aspect, the invention relates to a peptide-grafted antibody or antibody fragment comprising at least one peptide grafted into at least one CDR of the antibody or antibody fragment, the peptide comprising the sequence of the exendin, GLP1, glucagon or oxyntomodulin peptide or a functional fragment thereof, wherein the peptide-grafted antibody has agonist activity against the GLP1 receptor, the glucagon receptor, or both.

The grafted peptide comprises a basic amino acid upstream and adjacent to the natural N-terminal amino acid of the exendin, GLP1, glucagon or oxyntomodulin peptide, wherein the basic amino acid is an arginine or a lysine.

In another aspect, the invention relates to a polynucleotide encoding a peptide-grafted antibody or antibody fragment as described above.

Disclosed is also a mammalian cell line for high-throughput screening for agonists of a Class B GPCR, the cell line comprising: a first stably integrated recombinant nucleic acid construct comprising a reporter gene operably associated with a cAMP responsive promoter, the reporter gene encoding a cell-surface expressed protein, and a second stably integrated recombinant nucleic acid construct comprising a sequence encoding a Class B GPCR operably associated with a promoter.

The mammalian cell line may be recombination competent.

Disclosed is also a method for screening for peptide-grafted antibodies having agonist activity against a Class B GPCR, the method comprising: stably transfecting cells from a cell line as described above with a library of polynucleotides encoding peptide-grafted antibodies, wherein the peptide is a candidate peptide agonist of a Class B GPCR; culturing the cells under conditions that permit expression of the peptide-grafted antibodies and expression of the Class B GPCR, and screening the cells for expression of the cell-surface expressed protein.

Disclosed is also a method for screening for peptide-grafted antibodies having agonist activity against a Class B GPCR, the method comprising: stably transfecting cells from a recombination competent cell line as described above with either (a) a nucleic acid sequence encoding a plurality of VH gene sequences, a candidate peptide agonist of the Class B GPCR and at least one JH gene sequence, and comprising one or more pairs of RSS sequences positioned to allow recombination of the nucleic acid sequence to form a polynucleotide encoding an antibody heavy chain comprising the peptide agonist, or (b) a nucleic acid sequence encoding a plurality of VL gene sequences, a candidate peptide agonist of the Class B GPCR and at least one JL gene sequence, and comprising one or more pairs of RSS sequences positioned to allow recombination of the nucleic acid sequence to form a polynucleotide encoding an antibody light chain comprising the peptide agonist; culturing the cells under conditions that permit V(D)J recombination, expression of the antibody heavy or light chain comprising the peptide agonist and expression of the Class B GPCR, and screening the cultured cells for expression of the cell-surface expressed protein.

Disclosed is also a method for preparing a heavy-chain only peptide-grafted antibody having agonist activity against a Class B GPCR, the method comprising: stably transfecting cells from a recombination competent cell line as described above with a nucleic acid sequence encoding a plurality of VH gene sequences, a candidate peptide agonist of the Class B GPCR and at least one JH gene sequence, and comprising one or more pairs of RSS sequences positioned to allow recombination of the nucleic acid sequence to form a polynucleotide encoding an antibody heavy chain comprising the peptide agonist; culturing the cells under conditions that permit V(D)J recombination, expression of the antibody heavy chain comprising the peptide agonist and expression of the Class B GPCR; screening the cultured cells for expression of the cell-surface expressed protein; isolating a cell that expresses the cell-surface expressed protein, and isolating a polynucleotide encoding the peptide-grafted antibody having agonist activity against a Class B GPCR from the isolated cell.

### BRIEF DESCRIPTION OF THE DRAWINGS

These and other features of the invention will become more apparent in the following detailed description in which reference is made to the appended drawings.

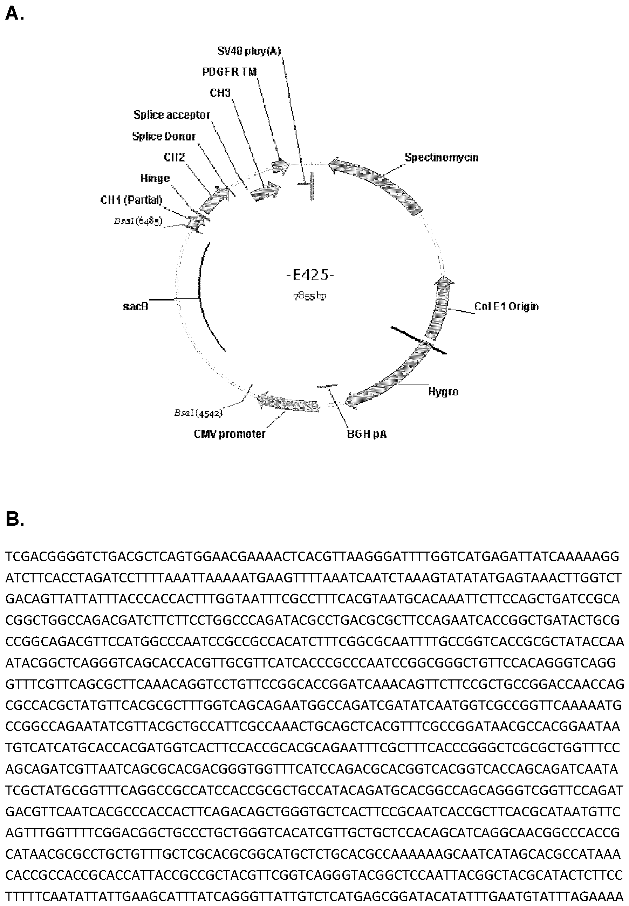

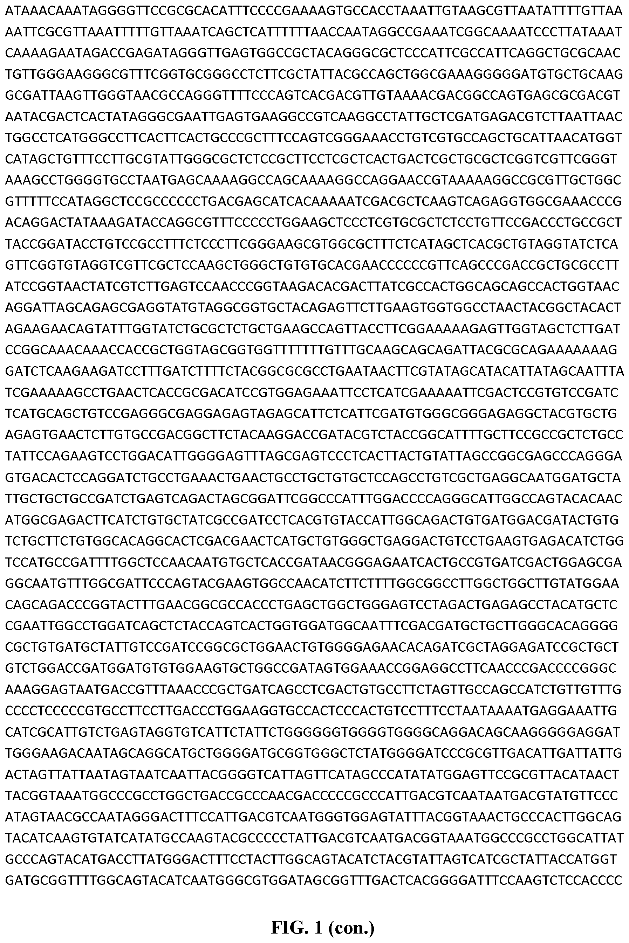

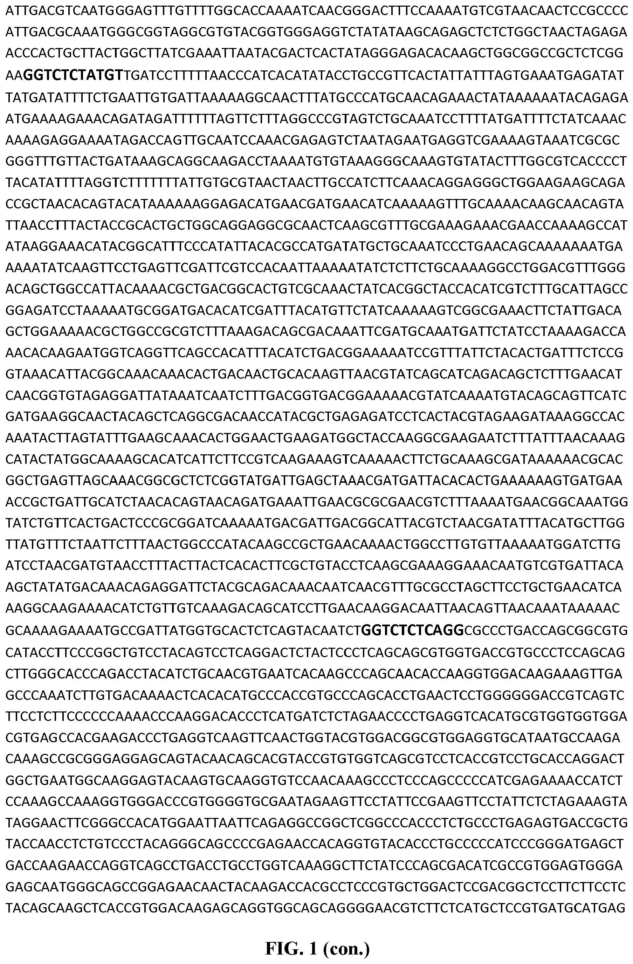

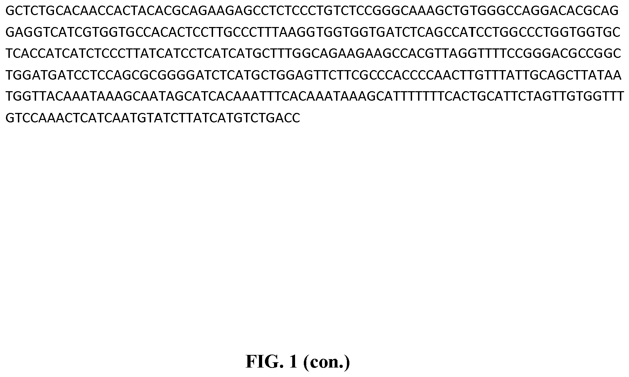

**Figure 1** shows (A) a schematic diagram, and (B) the nucleic acid sequence [SEQ ID NO:1], of vector E425 for receiving heavy chain variable cDNA PCR products. The vector is digested with BsaI to generate the appropriate overhangs for ligation (CAGG; highlighted in bold), which is in-frame with the IgG CH1 sequence. The BsaI site downstream of the CMV promoter is highlighted in bold and generates the overhang to accept the ATGT of the cDNA PCR product.

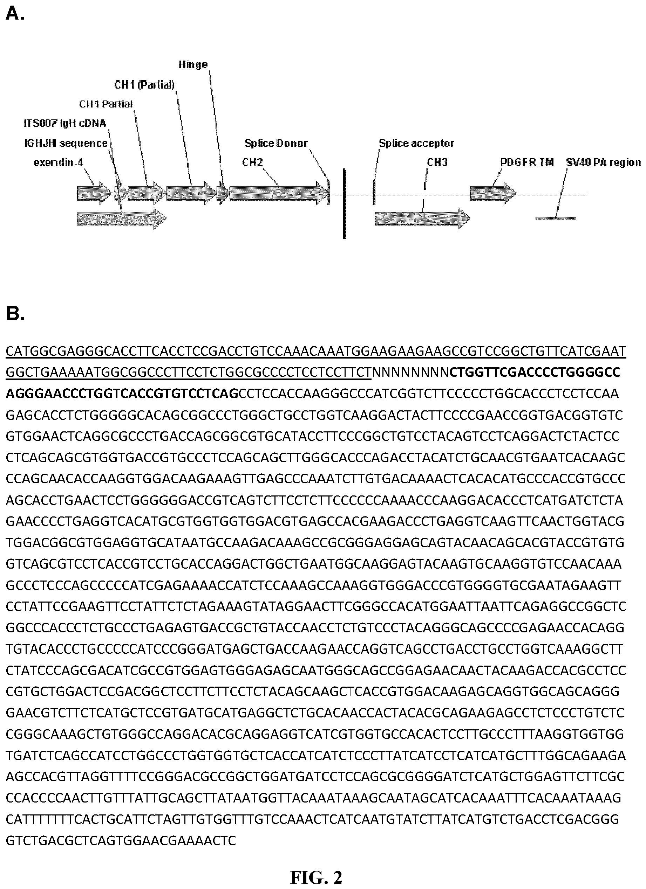

**Figure 2**(A) shows a schematic diagram of ITS007-P31, which served as the template for PCR of GLP1R binding peptide-grafted antibodies using primers ET70 and ET71. Each of the peptide-grafted antibodies differed in the JH sequence used and in the intervening amino acids between the 3' end of Exendin and the JH gene segment as a result of the initial V(D)J recombination which had resulted in deletions and/or additions at this junction; (B) shows the nucleic acid sequence [SEQ ID NO:2] of the PCR product that resulted from amplifying one peptide-grafted antibody in which 8 nucleotides (Ns) had been inserted between the 3' Exendin sequences (underlined) and a JH5 gene segment (bold). The PCR product encompasses the histidine at the N-terminal end of Exendin through the SV40 poly(A) sequence.

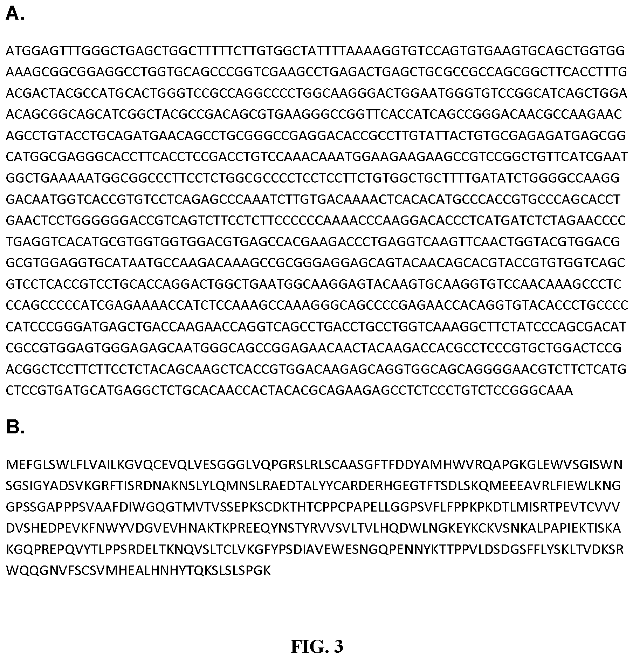

**Figure 3** shows (A) the nucleic acid sequence [SEQ ID NO:50] and (B) the amino acid sequence [SEQ ID NO:51] for the agonist peptide-grafted HCAb ITS007-V125.

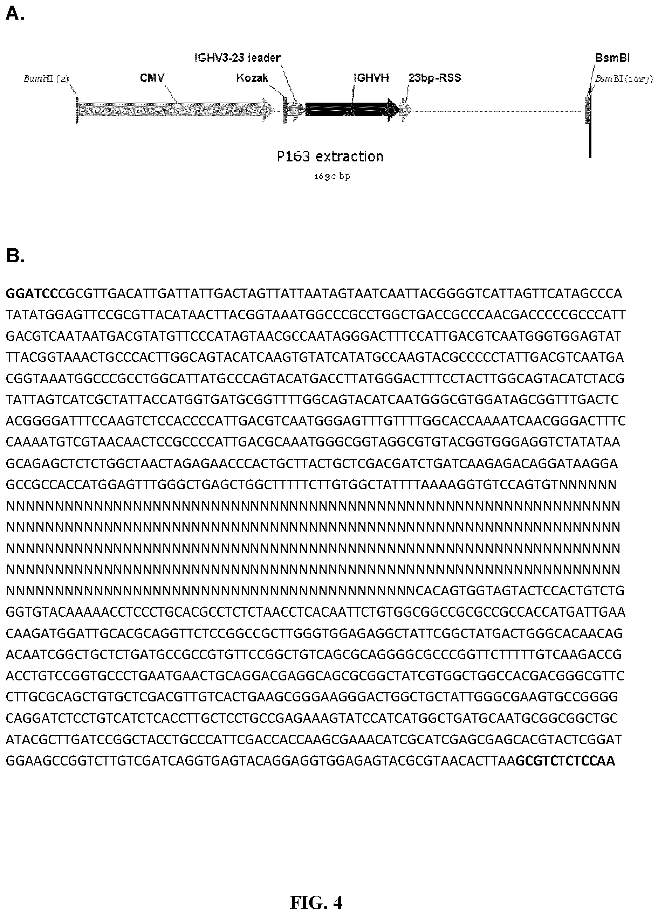

**Figure 4** shows (A) a schematic diagram and (B) a generic nucleic acid sequence [SEQ ID NO:3] of a fragment derived from P163 that contains a 5' BamHI site, CMV promoter, a heavy chain variable segment (depicted by Ns), the 23 base pair RSS, spacer sequences and a 3' BsmBI site. P163 represents a pool of 45 vectors each encoding a different VH gene segment upstream of the 23 bp RSS.

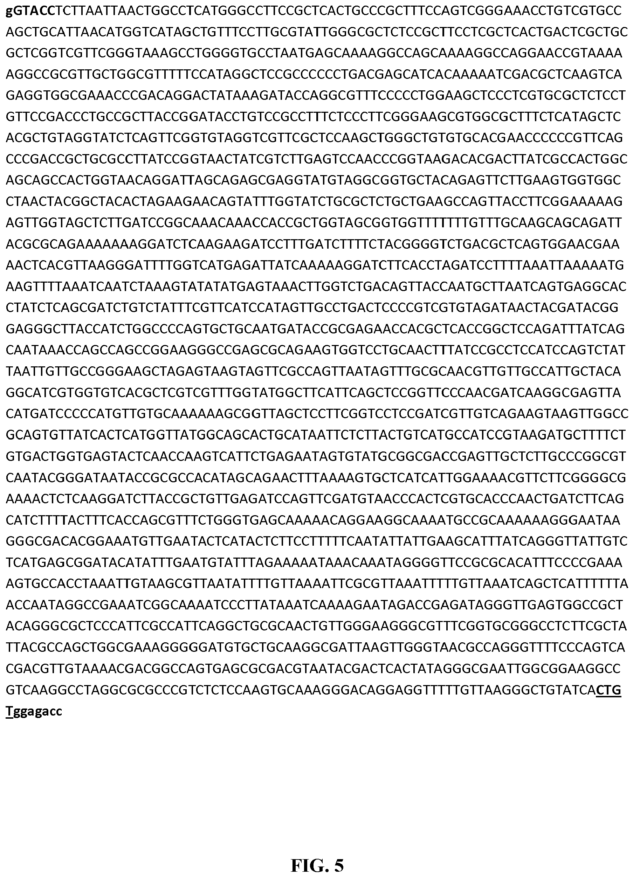

**Figure 5** shows the nucleic acid sequence [SEQ ID NO:4] of vector V610 digested with KpnI (bold) and BsaI (bold, overhang underlined).

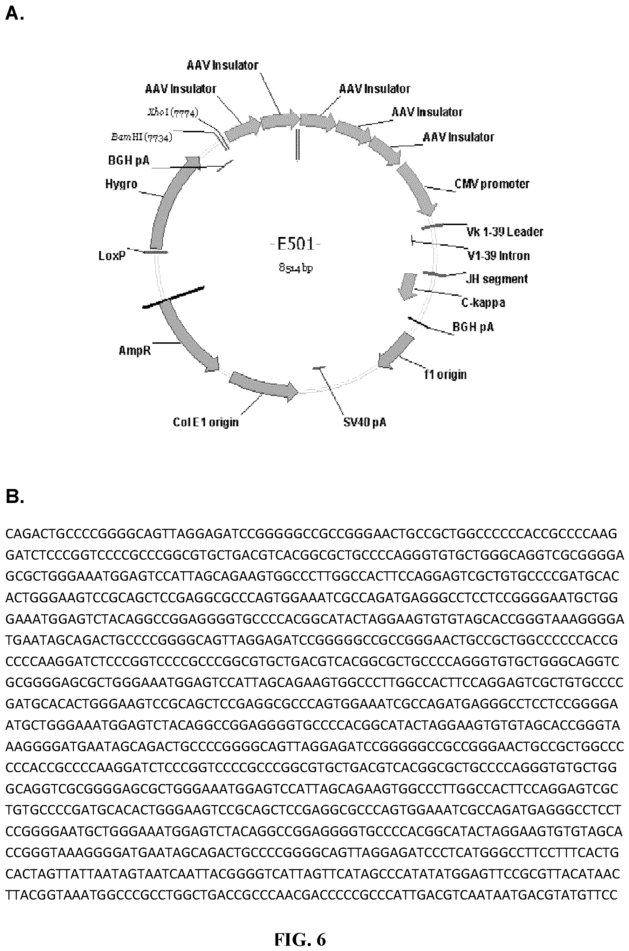

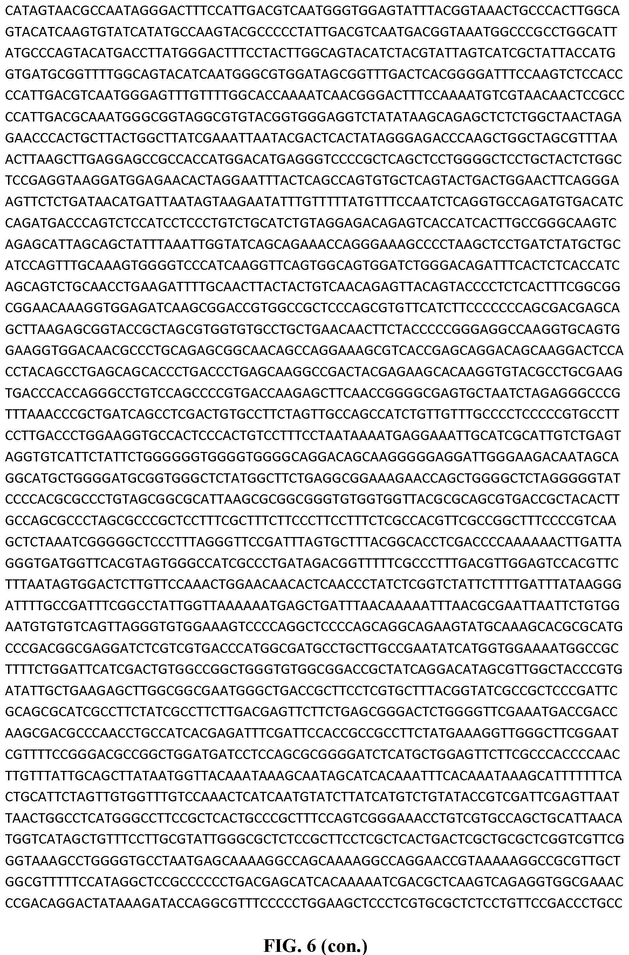

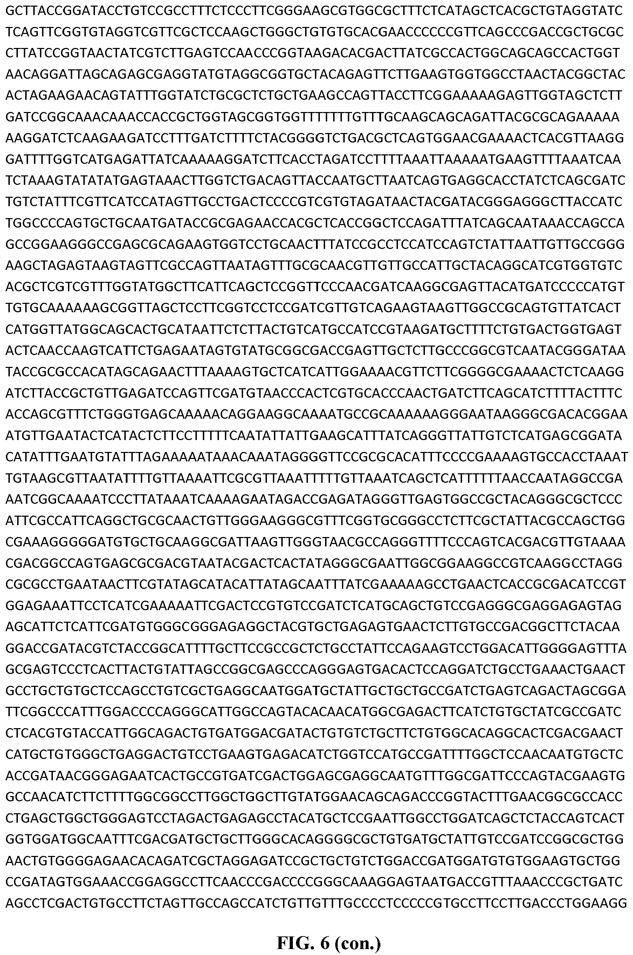

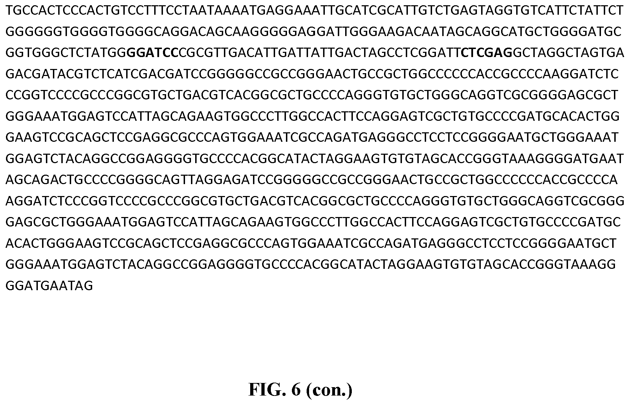

**Figure 6** shows (A) a schematic diagram and (B) the nucleic acid sequence [SEQ ID NO:5] of the acceptor vector E501. E501 represents the final CDR optimization vector to diversify the junction of the 3' VH framework and the 5' Exendin peptide sequences. The BamHI and XhoI cloning site for P163 and ITS007-P31 are shown in bold.

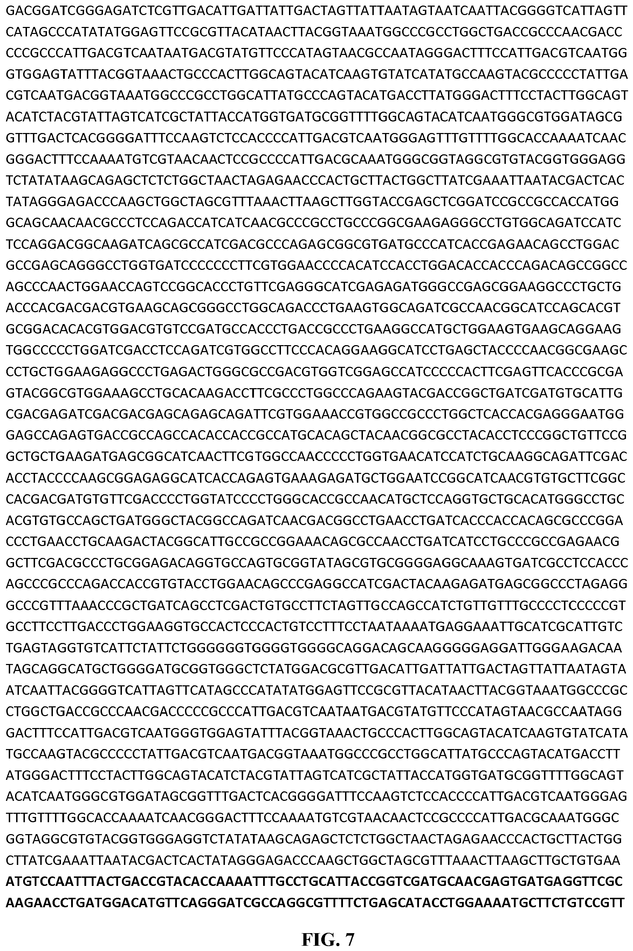

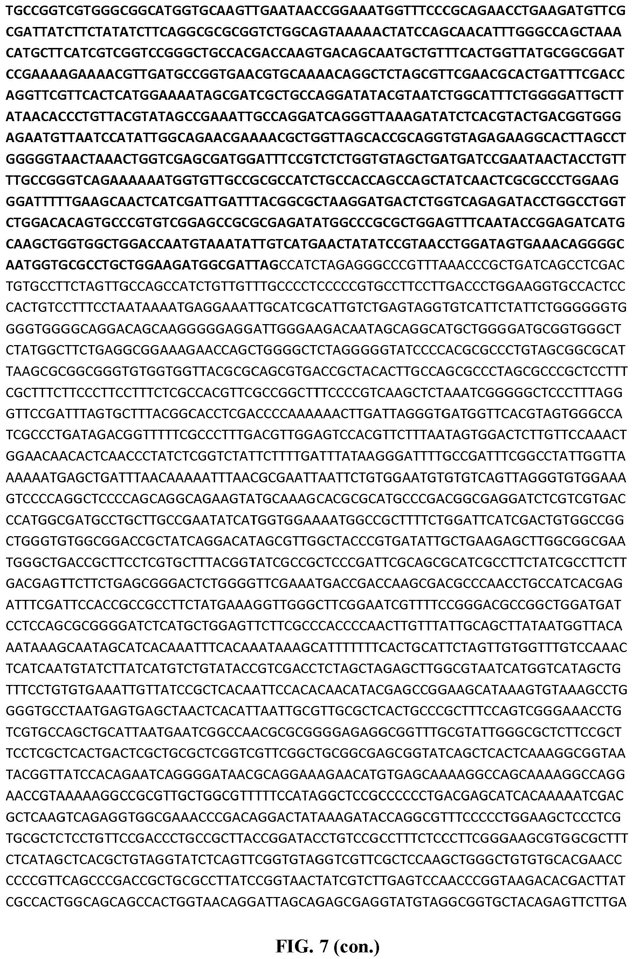

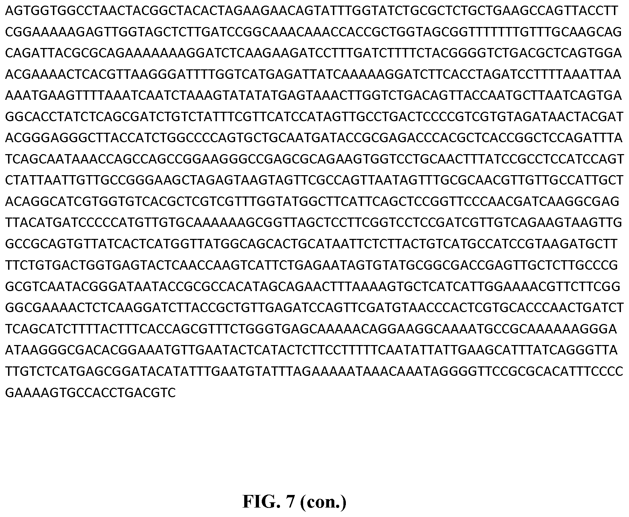

**Figure 7** shows the nucleic acid sequence [SEQ ID NO:6] for the vector comprising the V503 expression cassette containing the coding sequence for the CRE recombinase (shown in bold).

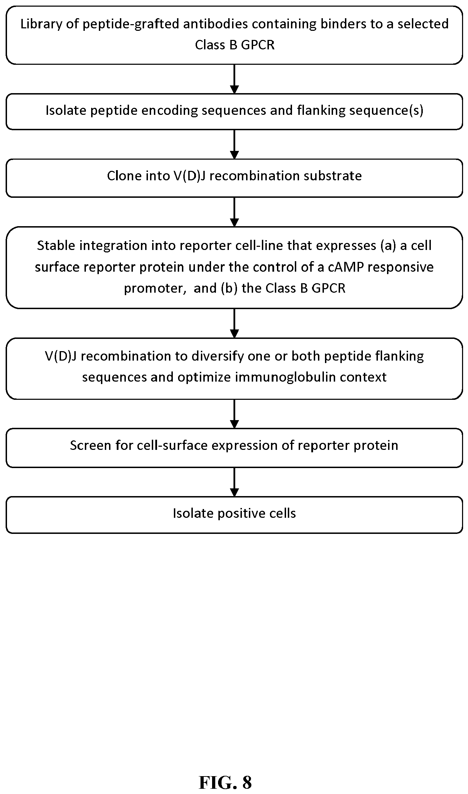

**Figure 8** presents a flow chart showing steps in the method of generating Class B GPCR agonists in one embodiment of the invention.

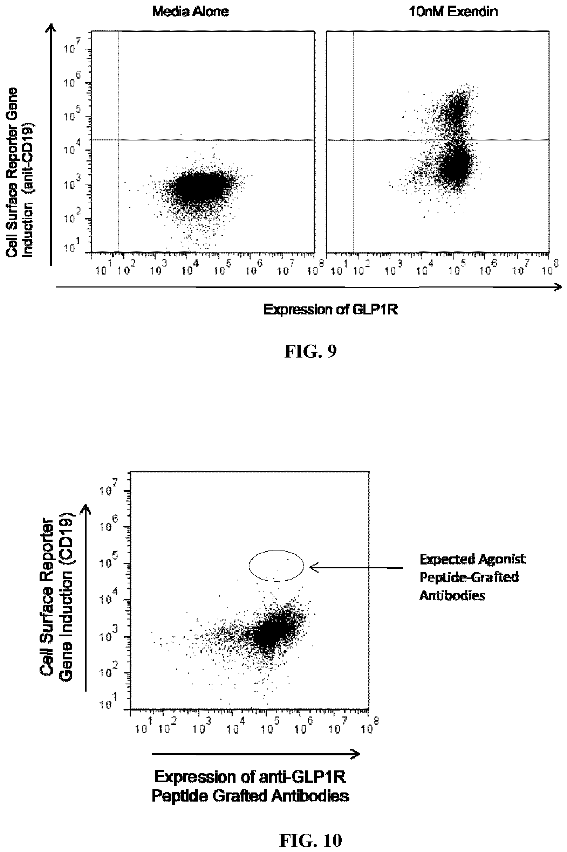

**Figure 9** shows the results of FACS analysis of the HuTARG-L696 reporter cell line after culturing with and without Exendin (10nM) for 24 hours. The cells were subsequently stained with 1ug/ul biotinylated anti-GLPIR, 1ug/ml SA647 and 1ug/ml anti-CD19-PE conjugated antibody for one hour with rotation, washed one time with PBS containing 2% FBS and analyzed by flow cytometry using the C6 Accuri (BD).
**Figure 10** shows the results of FACS analysis of the recombined cell line HuTARG ITS007-L031, which surface expresses anti-GLPIR peptide-grafted antibodies, stained with anti-CD19-PE conjugated antibody and an anti-IgG Fc Alexa-647 conjugated antibody. Cells which were surface IgG positive and CD19 positive (exemplified by circle) were FACS sorted. Heavy chain sequences were PCR amplified from the FACS sorted cells and isolated clones were analyzed for agonist activity.

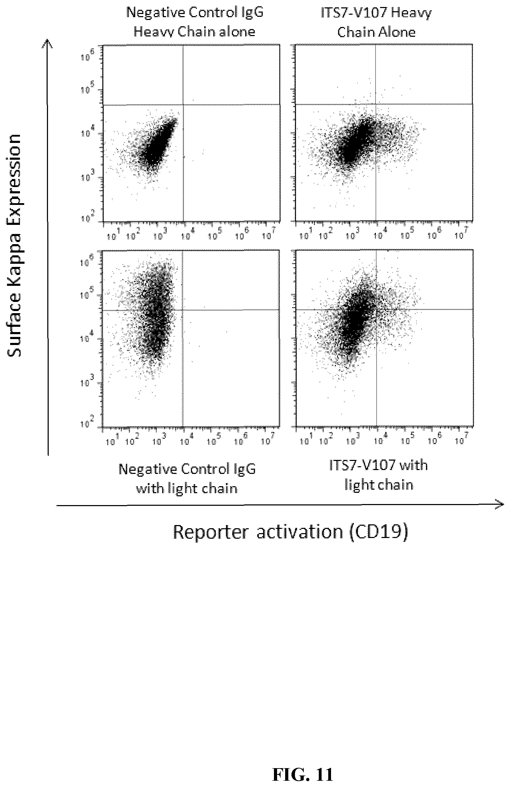

**Figure 11** shows the results of FACS analysis of HuTARG-L696 reporter cells that were transfected with expression vectors expressing an isolated exendin peptide-grafted antibody (ITS007-V107) or a negative control antibody, both with and without light chain. 48 hours post-transfection, surface kappa expression and CD19 reporter activation were determined by staining the samples with anti-Kappa Alexa488 and anti-CD 19-PE conjugated antibodies.

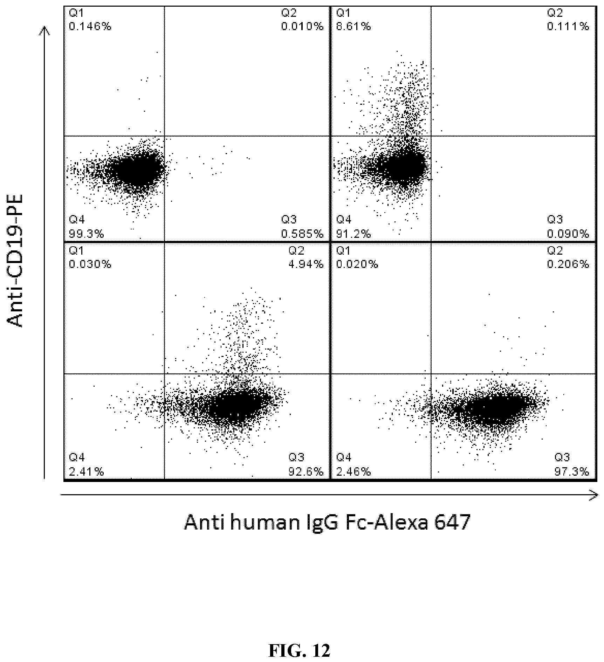

**Figure 12** shows the results of FACS analysis of L696.19 GLP1r/cAMP reporter cells that were seeded 1:3 in a 24-well plate. The following day the cells were either left untreated (upper left panel), treated with 50 nM exendin (upper right panel), or incubated with culture supernatants, 1 µg/ml, of peptide-grafted heavy chain ITS007-V125 (lower left panel) or ITS007-V129 (histidine to alanine mutant; lower right panel). Two days later cells were stained with anti-CD19-PE and anti-human IgG-Fc-647 and analyzed by FACS on a C6 Accuri (BD).

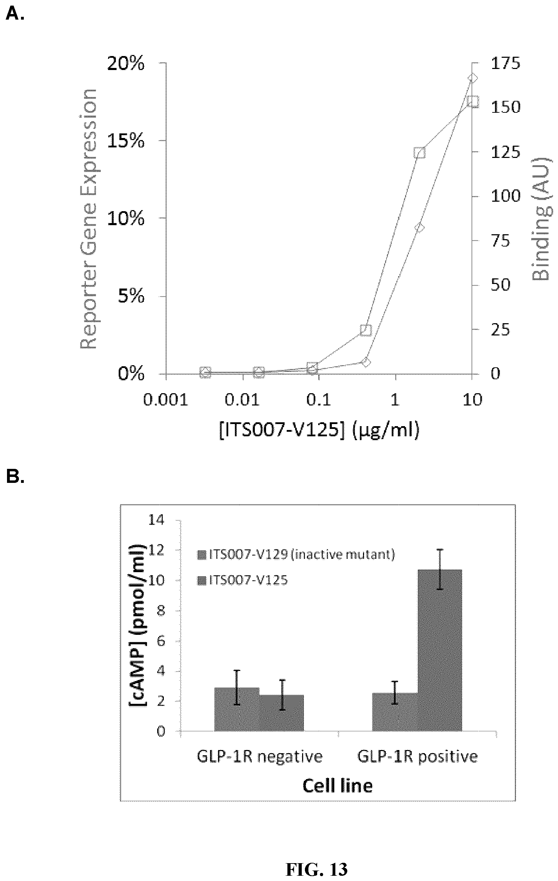

**Figure 13** presents results showing the GLP1R agonist activity of the peptide-grafted heavy-chain ITS007-V125. ITS007-V125 was cloned into a secretion vector, expressed and purified. The reporter cell line was treated with different concentrations of ITS007-V125 followed by overnight incubation. (A) shows the dose dependent binding to the GLP-1 receptor (squares) and agonist activity (diamonds) expressed as the percentage of cells expressing the surface marker and determined by FACS analysis of reporter gene expression. (B) shows the specificity of ITS007-V125 for GLP1R. A commercial ELISA (cAMP Parameter Assay Kit (R&D Systems)) was used to measure cAMP concentration produced following incubation of a reporter cell line mock transfected or transfected with the GLP1 receptor. ITS007-V129 is a mutant that binds to, but does not activate, GLP1R.

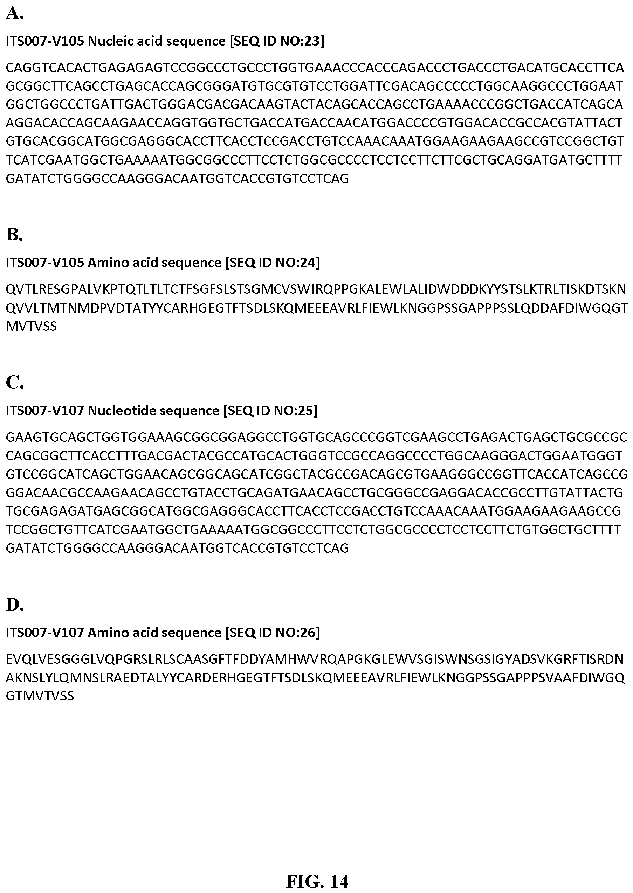

**Figure 14** presents (A) the nucleotide sequence of ITS007-V105 (VH sequence through the end of the JH) [SEQ ID NO:23]; (B) the corresponding amino acid sequence of ITS007-V105 [SEQ ID NO:24]; (C) the nucleotide sequence of ITS007-V107 (VH sequence through the end of the JH) [SEQ ID NO:25], and (D) the corresponding amino acid sequence of ITS007-V107 [SEQ ID NO:26].

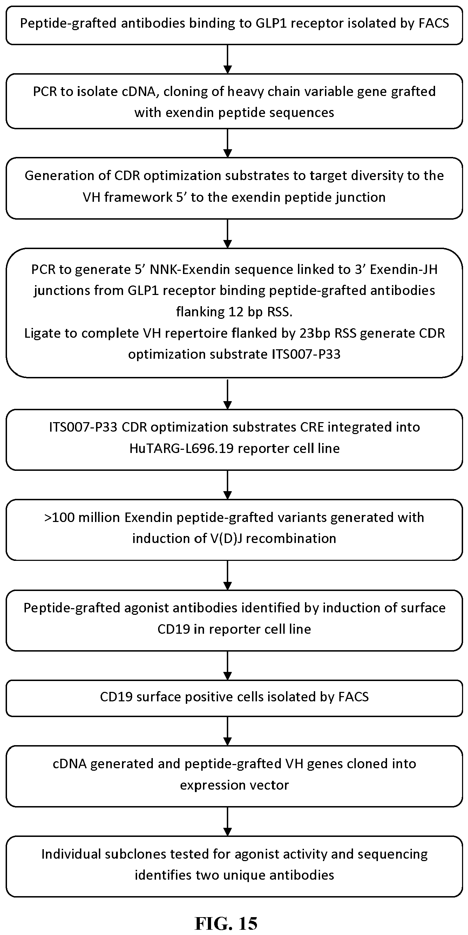

**Figure 15** presents a flow chart showing steps in the method of generating GLP1R agonists in one embodiment of the invention.

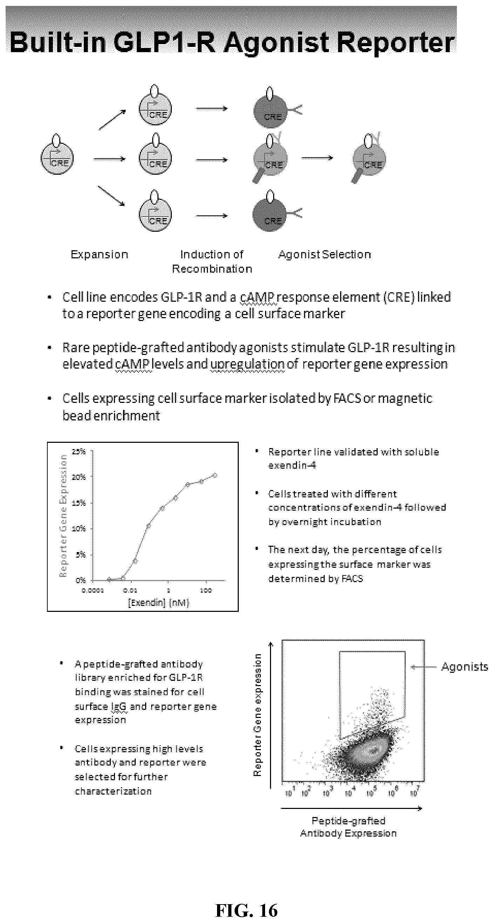

**Figure 16** presents an overview of the reporter cell line in accordance with an embodiment of the invention.

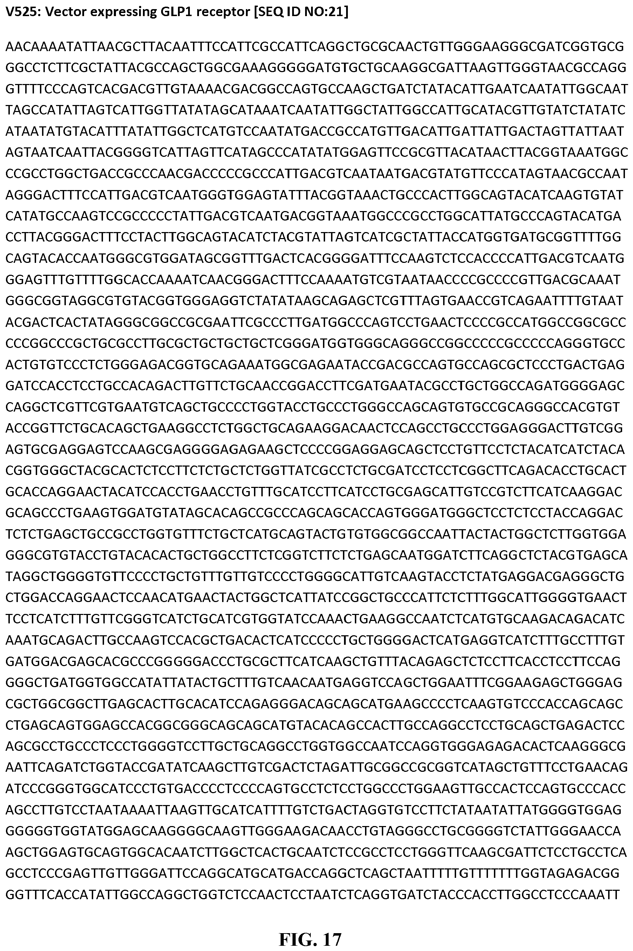

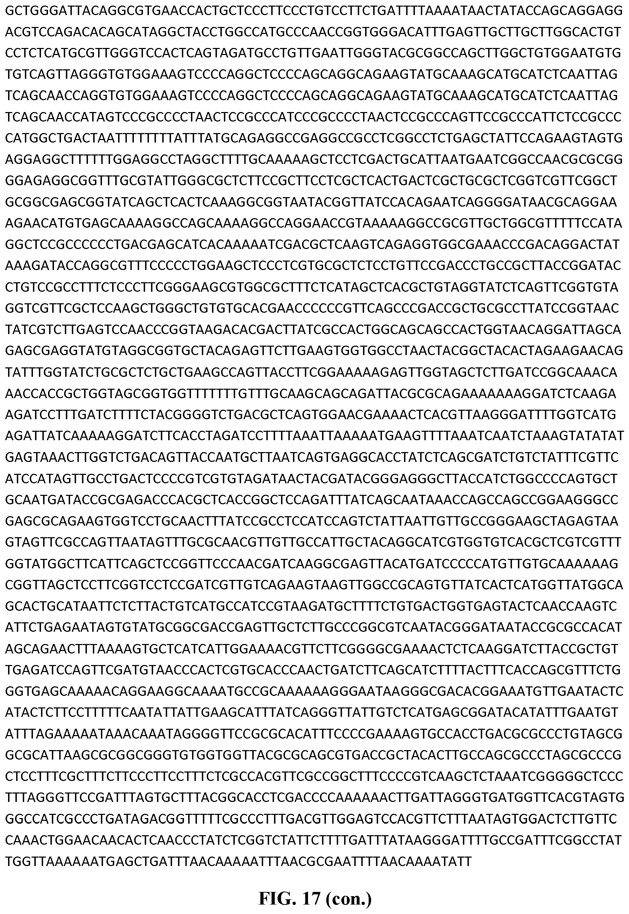

**Figure 17** presents the nucleotide sequence of vector V525 expressing the GLP1 receptor [SEQ ID NO:21].

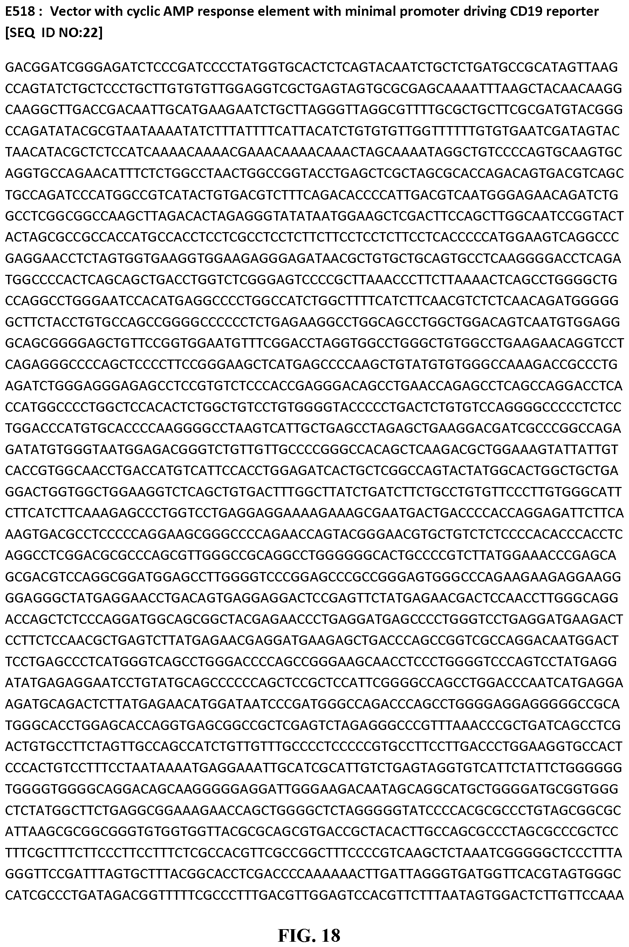

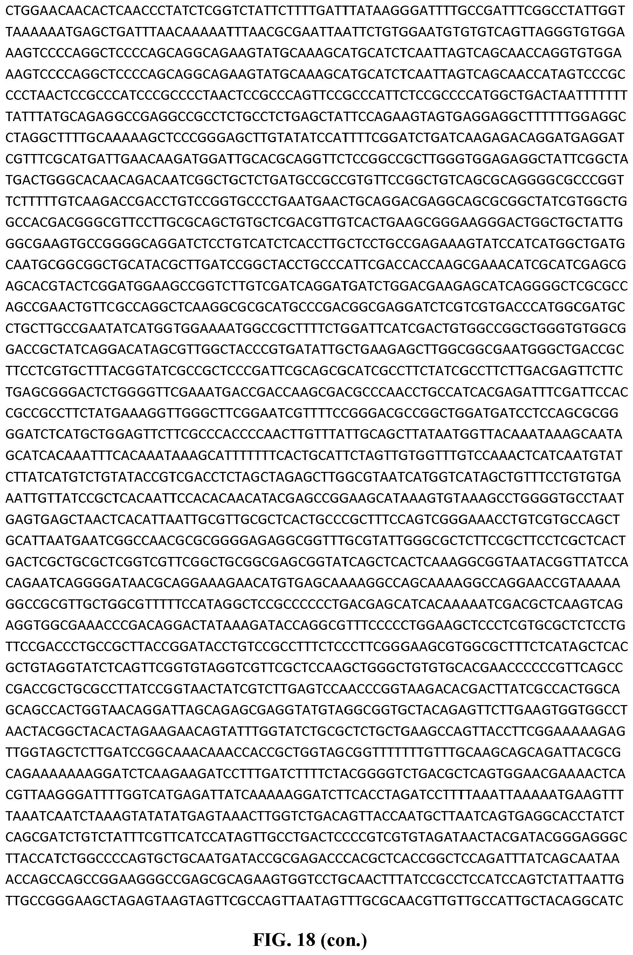

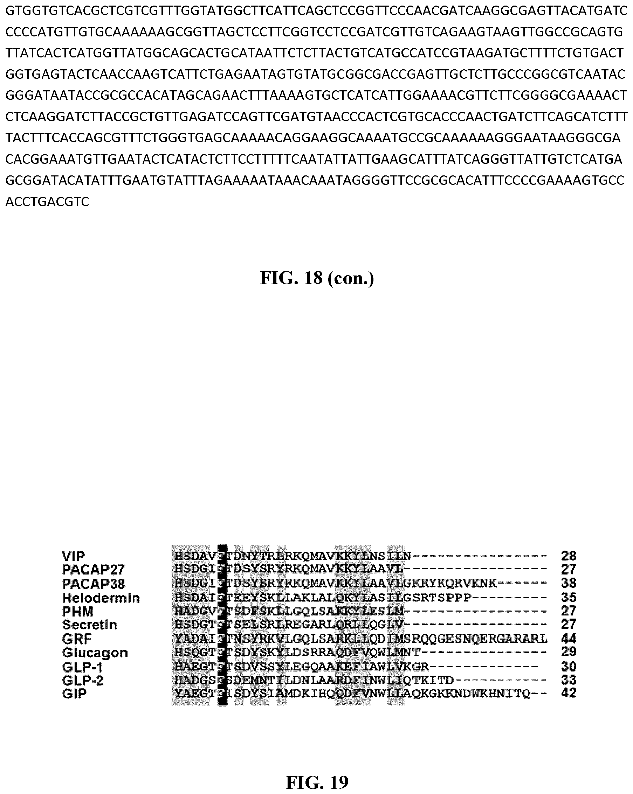

**Figure 18** presents the nucleotide sequence of vector E518 comprising a cyclic AMP response element with minimal promoter and the CD19 reporter gene [SEQ ID NO:22].
**Figure 19** presents a sequence alignment of peptides of the vasoactive intestinal peptide (VIP)/secretin family. Sequence identity is represented by a black box, and sequence homologies are represented by grey boxes. Numbers indicate the length of the peptides. (from Couvineau and Laburthe, 2012, Br J Pharmacol., 166(1):42-50).

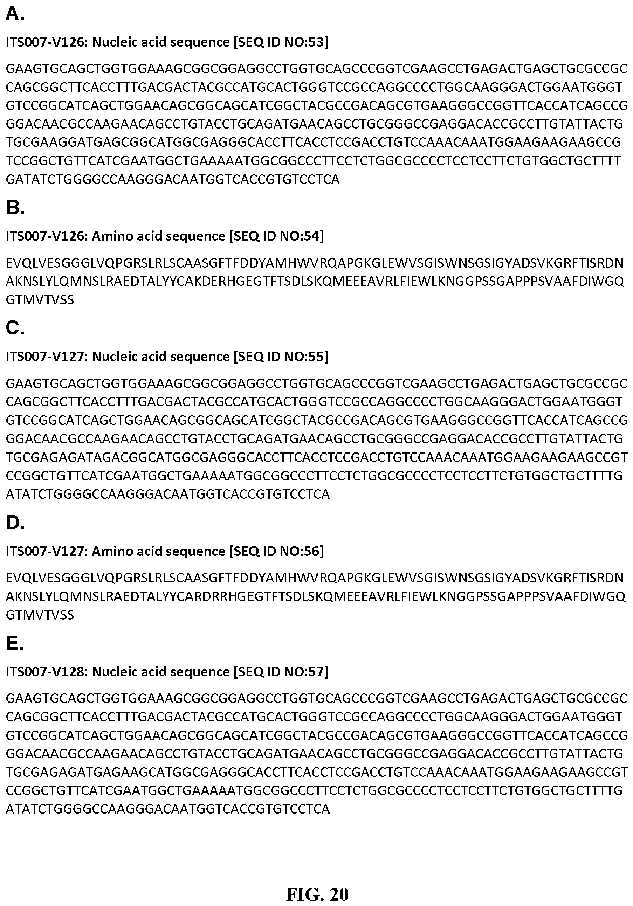

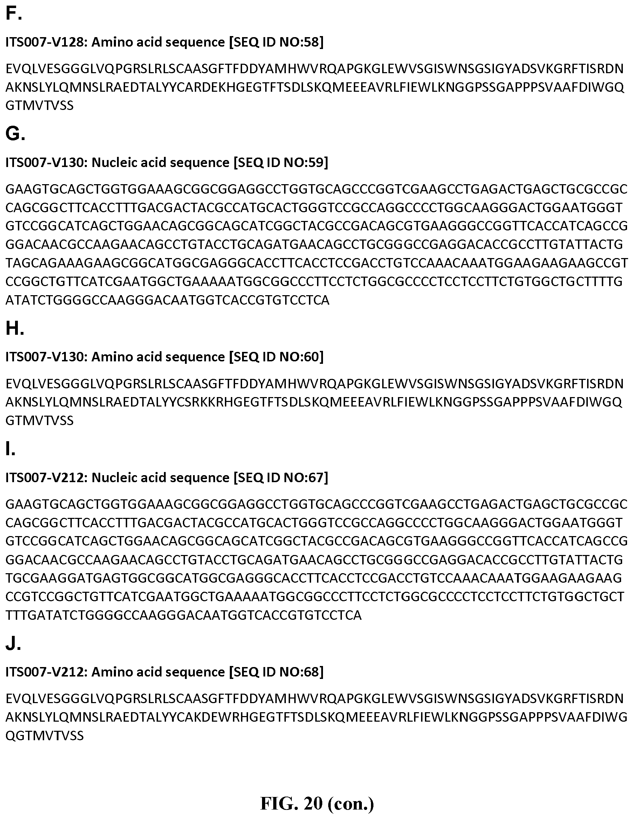

**Figure 20** presents the nucleic acid and amino acid sequences from VH to the end of JH for the variant HCAbs (A),(B) ITS007-V126 [SEQ ID NOs:53 & 54]; (C),(D), ITS007-V127 [SEQ ID NOs:55 & 56]; (E),(F) ITS007-V128 [SEQ ID NOs:57 & 58];. (G),(H) ITS007-V130 [SEQ ID NOs:59 & 60]; and (I),(J) ITS007-V212 [SEQ ID NOs:67 & 68].

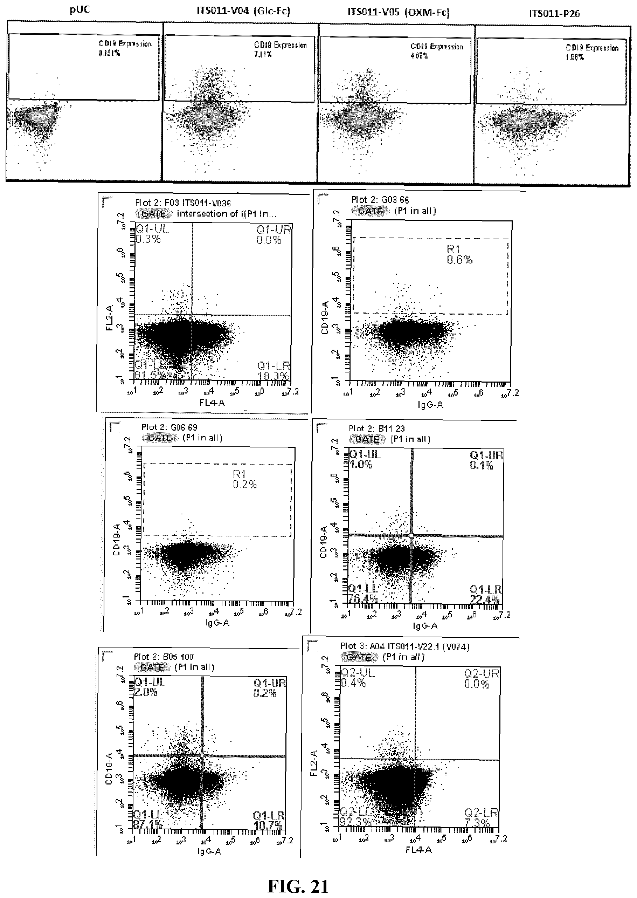

**Figure 21** shows results of FACS analysis of reporter cells transfected with expression vectors expressing glucagon (ITS011-V36, -V66 and -V69: centre row) peptide-grafted antibodies, oxyntomodulin (ITS011-V23, -V100 and -V74: bottom row) peptide-grafted antibodies or control antibodies, pUC (Empty Vector), ITS011-V04 (Glc-Fc) or ITS011-V05 (OXY-Fc) (top row).

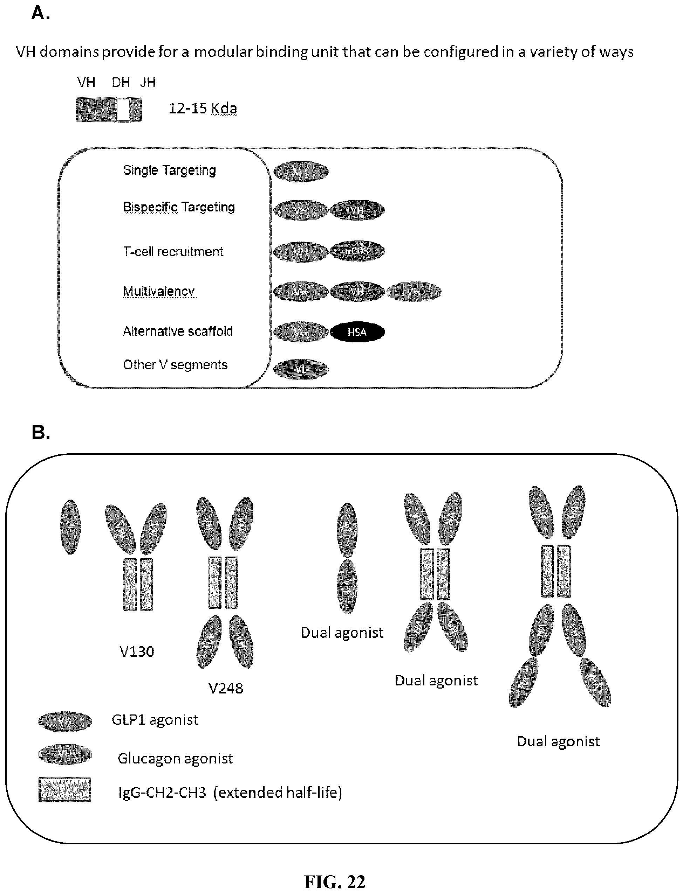

**Figure 22** presents a schematic diagram showing format options for (A) VH domain libraries, and (B) HCAb agonists in accordance with certain embodiments of the invention.

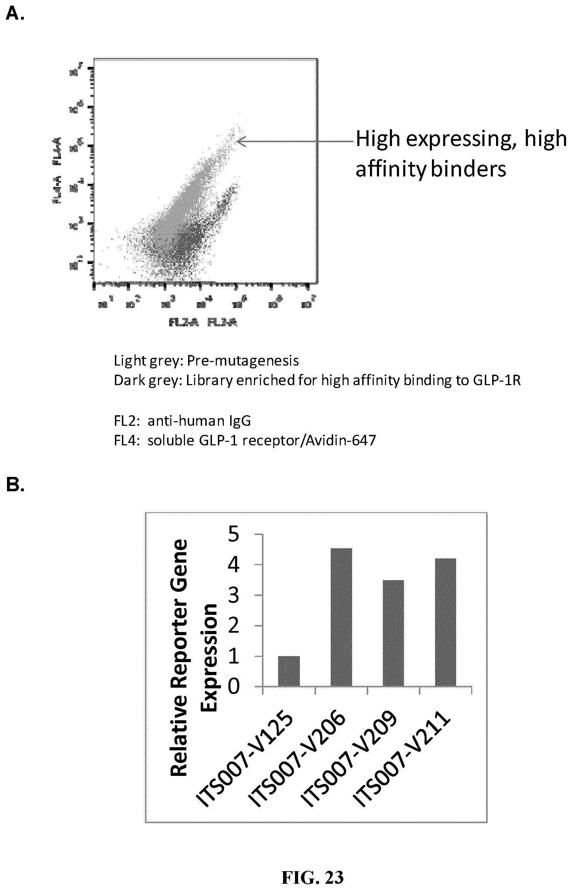

**Figure 23** shows (A) the results of FACS analysis of a HCAb variant library based on ITS007-V125, -V126 and -V130. The mutagenized library and original parent HCAbs prior to mutagenesis were stained with soluble N-terminal Biotinylated GLP1R for 1hr and then washed and incubated with anti-Human IgG PE labelled antibody as a measure of cell surface HCAb expression and Streptavidin-Alexa 647 conjugate to assess degree of binding to soluble GLP1R, and (B) potency characterization of HCAbs identified from the library with higher affinity for GLP1R. Activity was measured in a cAMP reporter based assay described above and values listed are fold improvement over the original ITS007-V125 clone prior to mutagenesis.

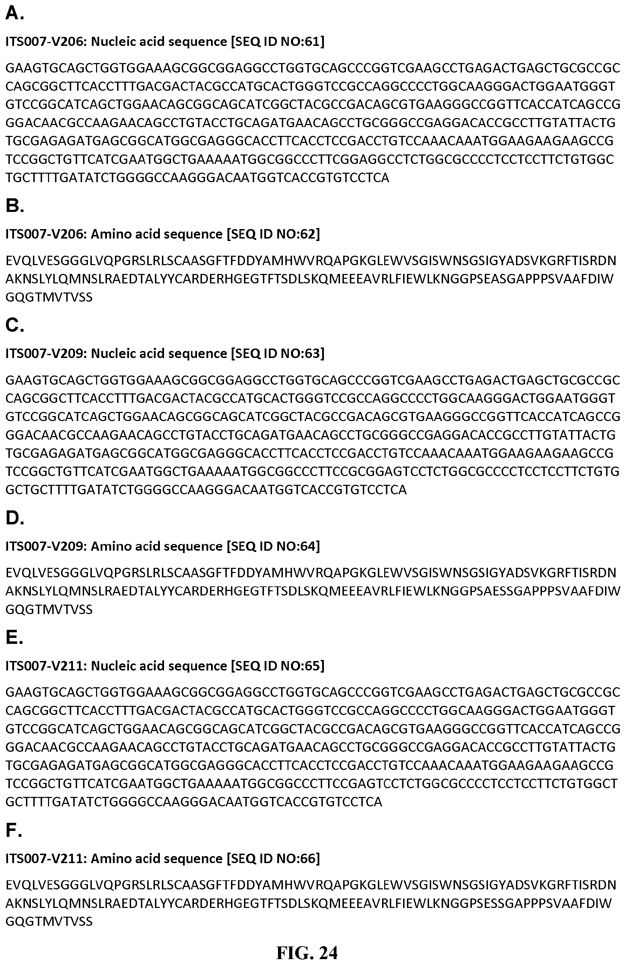

**Figure 24** shows the nucleic acid and amino acid sequences from VH to the end of JH for the variant peptide-grafted HCAbs (A),(B) ITS007-V206 [SEQ ID NOs:61 & 62]; (C),(D) ITS007-V209 [SEQ ID NOs:63 & 64], and (E),(F) ITS007-V211 [SEQ ID NOs:65 & 66].

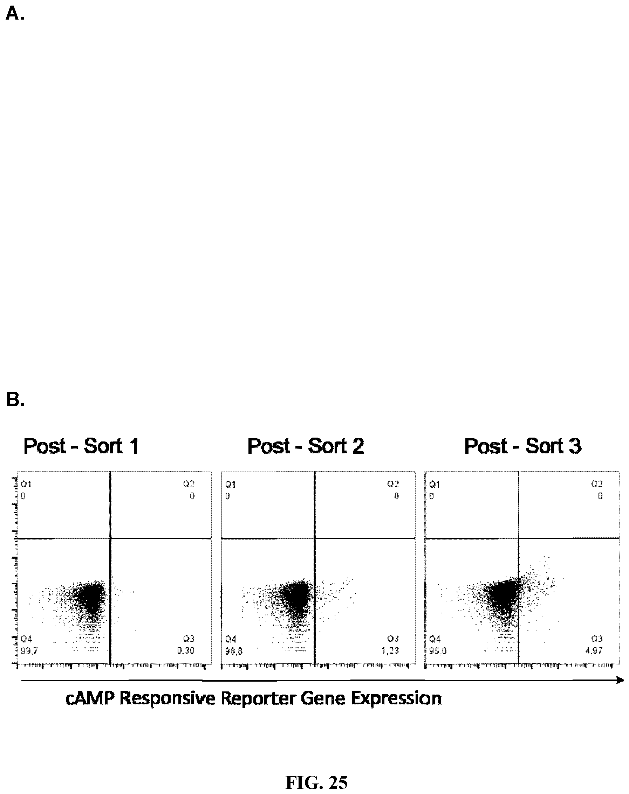

**Figure 25** shows the results of (A) the enrichment of reporter positive events for increased potency after three rounds of FACS sorting with (A) representing CDR3 mutants, and (B) representing CDR1 and CDR2 mutants, with increased activity.

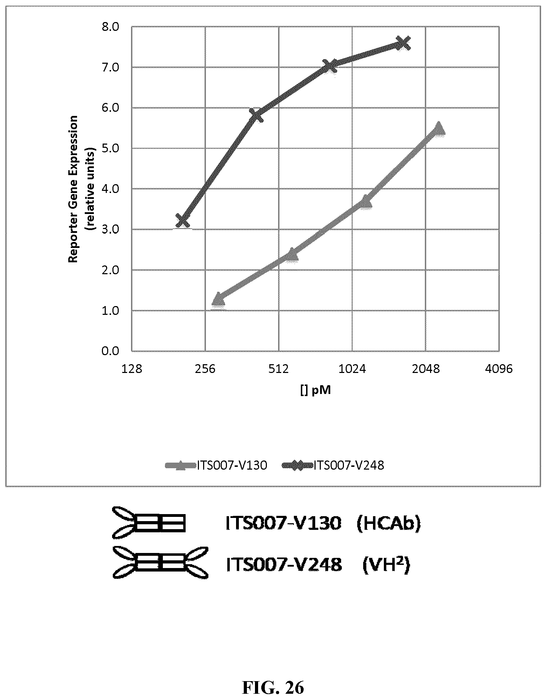

**Figure 26** shows the improved GLP1R agonist activity of a HCAb in the VH² configuration in the 293-GLP1R reporter cell line.

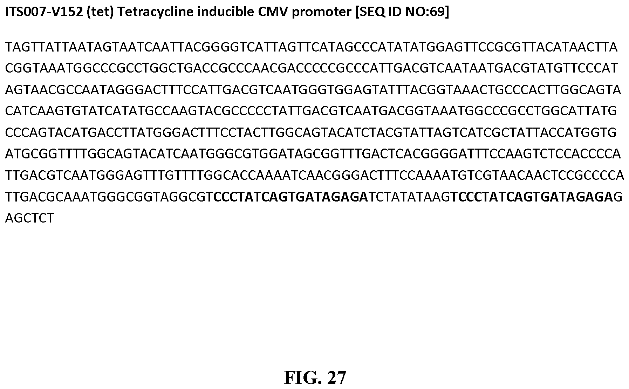

**Figure 27** presents the nucleic acid sequence of the ITS007-V152 (tet) tetracycline inducible CMV promoter [SEQ ID NO:69]. Repressor binding sites are indicated in bold flanking the TATTA box.

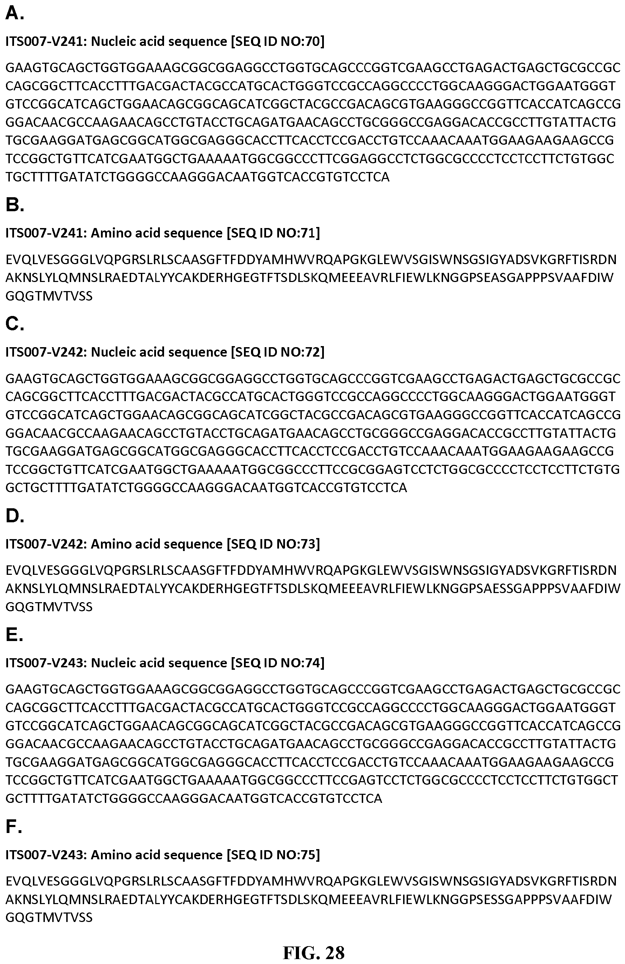

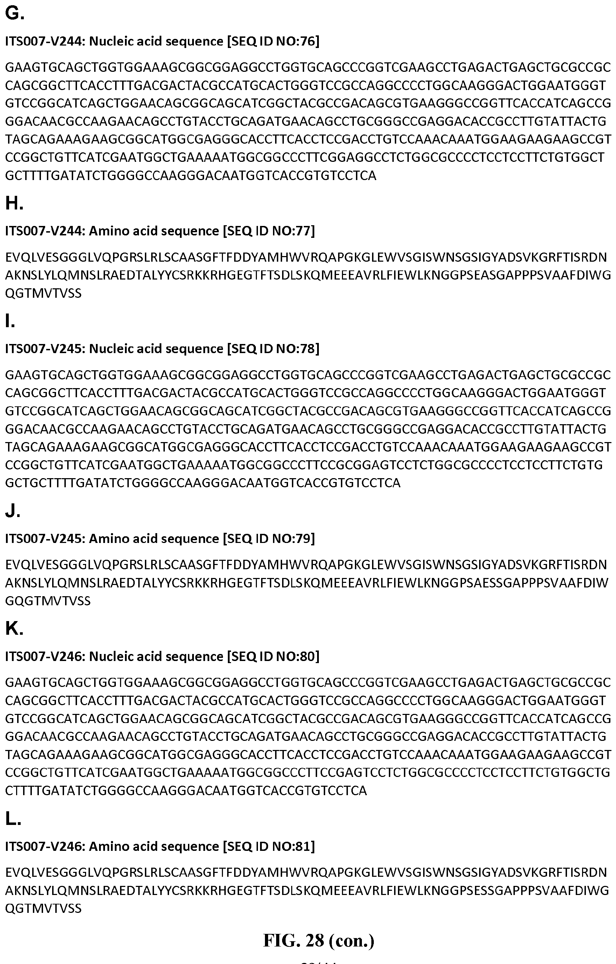

**Figure 28** presents the nucleic acid and amino acid sequences from VH to the end of JH for the variant peptide-grafted HCAbs (A),(B) ITS007-V241 [SEQ ID NOs:70 & 71]; (C),(D) ITS007-V242 [SEQ ID NOs:72 & 73]; (E),(F) ITS007-V243 [SEQ ID NOs:74 & 75]; (G),(H) ITS007-V244 [SEQ ID NOs:76 & 77]; (I),(J) ITS007-V245 [SEQ ID NOs:78 & 79], and (K),(L) ITS007-V246 [SEQ ID NOs:80 & 81].

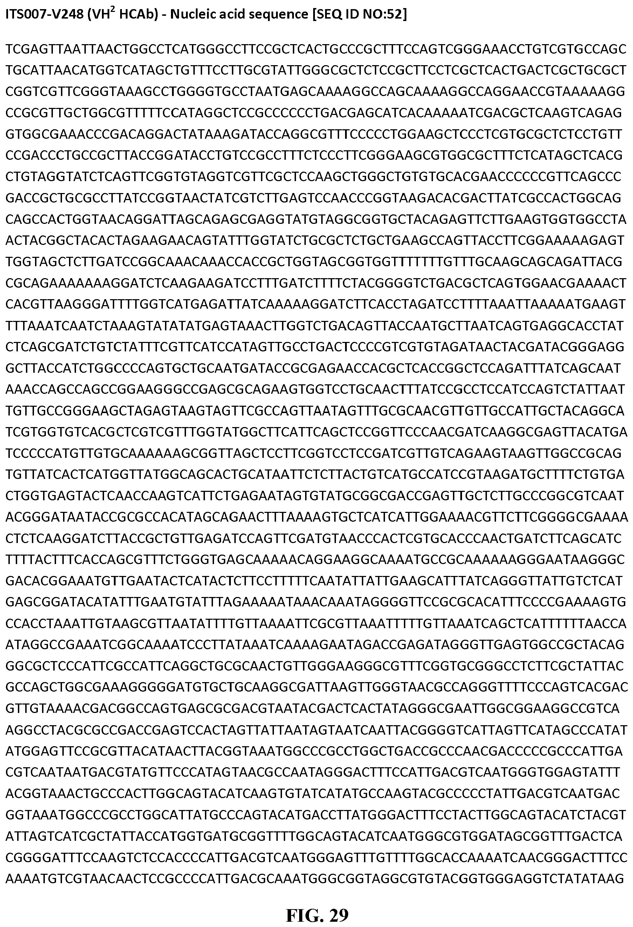

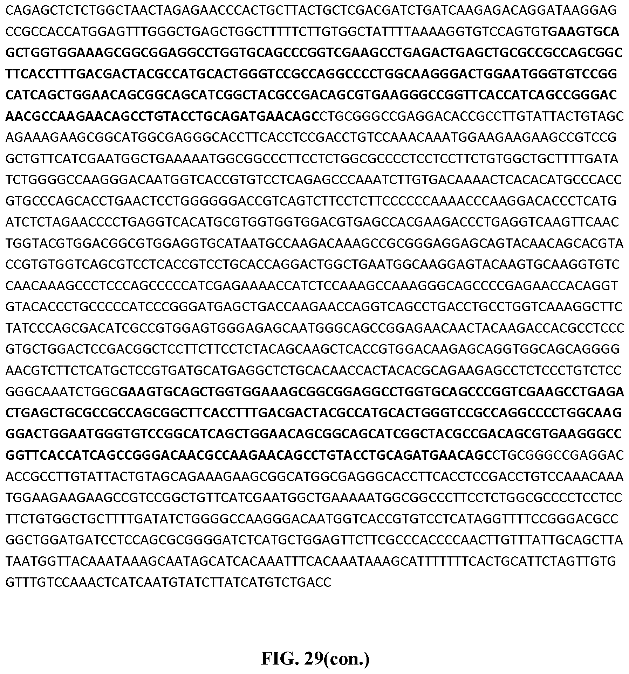

**Figure 29** presents the nucleic acid sequence for the VH² HCAb ITS007-V248 [SEQ ID NO:52].

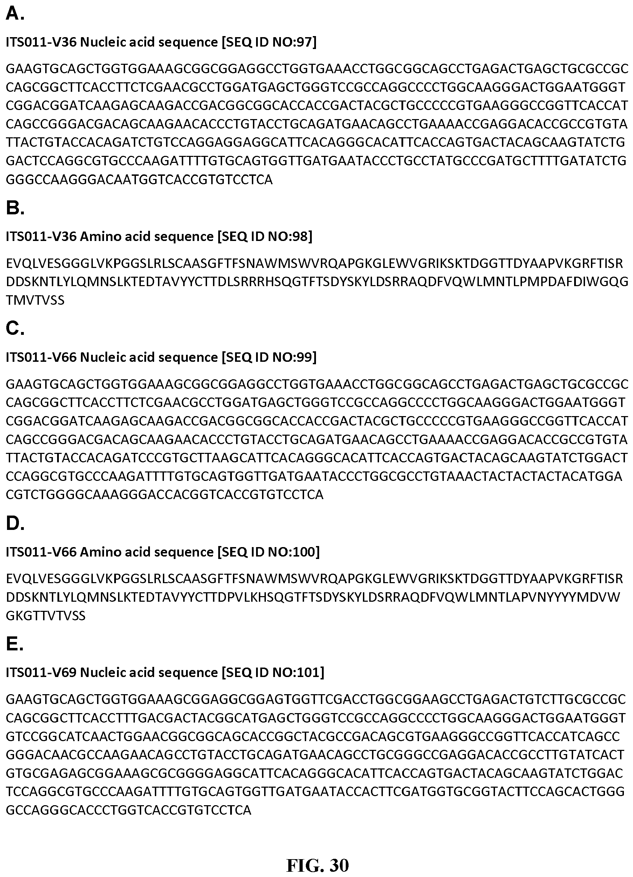

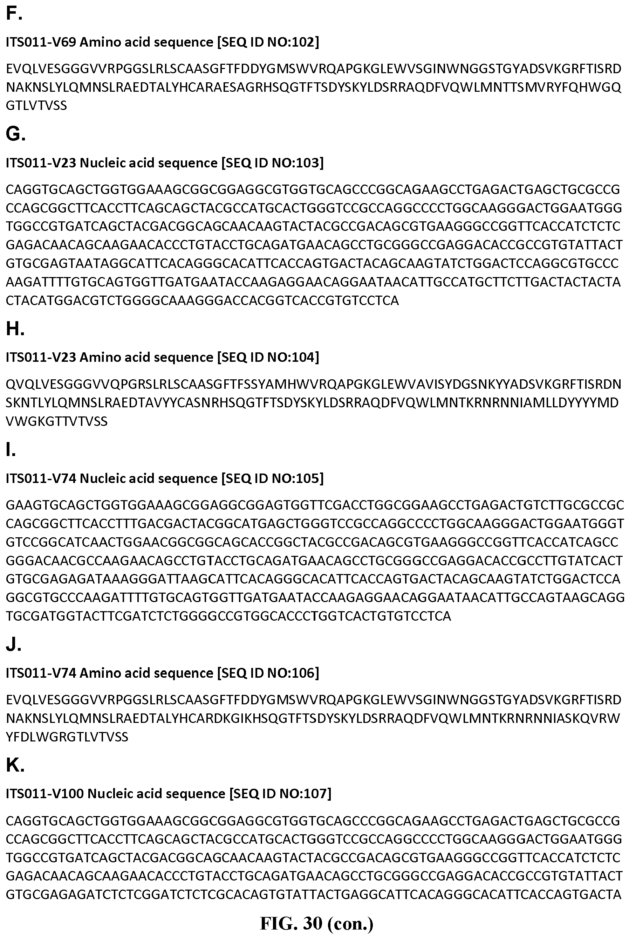

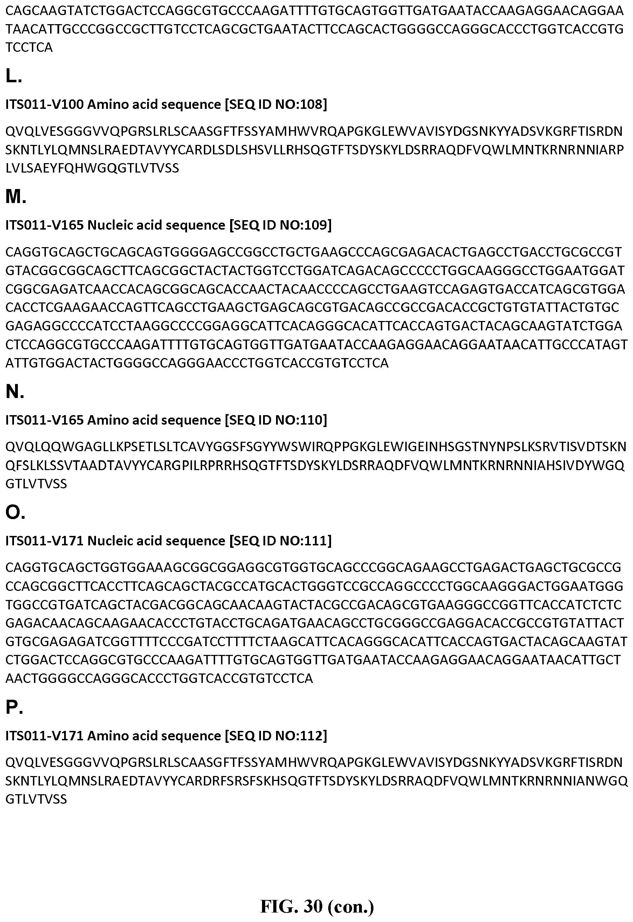

**Figure 30** presents the nucleic acid and amino acid sequences from VH to the end of JH for the variant peptide-grafted HCAbs (A),(B) ITS011-V36 [SEQ ID NOs:97 & 98]; (C),(D) ITS011-V66 [SEQ ID NOs:99 & 100]; (E),(F) ITS011-V69 [SEQ ID NOs:101 & 102]; (G),(H) ITS011-V23 [SEQ ID NOs:103 & 104]; (I),(J) ITS011-V74 [SEQ ID NOs:105 & 106]; (K),(L) ITS011-V100 [SEQ ID NOs:107 & 108]; (M),(N) ITS011-V165 [SEQ ID NOs:109 & 110], and (O),(P) ITS011-V171 [SEQ ID NOs:111 & 112].

### DETAILED DESCRIPTION OF THE INVENTION

In co-owned International Patent Application No. PCT/CA2013/050204 (WO 2013/134881) it was demonstrated that ligands to GLP1R can be used to generate a peptide-grafted antibody that can bind to this receptor. Although these peptide-grafted antibodies were able to bind to the receptor, they were not able to activate the receptor. The methods described herein allow for the generation and identification of peptide-grafted antibody agonists to Class B G-protein coupled receptors (GPCRs), including the GLP1 receptor.

In addition to ensuring that the grafted peptide itself is in the correct orientation and conformation relative to the Ig scaffold, successful peptide grafting also requires that the structure of the IgG molecule itself is not adversely affected. Both pairing and expression are potentially compromised by the grafted peptide sequences. The methods described herein allow for grafting a peptide into the complete IgG scaffold. The methods further allow for interrogation of conformations within the context of the full human VH repertoire which also provides for additional conformational opportunities.

The methods described herein may result in coupling of *de novo* protein engineering with a functional assay so that the variants can be assayed directly in the cells. While International Patent Application No. PCT/CA2013/050204 (WO 2013/134881) describes the ability to generate peptide-grafted variants within the full IgG scaffold so that the relevant sequence context and conformations generated represent the final drug candidate and does not require any additional reformatting, only binders were identified and the task of finding a rare agonist amongst these binders by known methods would not have been practical. The methods described herein allow for the screening of hundreds of millions of variants for a functional activity. These methods may allow for functional screening of peptide-grafted variants within the full scaffold directly in a mammalian cell thus allowing for the isolation of rare variants with agonist activity. The methods may allow for the generation of large numbers of variants differing in both length and composition of peptide flanking sequences directly within the complete Ig scaffold, and for assaying those variants for function without reformatting thus providing for the evaluation of an extremely large number of peptide-grafted variants and identification of peptide-grafted antibody agonists from amongst those variants.

The methods may be based on an engineered mammalian cell line that uses V(D)J recombination to generate large libraries of peptide-grafted antibody variants, displays the variants on the cell surface for the purpose of antigen selection and indicates GPCR signalling by expressing a reporter gene that can be readily detected by standard methods, such as flow cytometry.

Disclosed are peptide-grafted antibody agonists of GPCRs identified by the methods disclosed herein. The peptide-grafted antibody agonists may be heavy-chain only antibodies. Disclosed are isolated VH domains from these heavy-chain only antibodies and their use to prepare alternately formatted heavy-chain only antibodies, such as those shown in Figure 22. Disclosed are alternatively formatted heavy-chain only antibodies prepared from these isolated VH domains.

Some embodiments relate to peptide-grafted antibodies to GLP1R or the glucagon receptor, in which the grafted peptide has a sequence derived from one of the GLP1, exendin, glucagon or oxyntomodulin sequences. Amino terminal fusions to these peptides have been shown to negate activity of the peptide. As such, it was generally accepted in the field that a free amino terminus of the ligand was required for activation of the cognate receptor. As demonstrated herein, however, a free amino terminus is not required for agonist activity and using the methods disclosed herein, it is possible to generate a GLP1R or glucagon receptor agonist that contains additional amino acids at the amino terminus of the ligand. As demonstrated herein, a functional GLP1R or glucagon receptor agonist can be generated by peptide grafting of the ligand completely within an immunoglobulin.

### Definitions

Unless defined otherwise, all technical and scientific terms used herein have the same meaning as commonly understood by one of ordinary skill in the art to which this invention belongs.

As used herein, the term "about" refers to an approximately +/-10% variation from a given value. It is to be understood that such a variation is always included in any given value provided herein, whether or not it is specifically referred to.

The term "plurality" as used herein means more than one, for example, two or more, three or more, four or more, and the like.

"Naturally occurring," as used herein, as applied to an object, refers to the fact that an object can be found in nature. For example, an organism, or a polypeptide or polynucleotide sequence that is present in an organism that can be isolated from a source in nature and which has not been intentionally modified by man in the laboratory is naturally occurring.

The term "isolated," as used herein with reference to a material, means that the material is removed from its original environment (for example, the natural environment if it is naturally occurring). For example, a naturally occurring polynucleotide or polypeptide present in a living animal is not isolated, but the same polynucleotide or polypeptide separated from some or all of the co-existing materials in the natural system, is isolated. Such polynucleotides could be part of a vector and/or such polynucleotides or polypeptides could be part of a composition, and still be isolated in that such vector or composition is not part of its natural environment.

The term "recombination-competent" when used herein with reference to a host cell means that the host cell is capable of mediating RAG-1/RAG-2 recombination. The host cell may, therefore, express RAG-1 and RAG-2, or functional fragments thereof, or may be modified (for example, transformed or transfected with appropriate genetic constructs) such that it expresses RAG-1 and RAG-2, or functional fragments thereof. The expression of one or both of RAG-1 and RAG-2 in the recombination-competent host cell may be constitutive or it may be inducible. A recombination-competent host cell may optionally further express TdT, or a functional fragment thereof.

The use of the word "a" or "an" when used herein in conjunction with the term "comprising" may mean "one," but it is also consistent with the meaning of "one or more," "at least one" and "one or more than one."

As used herein, the terms "comprising," "having," "including" and "containing," and grammatical variations thereof, are inclusive or open-ended and do not exclude additional, unrecited elements and/or method steps. The term "consisting essentially of' when used herein in connection with a composition, use or method, denotes that additional elements and/or method steps may be present, but that these additions do not materially affect the manner in which the recited composition, method or use functions. The term "consisting of' when used herein in connection with a composition, use or method, excludes the presence of additional elements and/or method steps. A composition, use or method described herein as comprising certain elements and/or steps may also, in certain embodiments consist essentially of those elements and/or steps, and in other embodiments consist of those elements and/or steps, whether or not these embodiments are specifically referred to.

It is contemplated that any embodiment discussed herein can be implemented with respect to any method or composition disclosed herein, and vice versa. Furthermore, compositions and kits disclosed herein can be used to achieve methods disclosed herein.

### PEPTIDE-GRAFTED ANTIBODY AGONISTS

Certain embodiments of the invention relate to antibodies comprising one or more Class B GPCR agonist peptides grafted into one or more CDR regions. In certain embodiments these peptide-grafted antibody agonists are prepared by the methods disclosed herein.

Examples of Class B GPCRs that may be targeted include glucagon receptor and glucagon-like peptide 1 receptor (GLP1R).

As is known in the art, Class B GPCRs can be further categorized into subgroups (Harmar, (2001), Genome Biol., 2(12); reviews 3013.1-3013.10). Subfamily B1 comprises the classical hormone receptors including, for example, pituitary adenylate cyclase-activating polypeptide (PACAP) type 1 receptor, calcitonin receptor (CALCR), corticotropin-releasing hormone receptor (CRHR), glucose-dependent insulinotropic polypeptide receptor/gastric inhibitory polypeptide receptor (GIPR), glucagon receptor, glucagon-like peptide receptors (GLP1R and GLP2R), growth hormone releasing hormone receptor (GHRHR), parathyroid hormone receptor (PTHR), secretin receptor (SCTR) and vasoactive intestinal peptide (VIP) receptor. All members of the B1 subfamily are capable of regulating intracellular concentrations of cyclic AMP (cAMP).

Peptides included in the peptide-grafted antibodies may be derived from the sequence of a natural ligand for the target GPCR or from other known agonistic or binding peptides. For example, the peptide may include all or a fragment of the sequence of the natural ligand or known agonist or binder. In certain embodiments, the binding peptide may be identified by the methods described in International Patent Application No. PCT/CA2013/050204 (WO 2013/134881). In certain embodiments, it is also contemplated that antagonistic or inactive peptides may be used as a starting sequence with the methods described herein being used to introduce sufficient sequence diversity to result in a grafted peptide having agonist activity. Non-limiting examples of ligands to Subfamily B1 GPCRs are shown in Table 1A (see also, Harmar, 2001, *ibid.*).

**Table 1A: Ligands for Subfamily B1 Receptors**

| **Receptor** | **Ligands** |
|---|---|
| Calcitonin receptor (CALCR) | Calcitonin |
| | Adrenomedullin |
| | Peptide histidine methionine (PHM) |
| Calcitonin receptor-like receptor (CALCRL) | Calcitonin gene related peptide |
| | Amylin |
| | CGRP |
| Corticotropin-releasing factor 1 receptor | Corticotropin releasing factor (CRF) |
| | Urocortin |
| Corticotropin-releasing factor 2 receptor | Urocortin |
| | Urocortin II |
| | Urocortin III |
| Glucose-dependent insulinotropic polypeptide receptor/gastric inhibitory polypeptide receptor (GIPR) | Gastric inhibitory peptide (GIP) |
| Glucagon receptor | Glucagon |
| | Oxyntomodulin |
| Glucagon-like peptide 1 receptor (GLP1R) | GLP1 |
| | Exendin |
| | Oxyntomodulin |
| Glucagon-like peptide 2 receptor (GLP2R) | GLP-2 |
| Growth hormone releasing hormone receptor (GHRHR) | Growth hormone releasing hormone |
| | Growth hormone releasing factor (GRF) |
| Parathyroid hormone 1 receptor (PTH1R) | Parathyroid hormone (PTH) |
| | PTH-related peptide |
| | PTH (1-34) peptide |
| Parathyroid hormone 2 receptor (PTH2R) | TIP39 |
| Secretin receptor (SCTR) | Secretin |
| Vasoactive intestinal peptide receptor 1 (VPAC₁) | Vasoactive intestinal peptide (VIP) |
| | Pituitary adenylate cyclase-activating polypeptide (PACAP) |
| | Helodermin |
| Vasoactive intestinal peptide receptor 2 (VPAC₂) | Vasoactive intestinal peptide (VIP) |
| | Pituitary adenylate cyclase-activating polypeptide (PACAP) |
| | Helodermin |
| Pituitary adenylate cyclase-activating polypeptide type I receptor (PAC₁) | Pituitary adenylate cyclase-activating polypeptide (PACAP) |

The amino acids sequences for various ligands and agonists of Class B GPCRs are known in the art and are available from publicly accessible databases such as the GenBank sequence database maintained by the National Center for Biotechnology Information. Exemplary sequences are provided in Table 1B below (see also Figure 23):

**Table 1B: Sequences of Ligands for Subfamily B1 Receptors**

| **Ligand** | **Sequence** | **SEQ ID NO** |
|---|---|---|
| Exendin-4 | HGEGRFTSDLSKQMEEEAVRLFIEWLKNGGPSSGAPPPS | **7** |
| GLP1 | HAEGTFTSDVSSYLEGQAAKEFIAWLVKGR | **8** |
| GLP2 | HADGSFSDEMNTILDNLAARDFINWLIQTKITDR | **9** |
| Glucagon | HSQGTFTSDYSKYLDSRRAQDRVQWLMNT | **10** |
| Oxyntomodulin | HSQGTFTSDYSKYLDSRRAQDFVQWLMNTKRNRNNIA | **34** |
| PACAP-38 | HSDGIFTDSYSRYRKQMAVKKYLAAVLGKRYKQRVKNK | **11** |
| PTH | | **12** |
| VIP | HSDAVFTDNYTRLRKQMAVKKYLNSILN | **43** |
| PACAP-27 | HSDGIFTDSYSRYRKQMAVKKYLAAVL | **44** |
| Helodermin | HSDAIFTEEYSKLLAKLALQKYLASILGSRTSPPP | **45** |
| PHM | HADGVFTSDFSKLLGQLSAKKYLESLM | **46** |
| Secretin | HSDGTFTSELSRLREGARLQRLLQGLV | **47** |
| GRF | | **48** |
| GIP | | **49** |

Peptides corresponding to homologues of the ligands described above and in Table 1 are also known in the art.

The peptide-grafted antibody agonist may comprise a single peptide or it may comprise a plurality of peptides, for example, 2, 3, 4, 5 or 6 peptides. When the peptide-grafted antibody agonist comprises a plurality of peptides, the peptides may be grafted into the same CDR or into different CDRs. Preferably the peptides are grafted into different CDRs. The plurality of peptides may be agonists of the same GPCR or they may target different GPCRs. In certain embodiments, therefore, the peptide-grafted antibody agonist may have multiple specificities. For example, the peptide-grafted antibody agonist could be a dual agonist targeting two different GPCRs, such as GLP1R and the glucagon receptor.

In certain embodiments of the invention, the peptide-grafted antibody agonists described herein comprise a peptide agonist of the GLP1 receptor. Agonist peptides suitable for targeting GLP1R include, for example, GLP1 [SEQ ID NO:8] and exendin [SEQ ID NO:7], as well as modified versions and functional fragments of these peptides.

In certain embodiments, the peptide-grafted antibody agonists described herein comprise a peptide agonist of the glucagon receptor. Agonist peptides suitable for targeting the glucagon receptor include, for example, glucagon [SEQ ID NO:10] and oxyntomodulin [SEQ ID NO:34], as well as modified versions and functional fragments of these peptides.

The inserted peptide sequence includes an additional basic amino acid upstream and adjacent to the N-terminal amino acid of the peptide, which is an arginine or lysine. As shown herein, certain peptide sequences that comprise an N-terminal histidine benefit from the insertion of a basic amino acid immediately N-terminal to this histidine. Non-limiting examples of native peptide agonists of Class B GPCRs that comprise an N-terminal histidine are exendin, GLP1, GLP2, glucagon, oxyntomodulin, PACAP-27, PACAP-38, VIP, helodermin, PHM and secretin.

In certain embodiments, the inserted peptide sequence will include heterologous flanking sequences either at the N-terminal end, the C-terminal end, or at both. Typically, the flanking sequences will include between one and about 20 amino acids, for example, between one and about 18 amino acids, between one and about 15 amino acids, between one and about 12 amino acids, and between one and about 10 amino acids, or any amount therebetween.

In certain embodiments of the invention, the peptide-grafted antibody comprises a heterologous flanking sequence at the N-terminal end of the inserted peptide and optionally a heterologous flanking sequence at the C-terminal end. In some embodiments, the peptide-grafted antibody comprises a flanking sequence at the N-terminal end of the inserted peptide in which the amino acid proximal to the N-terminal amino acid of the peptide is a basic amino acid, and optionally further comprises a flanking sequence at the C-terminal end. In some embodiments, the peptide-grafted antibody comprises a flanking sequence at the N-terminal end of the inserted peptide in which the amino acid proximal to the N-terminal amino acid of the peptide is an arginine or lysine, and optionally further comprises a flanking sequence at the C-terminal end.

In certain embodiments, the peptide-grafted antibody optionally includes a heterologous flanking sequence at the C-terminal end, but does not include a heterologous flanking sequence at the N-terminal end of the inserted peptide. In some embodiments, the peptide-grafted antibody optionally comprises a flanking sequence at the C-terminal end, does not include a flanking sequence at the N-terminal end of the inserted peptide but is inserted within the CDR region such that the amino acid proximal to the N-terminal amino acid of the peptide is predefined. For example, the amino acid proximal to the N-terminal amino acid of the inserted peptide may be a basic amino acid. In some embodiments, the inserted peptide optionally comprises a flanking sequence at the C-terminal end, and is inserted into the CDR region such that the amino acid proximal to the N-terminal amino acid of the peptide is an arginine or lysine.

In some embodiments, the GLP1R or glucagon receptor peptide agonist comprises a flanking sequence at the N-terminal end of the inserted peptide in which the amino acid proximal to the N-terminal amino acid of the peptide is an arginine or lysine, and optionally further comprises a flanking sequence at the C-terminal end. In some embodiments, the GLP1R or glucagon receptor peptide agonist optionally comprises a flanking sequence at the C-terminal end, and is inserted into the CDR region such that the amino acid proximal to the N-terminal amino acid of the peptide is an arginine or lysine.

A peptide that is described as being inserted into a CDR region may be inserted within the CDR such that the amino acids that make up the CDR are retained, or it may replace part or all of the CDR. The peptide-grafted antibodies may comprise an agonist peptide inserted into, or replacing part or all of, one CDR, or it may comprise a plurality of peptides with each peptide inserted into, or replacing part or all of, a CDR. The peptide-grafted antibodies may comprise a peptide grafted into a heavy chain CDR or into a light chain CDR or into both. In some embodiments, the peptide-grafted antibodies may comprise a peptide grafted into a heavy chain CDR3 or into a light chain CDR3 or into both. In certain embodiments, the peptide-grafted antibodies may comprise a peptide grafted into at least a heavy chain CDR. In some embodiments, the peptide-grafted antibodies may comprise a peptide grafted into the heavy chain CDR3. In some embodiments, the peptide replaces the D segment in the heavy chain CDR3.

The final peptide-grafted antibody may be a full-length immunoglobulin (such as a full-length IgA, IgA2, IgD, IgE, IgG (*i.e.* an IgG1, IgG2, IgG3 or IgG4) or IgM) or an immunoglobulin fragment (such as a Fab, Fab', F(ab')₂, Fd, Fvor single-chain Fv (scFv) fragment, or a single domain antibody). Full-length immunoglobulins may comprise both a heavy chain and a light chain, or they may include just a heavy chain or just a light chain. Certain embodiments of the invention relate to peptide-grafted antibodies which are full-length IgG immunoglobulins. In some embodiments, the peptide-grafted antibody comprises just the heavy chain of a full-length IgG immunoglobulin, or a functional fragment thereof.

In some embodiments, the peptide-grafted antibody is a heavy chain only antibody (HCAb). In this context, a HCAb may include the VH domain together with the CH1, CH2 and CH3 regions, or it may contain the VH domain and the CH2 and CH3 regions only. In the latter conformation, the VH domain is attached directly to the hinge region of the antibody. The HCAb may include two "arms" of the antibody (*i.e*. one arm comprising a VH domain and constant regions linked via a disulphide bridge to a second arm comprising a VH domain and constant regions), or it may include just a single arm (*i.e.* one arm comprising a VH domain and constant regions). Fragments of HCAbs are also contemplated, for example, fragments including two VH domains and the hinge region only without any constant regions, or a single VH domain.

The modular nature of the fully human VH single domain makes it a versatile building block for generating alterative format HCAbs that may find use as therapeutics. The VH single domain is approximately 12-15 KDa and contains only the variable gene segment through to the end of the JH gene segment. Certain embodiments relate to alternative format HCAbs comprising two or more VH domains as outlined in Figure 22A & B. The alternative format HCAbs may be monospecific or they may be bispecific or they may be multispecific. For example, in some embodiments, the alternative format HCAb may comprise two or more linked VH domains, each domain comprising a different peptide agonist, and each peptide agonist targeted to a different GPCR. In some embodiments, these VH domains may be attached to constant regions. In some embodiments, the alternative format HCAbs may comprise a CH2 region, a CH3 region, a first VH domain attached to the CH2 region and one or more VH domains attached to the CH3 region. In this latter format the VH domains may be identical or different. When the VH domains are different they may, for example, target different GPCR thus providing an HCAb with dual or multi-specificity. In some embodiments, one or more VH domains may be attached to an alternate scaffold, such as human serum albumin (HSA).

In certain embodiments, the peptide-grafted antibody is an HCAb comprising one or more Class B GPCR agonist peptides grafted into one or more CDR regions. In some embodiments, the peptide-grafted antibody is an HCAb comprising a plurality of Class B GPCR agonist peptides grafted into one or more CDR regions. In some embodiments, the peptide-grafted antibody is an HCAb comprising a plurality of Class B GPCR agonist peptides each peptide grafted into a different CDR region. In some embodiments, the peptide-grafted antibody is an HCAb comprising a peptide targeting GLPR and a peptide targeting the glucagon receptor.

Although the foregoing description has focussed primarily on the use of V(D)J recombination to peptide graft into the heavy chain variable region, it will be readily appreciated that the same approach can be used with the light chain variable region simply by replacing the heavy chain VH and JH gene segments with the light chain variable (VL) and joining (JL) gene segments. In such embodiments, the peptide sequence may be used as an artificial D gene segment. Certain embodiments, therefore, relate to peptide-grafted agonist antibodies comprising one or more peptides grafted into the light chain variable region. These peptide-grafted antibodies may optionally further comprise one or more peptides grafted into their cognate heavy chain variable regions.

The peptide-grafted agonist antibodies and fragments thereof have utility in a variety of contexts including as therapeutics, diagnostics and as research reagents. For example, peptide-grafted agonists of GLP1R, GLP2R or the glucagon receptor may find therapeutic use in the management of diabetes and/or weight loss; peptide-grafted agonists of PTH1R may find therapeutic application in the treatment of osteoporosis; and peptide-grafted agonists of VPAC₁ or VPAC₂ have potential as therapeutics for inflammatory and neurodegenerative diseases. In addition, the antibodies or fragments thereof may find use in diagnostic applications, for example when labelled, the antibodies or fragments may be used as diagnostic and/or imaging agents.

### METHODS

Disclosed are high-throughput methods of identifying peptide-grafted antibody agonists of Class B GPCRs. The methods make use of a reporter cell line that expresses the target GPCR and also includes coding sequences for a surface-expressed reporter protein under control of a cAMP responsive promoter. Typically, the reporter cell line may be a mammalian cell line. The cell line may be a human cell line.

In general, the methods described herein start with a library of polynucleotides encoding peptide-grafted antibodies, each antibody comprising at least one GPCR-targeting peptide grafted into a CDR region. Preferably at least some members of the library will have been shown to bind to the target GPCR. The library may have been generated by various art known methods, including methods incorporating random mutagenesis and/or phage display. The library may be generated using the *de novo* antibody generation methods described in International Patent Application No. PCT/CA2013/050204 (WO 2013/134881). This approach is based on generation of V(D)J recombination substrates which comprise the full repertoire of human VH and JH sequences or the full repertoire of VL and JL sequences and in which the peptide sequences, with optional flanking sequences, replace the D segments or in the case of VL and JL sequences, function as artificial D segments. The substrates are introduced into recombination competent host cells and allowed to undergo recombination. The approach thus allows introduction of diversity into the regions flanking the inserted peptide through V(D)J recombination with simultaneous interrogation of conformations of the grafted peptide within the context of the full human VH or VL repertoire.

In general when the peptide-grafted antibodies are heavy chain only antibodies, the library of peptide-grafted antibodies may be generated using the full repertoire of VH sequences. However, the use of a restricted number of VH gene segments is also contemplated. Examples of restricted sets would include sets comprising one or a combination of the following VH gene segments that have been shown to express well in the HCAb format: VH1-8, VH3-11, VH3-15, VH3-20, VH3-30 and VH4-34.

The initial library of polynucleotides encoding peptide-grafted antibodies is screened functionally using the reporter cell line to identify those peptide-grafted antibodies having agonist activity against the target GPCR. The library may be screened directly, or the peptide-grafted antibody encoding polynucleotides may be subjected to a further V(D)J recombination step to increase the diversity of the library.

The peptide-grafted antibodies may be subjected to a further V(D)J recombination step. An overview of the steps in an exemplary method comprising a further V(D)J recombination step is provided in Figure 8. The V(D)J recombination step may introduce diversity at both ends of the grafted peptide, or it may be targeted to introduce diversity at just one terminus of the grafted peptide. As the N-terminal region of certain GPCR agonists is believed to be important for activity, it may be advantageous to target the additional V(D)J recombination step to introduce additional diversity at the N-terminal end of the grafted peptide.

The V(D)J recombination step is carried out essentially as described in International Patent Application No. PCT/CA2013/050204 (WO 2013/134881). For this step, sequences encoding the grafted peptide and flanking regions as appropriate are amplified from the library with primers that introduce the required RSS sequence or sequences for RAG-mediated recombination, and the amplified sequences are subsequently ligated into a V(D)J substrate polynucleotide that comprises sequences encoding one or a plurality of VH segments and/or one or a plurality of JH segments, together with the required RSS sequences, or sequences encoding one or a plurality of VL segments and/or one or a plurality of JL segments, together with the required RSS sequences. One skilled in the art will appreciate that if additional sequence diversity is to be introduced to one end of the grafted peptide, then the amplified sequences from the original polynucleotide encoding the peptide-grafted antibody will include sequences adjacent to the terminus that is not being diversified. For example, if additional diversity is to be targeted into the N-terminal region of the grafted peptide, then the amplified sequences from the original polynucleotide encoding the peptide-grafted antibody may include the peptide-JH or peptide-JL junction and JH or JL sequences, and the V(D)J substrate polynucleotide may include sequences encoding one or a plurality of VH or VL segments only. Likewise, if additional diversity is to be targeted to the C-terminal region of the grafted peptide, then the amplified sequences from the original polynucleotide encoding the peptide-grafted antibody may include the VH-peptide or VL-peptide junction and VH or VL sequences, and the V(D)J substrate polynucleotide may include sequences encoding one or a plurality of JH or JL segments only.

The primers used to amplify the sequences from the original polynucleotide encoding the peptide-grafted antibody may optionally comprise degenerate sequences in order to introduce additional diversity into the sequences flanking the peptide. Various degenerate sequences suitable for introducing diversity are known in the art. Examples of degenerate sequences that may be used include, but are not limited to, NNK, NNN, NNR, NNY and NNS, wherein N is any nucleotide, K is guanine or thymine, R is guanine or adenine, Y is thymine or cytosine, and S is guanine or cytosine.

Suitable RSS sequences for incorporation into the primers and V(D)J substrate polynucleotide are known in the art. The RSS sequences preferably consist of two conserved sequences (for example, heptamer, 5'-CACAGTG-3', and nonamer, 5'-ACAAAAACC-3'), separated by a spacer of either 12 +/- 1 bp (a "12-signal" RSS) or 23 +/- 1 bp (a "23-signal" RSS). Other functional RSS sequences are known in the art. Examples of such RSS sequences, including their characterization as high, medium or low efficiency RSSs, are presented in Table 2 and 3.

**Table 2. Exemplary Recombination Signal Sequences (12 nucleotide spacer)**

| | **Heptamer** | **Spacer** | **Nonamer** |
|---|---|---|---|
| | **H12** | **S12** | **N12** |
| **Part I. Efficiency: HIGH** | | | |
| **1** | CACAGTG | ATACAGACCTTA [SEQ ID NO:13] | ACAAAAACC |
| **2** | CACAGTG | CTACAGACTGGA [SEQ ID NO:14] | ACAAAAACC |
| **3** | CACAGTG | CTCCAGGGCTGA [SEQ ID NO:15] | ACAAAAACC |
| **4** | CACAGTG | CTACAGACTGGA [SEQ ID NO:14] | ACAAAAACC |
| **5** | CACAGTG | CTACAGACTGGA [SEQ ID NO:14] | ACAAAAACC |
| **6** | CACAGTG | CTACAGACTGGA [SEQ ID NO:14] | ACAAAAACC |
| **7** | CACAGTG | GTACAGACCAAT [SEQ ID NO:16] | ACA**G**AAACC |

| **Part II. Efficiency: MEDIUM (-10-20% of High)** | | | |
|---|---|---|---|
| **8** | CAC**G**GTG | CTACAGACTGGA [SEQ ID NO:14] | ACAAAAACC |
| **9** | CACA**A**TG | CTACAGACTGGA [SEQ ID NO:14] | ACAAAAACC |
| **10** | CACAG**C**G | CTACAGACTGGA [SEQ ID NO:14] | ACAAAAACC |
| **11** | CACAGTG | CTACAGACTGGA [SEQ ID NO:14] | ACAAAAACC |
| **12** | CACAGTG | CTACAGACTGGA [SEQ ID NO:14] | ACAAAAACC |
| **13** | CACAGTG | CTACAGACTGGA [SEQ ID NO:14] | ACAAAAACC |
| **14** | CACAGTG | CTACAGACTGGA [SEQ ID NO:14] | ACAAAAACC |
| **15** | CACAGTG | CTACAGACTGGA [SEQ ID NO:14] | ACAAAAACC |
| **16** | CACAGTG | CTACAGACTGGA [SEQ ID NO:14] | ACAAAAACC |
| **17** | CACAGTG | CTACAGACTGGA [SEQ ID NO:14] | **CA**AAAA**C**CC |
| **18** | CACAGTG | CTACAGACTGGA [SEQ ID NO:14] | ACAAAAACC |
| **19** | CACA**A**TG | CTACAGACTGGA [SEQ ID NO:14] | ACAAAAACC |
| **20** | CACAG**C**G | CTACAGACTGGA [SEQ ID NO:14] | ACAAAAACC |

| **Part III. Efficiency: LOW (∼1% or less of High)** | | | |
|---|---|---|---|
| **21** | **T**ACAGTG | CTACAGACTGGA [SEQ ID NO:14] | ACAAAAACC |
| **22** | **G**ACAGTG | CTACAGACTGGA [SEQ ID NO:14] | ACAAAAACC |
| **23** | CA**T**AGTG | CTACAGACTGGA [SEQ ID NO:14] | ACAAAAACC |
| **24** | CACA**A**TG | CTACAGACTGGA [SEQ ID NO:14] | ACAAAAACC |
| **25** | CACAGTG | CTACAGACTGGA [SEQ ID NO:14] | ACAAAAACC |
| **26** | CA**G**AGTG | CTCCAGGGCTGA [SEQ ID NO:15] | ACAAAAACC |
| **27** | CACAGTG | CTCCAGGGCTGA [SEQ ID NO:15] | A**A**AAAAACC |
| **28** | C**T**CAGTG | CTCCAGGGCTGA [SEQ ID NO:15] | ACAAAAACC |

**Table 3. Exemplary Recombination Signal Sequences (23 Nucleotide Spacer)**

| | **Heptamer** | **Spacer** | **Nonamer** | **Ref.*** |
|---|---|---|---|---|
| | **H23** | **S23** | **N23** | |
| **Part I. Efficiency: HIGH** | | | | |
| **1** | CACAGTG | GTAGTACTCCACTGTCTGGCTGT [SEQ ID NO:17] | ACAAAAACC | 4 |
| **2** | CACAGTG | GTAGTACTCCACTGTCTGGCTGT [SEQ ID NO:17] | ACAAAAACC | 3 |
| **3** | CACAGTG | GTAGTACTCCACTGTCTGGGTGT [SEQ ID NO:17] | ACAAAAACC | 1 |
| **4** | CACAGTG | TTGCAACCACATCCTGAGTGTGT [SEQ ID NO:18] | ACAAAAACC | 2 |
| **5** | CACAGTG | GTAGTACTCCACTGTCTGGCTGT [SEQ ID NO:17] | ACAAAAACC | 2 |
| **6** | CACAGTG | ACGGAGATAAAGGAGGAAGCAGG [SEQ ID NO:19] | ACAAAAACC | 2 |
| **7** | CACAGTG | GCCGGGCCCCGCGGCCCGGCGGC [SEQ ID NO:20] | ACAAAAACC | 5 |

| **Part II. Efficiency: MEDIUM (-10-20% of High)** | | | | |
|---|---|---|---|---|
| **8** | CACAGTG | GTAGTACTCCACTGTCTGGCTGT [SEQ ID NO:17] | ACAAAAACC | 3 |

| | **Heptamer** | **Spacer** | **Nonamer** | **Ref.*** |
|---|---|---|---|---|
| | **H23** | **S23** | **N23** | |
| **9** | CACAGTG | GTAGTACTCCACTGTCTGGCTGT [SEQ ID NO:17] | ACAAAAACC | 3 |
| **10** | CACAGTG | GTAGTACTCCACTGTCTGGCTGT [SEQ ID NO:17] | ACAAAAACC | 3 |
| **11** | CACA**A**TG | GTAGTACTCCACTGTCTGGCTGT [SEQ ID NO:17] | ACAAAAACC | 3 |
| **12** | CACAG**C**G | GTAGTACTCCACTGTCTGGCTGT [SEQ ID NO:17] | ACAAAAACC | 3 |
| **13** | CACAGT**A** | GTAGTACTCCACTGTCTGGCTGT [SEQ ID NO:17] | ACAAAAACC | 3 |
| **14** | CACAGTG | GTAGTACTCCACTGTCTGGCTGT [SEQ ID NO:17] | ACAA**T**AACC | 3 |
| **15** | CACAGTG | GTAGTACTCCACTGTCTGGCTGT [SEQ ID NO:17] | ACAA**G**AACC | 3 |
| **16** | CACAGTG | GTAGTACTCCACTGTCTGGCTGT [SEQ ID NO:17] | ACA**CG**AACC | 3 |
| **17** | CACAGTG | GTAGTACTCCACTGTCTGGCTGT [SEQ ID NO:17] | ACAAAAACC | 3 |
| **18** | CACAGTG | GTAGTACTCCACTGTCTGGCTGT [SEQ ID NO:17] | ACA**CG**AACC | 3 |
| **19** | CACA**A**TG | GTAGTACTCCACTGTCTGGCTGT [SEQ ID NO:17] | ACAAAAACC | 3 |
| **20** | CACAG**C**G | GTAGTACTCCACTGTCTGGCTGT [SEQ ID NO:17] | ACAAAAACC | 3 |
| **Part III. Efficiency: LOW (∼1% or less of High)** | | | | |
| **21** | CACAGT**A** | GTAGTACTCCACTGTCTGGCTGT [SEQ ID NO:17] | ACAAAAACC | 3 |
| **22** | CACAGTG | GTAGTACTCCACTGTCTGGCTGT [SEQ ID NO:17] | ACAAAAACC | 3 |
| **23** | CACA**A**TG | GTAGTACTCCACTGTCTGGCTGT [SEQ ID NO:17] | ACAAAAACC | 3 |

| | **Heptamer** | **Spacer** | **Nonamer** | **Ref.*** |
|---|---|---|---|---|
| | **H23** | **S23** | **N23** | |
| **24** | CA**T**AGTG | GTAGTACTCCACTGTCTGGCTGT [SEQ ID NO:17] | ACAAAAACC | 3 |
| **25** | CACAGTG | GTAGTACTCCACTGTCTGGCTGT [SEQ ID NO:17] | **TGTCTCTGA** | 3 |
| **26** | CACAGTG | GTAGTACTCCACTGTCTGGGTGT [SEQ ID NO:17] | ACAAAAACC | 1 |
| **27** | CACAGTG | GTAGTACTCCACTGTCTGGGTGT [SEQ ID NO:17] | ACAAAAACC | 1 |
| **28** | CACAGTG | GTAGTACTCCACTGTCTGGGTGT [SEQ ID NO:17] | ACAAAAACC | 1 |

| | | | | |
|---|---|---|---|---|
| *(1) Akamatsu, 1994, *ibid*; (2) Cowell, 2004, *ibid*; (3) Hesse,1989 *ibid*; (4) Lee, 2003, *ibid*; (5) Nadel,1998, *ibid.* | | | | |

The V(D)J substrate polynucleotide into which the amplified sequences are ligated may further comprise one or more regulatory sequences allowing for expression of the peptide-grafted antibody once recombination has taken place, such as promoters, enhancers, terminators, alpha-factors, ribosome binding sites, polyadenylation signals and the like. Preferably, the V(D)J substrate polynucleotide further comprises additional coding sequences that encode a plasma membrane anchor domain such that the expressed peptide-grafted antibody is expressed on the surface of the reporter cell line, although secreted peptide-grafted antibodies are also contemplated. The V(D)J substrate polynucleotide may already be part of an appropriate vector for transfection into the reporter cell line, or the V(D)J substrate polynucleotide comprising the amplified sequences may subsequently be cloned into an appropriate vector for transfection into the reporter cell line.

When the library of peptide-grafted antibodies is not subjected to an additional V(D)J recombination step, the coding sequence for the peptide-grafted antibody may be ligated to additional coding sequences that encode a plasma membrane anchor domain such that the peptide-grafted antibody is expressed on the surface of the host cell, for example, by cloning the coding sequence for the peptide-grafted antibody into a vector that provides the additional plasma membrane anchor domain coding sequences.

The vector used to transfect the reporter cell line may optionally include coding sequences for the antibody light chain under appropriate expression control sequences, such that an intact antibody is expressed from the vector in the reporter cell line. Alternatively, the reporter cell line may subsequently be transfected with a vector comprising coding sequences for the light chain under appropriate expression control sequences.

The reporter cell line used in the methods described herein comprises a first stably integrated recombinant nucleic acid construct comprising a reporter gene encoding a cell surface expressed protein that is under the control of a cAMP responsive promoter such that expression of the protein takes place only in the presence of cAMP. A cAMP responsive promoter is one that includes at least one cAMP response element (CRE) and, optionally, other upstream activators. A variety of cAMP responsive promoters are known in the art. For example, cAMP response element binding protein (CREB) has been shown to occupy over 4000 sites in the human genome (Zhang et al., 2005, PNAS, 102(12):4459-4464). The cAMP responsive promoter may be a naturally occurring cAMP responsive promoter, or it may be a promoter sequence genetically engineered to include at least one CRE and, where necessary, other upstream activators. The CRE may be a full-length CRE (for example: TGACGTCA) or it may be a partial CRE (for example, TGACG or CGTCA).

The cell line also includes a second stably integrated recombinant nucleic acid construct that comprises a sequence encoding the target Class B GPCR under control of an appropriate promoter. The promoter may be a constitutive promoter or it may be an inducible promoter. A recombinant nucleic acid construct that comprises a sequence encoding the target Class B GPCR under control of a constitutive promoter may be used. The cell line is transfected with the vectors comprising the sequences encoding the peptide-grafted antibodies, cultured under conditions permitting expression of the peptide-grafted antibodies and expression of the Class B GPCR, and subsequently screened for surface expression of the protein encoded by the reporter gene. Cells expressing reporter gene comprise peptide-grafted antibodies having GPCR agonist activity. These cells can subsequently be isolated and the agonist peptide-grafted antibodies can optionally be further characterized, for example by sequencing.

The sequences encoding the peptide-grafted antibody agonist may be isolated for further downstream uses including, for example, sub-cloning into alternate expression vectors, further testing prior to therapeutic use and/or large scale production of the peptide-grafted antibody agonist.

The cell surface expressed protein encoded by the reporter gene included in the cell line may be one of a number of known surface expressed proteins or peptides for which an adequate detection method is available or can be readily designed. Examples include, but are not limited to, CD4, CD8, CD3, CD19, V5, FLAG or any epitope that is expressed on the surface of the cell and can be detected by a reagent. The cell surface expressed protein encoded by the reporter gene may be CD19.

Various detection methods appropriate for high-throughput assays are known in the art. Typically, these are flow cytometry based and involve the use of antibodies or ligands conjugated to detectable labels. It may be desirable to use detectable labels that also permit enrichment of positive cells, for example, via magnetic bead enrichment.

The reporter cell line may also be recombination competent such that the additional V(D)J recombination step described above for introducing additional diversity into one or both of the peptide flanking sequences may be conducted in the same cell line as the functional assay. In this context, the reporter cell line is capable of mediating RAG-1/RAG-2 recombination either via stably integrated nucleic acid constructs that express RAG-1 and RAG-2, or functional fragments thereof, or via transfection of the cell line with appropriate genetic constructs encoding RAG-1 and RAG-2, or functional fragments thereof. The expression of one or both of RAG-1 and RAG-2 in the recombination competent reporter cell line may be constitutive or it may be inducible. The recombination competent reporter cell line may optionally further express TdT, or a functional fragment thereof.

In the use of a recombination competent reporter cell line, the reporter cell line may further comprise sites engineered into the chromosome that permit stable chromosomal integration of the V(D)J recombination substrate polynucleotide, for example, CRE/Lox recombination sites, FLP/FRT recombination sites, attP/attB recombination sites or combinations thereof. Approaches such as froxing and floxing have also been reported and may be appropriate. Accordingly, the method may comprise amplifying sequences encoding the grafted peptide and one or both flanking regions from a library of peptide-grafted antibodies capable of binding the target Class B GPCR with primers that introduce the required RSS sequence or sequences for RAG-mediated recombination. The primers may optionally include degenerate sequences for further diversification. The amplified sequences are subsequently ligated into a V(D)J substrate polynucleotide that comprises sequences encoding one or a plurality of VH segments and/or one or a plurality of JH segments, or sequences encoding one or a plurality of VL segments and/or one or a plurality of JL segments, together with the required RSS sequences. The V(D)J substrate polynucleotide preferably further comprises additional coding sequences that encode a plasma anchor domain. The V(D)J substrate polynucleotide comprising the diversified peptide sequence is transfected into the reporter cell line under conditions that permit integration of the substrate into the corresponding site(s) engineered into the chromosome. The reporter cell is cultured under conditions that allow RAG-mediated recombination and expression of the resulting peptide-grafted antibodies. If a plasma membrane anchor domain was used, the antibodies are expressed on the cell surface. The cells can then be screened for expression of the reporter gene.

Once a peptide-grafted antibody agonist has been identified using the methods described above, the method may further comprise generating improved peptide-grafted antibody agonists from the initial antibody, for example, by using the V(D)J protein optimization techniques as described in International Patent Application PCT/CA2013/050203 (WO 2013/134880). For example, V(D)J protein optimization may be used to target specific amino acids in the CDR into which the peptide is grafted such that a library of polynucleotides encoding variant peptide-grafted antibodies is generated that differ in the sequences of this CDR. The library of polynucleotides encoding variant peptide-grafted antibodies may then be screened functionally using the reporter cell line to identify those peptide-grafted antibodies having increased agonist activity against the target GPCR.

### KITS

Disclosed are kits comprising the reporter cell line disclosed herein for use in high-throughput methods of identifying agonists of Class B GPCRs, including peptide-grafted antibody agonists. The kits may optionally comprise reagents for detecting the reporter protein and, for peptide-grafted antibody agonists, may further optionally comprise a V(D)J substrate polynucleotide, as described above.

Optionally, for peptide-grafted antibody agonists, the kit may additionally comprise vectors encoding one or more of RAG-1, RAG-2 and TdT that are suitable for transfecting the reporter cell line such that the cell line expresses, or is capable of expressing, RAG-1, RAG-2 and/or TdT. Alternatively, when the cell line is recombination competent, the kit may optionally comprise a vector encoding TdT.

The kit may further comprise one or more additional components to assist with cloning and/or transfection and/or reporter detection, such as buffers, enzymes, selection reagents, growth media and the like.

One or more of the components of the kit may optionally be lyophilised and the kit may further comprise reagents suitable for the reconstitution of the lyophilised components. Individual components of the kit would be packaged in separate containers and, associated with such containers, can be instructions for use. The instructions for use may be provided in paper form or in computer-readable form, such as a CD, DVD or the like.

To gain a better understanding of the invention described herein, the following examples are set forth. It will be understood that these examples are intended to describe illustrative embodiments of the invention and are not intended to limit the scope of the invention in any way. Examples not falling within the scope of the claims are for illustrative purposes only.

### EXAMPLES

Utilizing *in vitro* V(D)J mediated peptide-grafting methods as described in International Patent Application No. PCT/CA2013/050204 (WO 2013/134881), several hundred GLP1 receptor binders were generated. Forty-eight of these peptide-grafted antibodies were tested for agonist activity, but none was detected. Although conformational contexts were identified by these methods that were appropriate for binding to the receptor, the conformations were not appropriate for activating the receptor. As GLP1 peptide-Fc fusion proteins are active and a free amino terminus has been hypothesized by others to be essential for activity of the peptide, an approach of generating additional diversity at the amino terminal antibody-peptide junction in the isolated peptide-grafted antibodies that bound to the GLP1 receptor was adopted in an effort to find a conformational solution that would not require a free amino terminus. Briefly, the pool of GLP1 receptor binders was cloned into a second V(D)J diversification substrate to specifically target additional diversity to the amino terminal junction of the antibody scaffold and grafted peptide. V(D)J optimization vectors as described in International Patent Application No. PCT/CA2013/050203 (WO 2013/134880) were employed to target mutations and a library of approximately 100 million variants was generated.

To screen this many variants using traditional methods would not have been practical. In addition to creating an agonist peptide in a final antibody scaffold format and thus avoiding the requirement to reformat the antibody from phage format, generating diversity *de novo* in a mammalian cell also allows the mammalian cell line to be engineered to incorporate a functional reporter for cAMP activation. The ability to incorporate a cAMP functional reporter directly into the mammalian cell line capable of *de novo* diversity generation was an important factor in developing the assay described below that allowed a rare agonist to be identified from amongst a large pool of a receptor binders.

Although a number of different cAMP reporter assays have been previously described, those using reporter gene expression generally utilize luciferase. Luciferase-based reporter systems are enzymatic and give very high sensitivity. These systems often use destabilized enzyme to improve signal to noise ratios. In order to identify rare binders in the context described below, however, a reporter system that resulted in expression of the reporter on the cell surface was preferred. This would allow, for example, for magnetic bead enrichments. Whether or not a cell surface reporter system linked to cAMP levels would be possible was not clear prior to developing the assay described below. For example, it was not known whether expression driven from a minimal promoter containing CREs would be sufficient to generate levels useful for FACS or magnetic sorting. It was also unknown if constitutive reporter gene expression would result in down-regulation of the receptor over time.

In addition, the peptide-grafted libraries were constructed to generate surface expressed IgG so that peptide-grafted antibodies would not be secreted at high levels and thus activation of receptors on neighboring cells would be minimal. It was unclear prior to developing the reporter assay whether this tethered antibody would still be able to activate the receptor. On the other hand, an advantage of a membrane tethered antibody would be that the majority of the library would be expressing *antagonistic* peptide-grafted antibodies *(i.e.* peptide-grafted antibodies that bind to, but do not activate, the receptor) and the secretion of these antagonists in large quantities by the library would have potentially prevented the activation of the receptor by a rare agonist.

An overview of the development of the reporter cell line is shown in Figure 16.

The cyclic AMP reporter assay as described in Example 1 below was developed such that activation of the receptor resulted in the expression of the cell surface protein CD19. The reporter was engineered with cAMP responsive elements (CREs) upstream of the CD19 open reading frame. In order to find the most suitable chromosomal location with the appropriate number and ratio of integrated reporter cassettes and GLP1 receptor expression cassettes, the strategy described in Example 1 was employed to empirically identify a useful reporter cell line.

A flow chart outlining the steps described in Examples 1-5 below is provided in Figure 15.

### EXAMPLE 1: Generation of Reporter Cell Line

HuTARG™ cells, which are HEK293 cells engineered to be capable of *de novo* antibody generation, were used to generate the reporter cell line. HuTARG™ cells have been stably transfected with expression cassettes for RAG1 and RAG-2 and Tdt. RAG-1 expression is inducible as the gene has been placed downstream of a Tet inducible promoter. HuTARG™ cells have also been stably transfected with a plasmid containing a LoxP site for subsequent insertion of V(D)J substrates.

HuTARG™ cells were modified to express the GLP1 receptor as well as express a reporter gene that was activated by cAMP. Approximately 20 million HuTARG™ cells without an integrated V(D)J substrate were transfected with a vector containing CMV driven GLP1R (V525, Origene, SEQ ID NO:21; Figure 17) and a second vector with a Neomycin resistant marker and a reporter gene (CD19) whose expression is controlled by a cAMP responsive promoter and a (E518; SEQ ID NO:22; Figure 18). cAMP promoter elements were derived from pGL4.29 (Promega). Twenty micrograms of DNA (50:50 mix) and sixty micrograms of PEI was used for the transfection. Neomycin selection was used to generate a pool of cells stably expressing different ratios of each vector.

Next, to remove stable integrants expressing constitutive CD19, the cell line pool was depleted of CD19 positive cells through two round of bead selection. Briefly, two million stable cells were trypsinized, centrifuged and incubated with an anti-CD19 antibody for one hour. Next, cells were centrifuged, washed and then stained with a biotinylated goat anti-human IgG antibody for an additional hour. The cells were then washed again, resuspended into 500ul of PBS + 2%FBS, incubated with 20ul of antibiotin microbeads (Miltenyi Biotech, cat#120-000-900) and then depleted as per manufacturer's suggested protocol using Miltenyi LS magnetic columns.

Depleted cells were subsequently expanded and depleted an additional time using the same protocol except using a Miltenyi LD magnetic column. Next, the twice-depleted cells were treated with 10nM Exendin (Anaspec) for 24 hours and then enriched for CD19 positive cells using the same staining procedure as above but instead utilizing Miltenyi MS column followed by a MS column and manufacturer's suggested protocol for positive selection. The magnetically isolated cells were expanded and maintained without ligand and subsequently FACS sorted for CD19 negative/GLP1R positive cells. Single cell clones confirmed to have minimal CD19 expression in the absence of ligand were evaluated for induction of CD19 in the presence of 10nM Exendin and the L696 subclone was selected.

Figure 9 shows the FACS plots for the L696 cell line in the absence or presence of 10nM Exendin demonstrating that CD19 expression is specifically induced by the addition of ligand for the GLP1 receptor. Additional experiments were performed with the L696 cell line confirming that cells constitutively expressing a membrane tethered Exendin-Fc fusion protein resulted in CD19 surface expression, thus confirming that a membrane IgG was an appropriate format for screening and that down-regulation of the receptor as a result of constitutive stimulation would not negatively impact the assay. This clone was named HuTARG-696 and was subsequently used to generate *de novo* peptide-grafted libraries as described in the following Examples.

### EXAMPLE 2: Cloning of CDR Diversification Vectors of GLP1-Receptor Binding Peptide-Grafted Antibodies

The cDNA from the Exendin peptide-grafted antibodies (greater than 500) previously generated as described in International Patent Application No. PCT/CA2013/050204 (WO 2013/134881) was amplified using the following primers.
CP360: GAGAGTTTGGATCCCAACTTTCTTGTCCACCTTGGTGTTGC [SEQ ID NO:27]

CP360 is the primer used in the reverse-transcription reaction to generate cDNA.
AL63: GAGAGATTTGGTCTCTATGTCGATCTGATCAAGAGACAGGATAAGGAGCC [SEQ ID NO:28]
MG302: GAGAGAGATTGGTCTCGCCTGAGTTCCACGACACCGTCACC [SEQ ID NO:29]

Primer AL63 anneals in the 5' untranslated region upstream the heavy chain variable region leader sequence found in all the VH gene segments utilized to generate fully human antibodies in International Patent Application No. PCT/CA2013/050204 (WO 2013/134881). All the antibodies generated *de novo* using the *in vitro* V(D)J system utilize the same promoter, 5'UTR and leader sequence, so that the primer is universal for all heavy chain variable genes. The MG302 primer anneals in CH1 of the heavy chain and is the reverse primer to amplify the heavy chain cDNA corresponding to the Exendin-peptide-grafted antibodies. The PCR product was generated using the KOD polymerase and was subsequently cloned into vector E425 (see Figure 1) using the flanking BsaI sites of each primer. E425 contains the appropriate corresponding BsaI sites downstream of a CMV promoter to drive expression of the cDNA and in the heavy chain CH1 to reconstitute the human Ig constant region (Figure 1). International Patent Application No. PCT/CA2013/050204 (WO 2013/134881) demonstrated that peptide-grafted antibodies that bound to the GLP1 receptor could be generated. Evaluation of individual clones isolated from the *de novo* Exendin peptide-grafted antibodies for GLP1 receptor agonist activity were, however, negative. The pool of approximately 5000 clones representing greater than 100 binders was subsequently used as the template for PCR using primers:
ET70:
ET71:

The use of the ET70 and ET71 primers generated a PCR product that extended 3' from the histidine codon at the start of the sequence encoding Exendin to the SV40 PolyA (see Figure 2). The ET70 primer includes a BsaI cloning site and three degenerate nucleotides upstream of the Exendin open reading frame and provides additional substrate diversity; it is equivalent to increasing the number of D segments available for the recombination reaction. The 3' end of Exendin was represented by all the GLP1 receptor binding clones that were isolated; these 3' junctions were previously shown to support binding to the receptor. The ET71 primer has a KpnI tail for cloning. The pool of Exendin-based sequences with degenerate 5' ends and 3' ends derived from receptor binding peptide-grafted antibodies was cloned into vector V610 (see Figure 5) as a pool of BsaI-KpnI fragments to generate a pool referred to as ITS007-P31 and which represented approximately 148,000 different subclones.

The ITS007-P31 pool was digested with BsmBI and XhoI and co-ligated with the BsmBI and BamHI fragment from P163 (see Figure 4) into the BamHI-XhoI sites of vector E501 (see Figure 6), which is a V(D)J substrate that also expresses a VK1-39-based light chain. The E501 vector additionally contains AAV insulators between the V(D)J recombination substrate and the light chain expression cassette. E501 also contains a LoxP site in-frame with a hygromycin resistance gene for selection of Cre-mediated integration. In the resulting pool of V(D)J substrates, each contained a 5' NNK sequence in the context of 3' flanking sequences isolated from GLP1 receptor binders. The pool was referred to as ITS007-P33.

The ITS007-P33 pool was Cre-integrated into the HuTARG-696 reporter cell line to a chromosomal site previously shown to support Cre recombination, V(D)J recombination and high expression. The integrated LoxP site contains an upstream CMV promoter and ATG/Kozak required for expression of the hygromycin resistance marker in ITS007-P33; this measure was introduced to minimize random integration of ITS007-P33. Two 10-cm plates of the HuTARG-L696 reporter cell line were seeded 24 hour prior to transfection in 12 mls of DMEM. 17.3ug of the ITS007-P33 DNA and 1.9ug of the V503 DNA was used per transfection and was added to 2.4ml of PRO293 serum free media. 2.4ml of PRO293 containing 57.6ul of 1mg/ml PEI was added to the DNA, vortexed and allowed to complex for 20 minutes prior to being added to the cells. Forty-eight hours post-transfection, Cre-mediated integrants were selected by the addition of Hygromycin B (100ug/ml). Hygromycin selection was allowed to proceed for 10 days. The Cre-integrated pool of V(D)J substrates was named HuTARG ITS007-L31.

### EXAMPLE 3: Induction of V(D)J Recombination

A 10-cm plate of the HuTARG ITS007-L31 cells was seeded for V(D)J induction (approximately 24 million cells). Induction was essentially as described in International Patent Application No. PCT/CA2013/050204 (WO 2013/134881) with the following modifications. Vector V429 expressing TdT was transfected into the cells and 24 hours following transfection, the 10-cm plate was split into a 175cm² flask at which time RAG-1 was induced by adding 1ug/ml Tetracycline. Tet-inducible RAG-1 was generated in the HuTARG cell line using the Tet-On system from Invitrogen (Carlsbad, CA). V(D)J induction was allowed to proceed for 5 days at which point the flask was trypsinized and seeded into a suspension shake flask at approximately 1.5 million cells/ml resulting in a total induced cell population of approximately 150 million cells. Puromycin (1ug/ml) was added to the shake culture (to select for in-frame peptide-grafted antibody sequences) and the cells were cultured for 2 days. Media was removed and replaced, and the culture was re-seeded at 1.5 million cells per ml and puromycin selection continued for another 3 days. Cells were subsequently harvested and plated back to adherent flasks where they were maintained under selection until used.

### EXAMPLE 4: Screening For Agonists

The puromycin-selected HuTARG ITS007-L31 cells from Example 3 were confirmed to have surface IgG. The HuTARG ITS007-L31 library was then analyzed for the presence of surface-expressed CD19, which would indicate that the GLP1R was activated.

Approximately 1 million recombined HuTARG ITS007-L31 cells that surface-expressed anti-GLPIR peptide-graft antibodies were stained with anti-CD 19-PE conjugated antibody (1ug/ml) and an anti-IgG Fc Alexa-647 conjugated antibody (1ug/ml). The results of FACS analysis of these cells is shown in Figure 10 and demonstrates that a large fraction of the library generated peptide-grafted antibodies that bind to the GLP1 receptor (X-axis), but only a small fraction of these cells were CD19 positive (Y-axis) and represented potential agonists. The CD19 positive/IgG positive population was FACS sorted and the cDNA was isolated from 1000 positive cells. The isolated cDNA was cloned into a expression vector to express the isolated heavy chain.

The expression vectors comprising the sequences for the isolated heavy chain antibodies were co-transfected into the HuTARG-L696 reporter cell line with a second vector expressing the VK1-39 kappa light chain. 48 hours post-transfection, cells were stained for surface CD19 expression. A number of positive clones were identified. On sequencing, it was determined that these positive clones represented just two different antibodies with agonist activity (Table 4; bold sequences represent the grafted exendin peptide, italicized sequences represent sequences from the VH or JH gene segment).

**Table 4: Sequences of Isolated Agonist Peptide-Grafted Antibodies**

| ID | IGHV | Sequences proximal to exendin (in bold) | SEQ ID NO |
|---|---|---|---|
| ITS007 -V105 | 2-70 | | 32 |
| ITS007 -V107 | 3-9 | | 33 |

The two peptide-grafted antibodies utilize different VH gene segments (VH2-70 or VH3-9) and also differed at both the VH-5' Exendin junction and the 3' Exendin-JH junction in both the length and composition of amino acids. The two sequences interestingly, however, both contained an arginine flanking the histidine at the N-terminus of the Exendin sequence. Both sequences utilized the JH3 gene segment. The complete nucleotide and amino acid sequences for ITS007-V105 and ITS007-V107 are provided in Figure 14 (SEQ ID NOs:23-26).

Preliminary mutagenesis studies in which the amino acid at the -1 position relative to the His in Exendin (*i.e*. Arg in the two sequences shown in Table 4) was mutated to other amino acids were conducted. Appreciable agonist activity in the above-described reporter cell line assay was seen only with Arg or Lys at this position.

### EXAMPLE 5: Activity of Agonists in Absence of Light Chain

In control experiments, agonist activity from the two antibodies (ITS007-V105 and ITS007-V107) was also observed in the absence of light chain. Figure 11 shows cells transfected with a human heavy chain (upper left), an intact human antibody with heavy chain and light chain (lower left), ITS007-V107 peptide-grafted heavy chain (upper right) or an intact peptide-grafted antibody comprising ITS007-V107 peptide-grafted heavy chain and a human light chain (lower right). The cells were co-stained for human kappa and for surface-expressed CD19.

The left-hand panels demonstrate that the heavy chain only transfection is negative for human kappa chain (upper panel) while the intact antibody in the lower left panel has cells with human kappa on the surface as an intact antibody. Both the left-hand panels are CD19 negative and reflect that the control antibody does not induce cAMP in the reporter cell line.

The right-hand panels show that only the lower panel has surface kappa expression but that both the right-hand panels, *i.e.* with and without the presence of a light chain, show CD19 staining, indicating that both the intact peptide-grafted antibody and the peptide-grafted heavy chain alone are capable of stimulating the GLP1 receptor.

The peptide-grafted antibody, ITS007-V107, was subsequently cloned and expressed as a single-domain VH-IgG antibody without CH1 or a light chain and it was confirmed that the peptide-grafted heavy chain was functional in this format as well (Figure 12). This peptide-grafted heavy chain, named ITS007-V125 (see Figure 3, SEQ ID NOs:50 and 51), was transfected into HEK293 cells and supernatant was collected 5 days post transfection. Expression levels were greater than 5ug/ml. The supernatant was then assayed on the HuTARG-L696 reporter line. Figure 12 demonstrates that the reporter cell line alone does not have significant CD19 surface expression (upper left panel). The reporter line incubated with 50nM Exendin for 48 hours resulted in approximately 8% of the cells showing various levels of CD19 expression (upper right panel). Incubation of the ITS007-V125 supernatant with the reporter cell line for 48 hours at a final concentration of 1ug/ml resulted in approximately 5% CD19 positive cells (lower left panel). In the same experiment, the cells were stained for anti-IgG demonstrating that the ITS007-V125 single-domain antibody was binding to the GLP1R on the reporter cell line.

A control single-domain VH-IgG antibody, ITS007-V129, was also generated. The control antibody is identical to ITS007-V125 except for a histidine to alanine mutation corresponding to the first amino-acid in the Exendin peptide. This mutant antibody demonstrated similar binding to the reporter cell line but minimal agonist activity (lower right panel of Figure 12). This result is consistent with mutagenesis studies of Exendin and GLP1 that indicate the central role of the amino terminal histidine in agonist activity.

ITS007-V125 was cloned into a secretion vector, expressed and purified. The reporter cell line was treated with different concentrations of ITS007-V125 followed by overnight incubation. The next day, binding and the percentage of cells expressing the surface marker were determined by FACS. The results are shown in Figure 13 and confirm that the peptide-grafted heavy-chain ITS007-V125 has GLP1R agonist activity.

### EXAMPLE 6: Generation of Peptide-Grafted Glucagon Receptor Agonists in a Heavy Chain Only (HCAb) Format

Experiments with the GLP1 agonists generated as described above indicated that the agonist activity did not require the light chain and the peptide-grafted heavy chain was sufficient for all activity consistent with the peptide sequences being the primary element responsible for function. Accordingly, peptide-grafted glucagon receptor agonists were generated in a heavy chain only (HCAb) format (*i.e*.. including VH + CH2 and CH3, but not CHI).

A single chain format offers a number of advantages with regards to the generation of potential therapeutics. For example, because it is a single chain it provides the opportunity to generate a more modular system of combining different specificities. A single chain also may provide more convenient manufacturing opportunities. Not all heavy chains are expressed well in the absence of the light chain in an *in vitro* mammalian system. A unique set of VH gene segments, different from what has previously be reported, was identified that performs well as a single chain in a mammalian cell. Specifically, the following VH gene segments were identified as expressing well in the HCAb format: VH1-8, VH3-11, VH3-15, VH3-20, VH3-30, VH4-34.

The repertoires for grafting the glucagon peptides were generated using the above-noted restricted VH gene set. Four different sets of V(D)J diversification substrates were generated as shown below. Two utilized the glucacon peptide sequence (SEQ ID NO:10) and two used the oxyntomodulin (OXN) sequence (SEQ ID NO:34). One set of diversification substrates had three nucleotides (TCC, corresponding to a serine) flanking the 5' RSS and three nucleotides (GTG corresponding to valine) flanking the 3' RSS. The second set included 9 additional nucleotides of 3 sets of NNK nucleotides on each side of the peptide sequences.
ITS011-P11 :
   12 bp-RSS TCC NNKNNKNNK Glucagon peptide seq NNKNNKNNK GTG 12 bp-RSS
ITS011-P12:
   12 bp-RSS TCC Glucagon peptide seq GTG 12 bp-RSS
ITS011-P13:
   12 bp-RSS TCC NNKNNKNNK OXN peptide seq NNKNNKNNK GTG 12 bp-RSS
ITS011-P14:
   12 bp-RSS TCC OXN peptide seq GTG 12 bp-RSS

The nucleotide sequence encoding the glucagon peptide employed was:

The nucleotide sequence encoding the oxyntomodulin peptide employed was:

The peptide-grafting V(D)J tripartite substrates were CRE integrated into a host HEK293 cells that contained a single chromosomal LoxP site. The site had been selected to be competent for V(D)J recombination. The HEK293 line also was engineered for assaying for cAMP induced reporter function similar to the GLP1 reporter cell line in the preceding Examples except the glucagon receptor was used instead of the GLP1 receptor. The activation of the receptor by the peptide ligand was shown to activate expression of a cell surface marker (see Figure 21; Glc-Fc control and OXN-Fc control).

The substrate was engineered to include two FLAG tag sequences in-frame with the LoxP site and hygromycin allowing for magnetic enrichment and/or hygromycin selection for CRE integration events. CRE integration was performed by transfecting the V(D)J substrate and a plasmid expressing the CRE recombinase (V503 - see Figure 7) at a ratio of 10:1. Transfected cells were expanded to approximately 200 million cells and then magnetically enriched using anti-FLAG antibodies and magnetic columns from Miltenyi. CRE pools of approximately 10-50,000 clones were generated and used for subsequent experiments.

V(D)J induction was performed as described for GLP1 in the Examples above. Cells expressing agonist peptide-grafted antibodies were identified by the expression of the cAMP responsive cell surface marker. Cells were magnetically enriched and then subsequently FACS sorted. cDNA was isolated from the FACS sorted cells and individual clones were assessed for activity as described below.

*PEI Max Transfections*: One day prior to transfections, ITS011-L05.13 cells were trypsinized and seeded 1:3 into a 24-well plate. Next day, cells were transfected as shown in Table 5.

**Table 5:**

| **Conditions** | **Plasmid** | | **Stock [DNA] ug/ul** | **ug DNA** | **ul DNA** | **# Wells per Sample** |
|---|---|---|---|---|---|---|
| **1** | pUC | Empty Plasmid | 0.500 | 0.800 | 1.6 | 1 |
| **2** | ITS011-V04 | Glc-Fc Secreted | 0.317 | 0.800 | 2.5 | 1 |
| **3** | ITS011-V05 | OXM-Fc Secreted | 0.306 | 0.800 | 2.6 | 1 |
| **4** | ITS011-P26 | ITS011-L31 cDNA Pool | 0.464 | 0.800 | 1.7 | 1 |
| **5 to 18** | ITS011-V96 to -V109 | Isolated clones from ITS011-P26 | 0.100 | 0.800 | 8.0 | 1 |

The above indicated amount of DNA for each transfection condition was each added to 50ul of sf Pro293 media and incubated at room temperature for 5 minutes. 64ul of 1mg/mL PEI Max was added to sf Pro293 in 1mL final volume and incubated for 5 minutes at room temperature. 50ul of PEI mix was added to each plasmid prep and incubated at room temperature for 20 minutes. Each 100ul transfection mix was added drop-wise to a well. Cells were returned to culture at 37 °C and were stained the next day.

*CD19-IgG Staining*: The day after transfection, cells were stained as follows. Media was removed from each well and each well was gently rinsed with PBS. PBS was aspirated off and each well was trypsinized with 75ul of IX trypsin and cells briefly incubated at 37 °C. Each well was collected with 500ul of complete DMEM and spun down to remove supernatant. Each pellet was resuspended with 100ul of Mouse αCD19-PE (1:200) and Goat αHumanFc647 (1:5000) in FACS Buffer, and samples were incubated for 1 hour at room temperature then each spun down to remove supernatant. Each sample was washed once with 300ul of PBS and wash was removed via spinning down, then each sample was resuspended with 250ul of PBS and analyzed via the Accuri (Beckton Dickinson, New Jersey).

### Results

Isolated cDNA was tested for agonist activity. Background agonist activity represented by expression of the cell surface reporter (CD19) activity was approximately 0.1% across experiments. Agonists displaying different amounts of activity were isolated (see Figure 21).

The sequences of representative agonist clones are shown in Table 6 and Figure 30. Strikingly, the clones with agonist activity had either the amino acid arginine or lysine following the natural amino terminal histidine in the glucagon and oxyntomodulin peptide sequences, as was observed for the GLP1 agonists in the preceding Examples. In contrast, the carboxy terminus junction of the peptides and JH segments did not show any obvious sequence conservation.

**Table 6: Sequences of Representative Glucagon Agonist Clones**

| **ID** | **VH Gene** | **Sequence*** | **JH Gene** | **SEQ ID NO** |
|---|---|---|---|---|
| **Glucagon** | | | | |
| ITS011 -V36 | 3-15 | | 3 | 37 |
| ITS011 -V66 | 3-15 | | 6 | 38 |
| ITS011 -V69 | 3-20 | | 1 | 39 |

| **Oxyntomodulin** | | | | |
|---|---|---|---|---|
| ITS011 -V23 | 3-30 | | 6 | 40 |
| ITS011 -V74 | 3-20 | | 2 | 41 |
| ITS011 -V100 | 3-30 | | 1 | 42 |
| ITS011 -V165 | 4-34 | | 4 | 113 |
| ITS011 -V171 | 3-30 | | 4 or 5 | 114 |

| | | | | |
|---|---|---|---|---|
| * Peptide sequence in bold; charged amino acid flanking terminal Histidine of peptide sequence in underlined bold italics; carboxy flanking sequences found between peptide and JH gene segment in italics. | | | | |

### Discussion

Many in the field believed that it would not be possible to generate a functional peptide-grafted antibody to either the GLP1 receptor or the glucagon receptor because of the perceived requirement for a free amino terminal amino acid. The use of the V(D)J system to generate large de novo libraries as well as the ability to couple those large repertoires with a single cell functional read out has allowed rare functional antibodies to be identified. Unexpectedly, these rare solutions for the two receptors using three different peptides all showed a requirement for a positively charged amino acid adjacent to the natural N-terminal amino acid. The presence of the arginine or lysine appears to substitute for the free amino group in the natural peptide. The presence of the charged amino acid alone, however, may not be sufficient to provide a functional agonist as other peptide-grafted antibodies having an arginine in the -1 position were identified that did not show agonist activity in the assays used in these experiments. The presence of the arginine or lysine does seem to be required as no agonist antibodies have been identified to date that did not have either a lysine or arginine in this position.

It is worth noting that both glucagon and GLP1 are generated by cleavage of a proprotein that is inactive until it is processed and that the natural amino acid in the proprotein upstream of the N-terminal histidine is an arginine.

### EXAMPLE 7: Mutagenesis of N-terminal Amino Acid Adjacent to Histidine in the GLP-1 HCAb Agonist V125

As described in Example 5, the original fully human peptide-grafted antibody ITS007-V107 was converted to a heavy chain only antibody HCAb format to generate an agonist HCAb, ITS007-V125 (Table 7 below, Figure 3). The VH domains of the two agonists V107 and V125 are identical and both molecules are functional as agonists of the GLP-1 receptor. Mutagenesis of the amino acid upstream of natural N-terminal histidine of the Exendin sequence in the V125 peptide-grafted agonist HCAb was conducted. Specifically, variants of the V125 peptide-grafted HCAb were generated so that the arginine flanking the histidine was mutated to every amino acid. While some mutations were found to be detrimental to expression, the only mutant to retain GLP-1 receptor agonist activity was when the arginine was mutated to a lysine.

Once it had been determined that the agonist antibodies all contained a positive charge adjacent to the N-terminal histidine, additional site-specific mutants were generated using PCR to introduce additional positive charges at other positions between the VH and peptide (ITS007-V126, V127 and V128; see Table 7 and Figure 20). An additional mutant ITS007-V130 (see Table 7 and Figure 20) was also generated using PCR. In V130, the entire junction between the VH and the peptide includes basic amino acids. All of these modified peptide-grafted antibodies were shown to have agonist activity against the GLP-1 receptor.

An additional HCAb agonist with increased potency was also identified that had an insertion of a single amino-acid in the amino terminal VH-peptide junction, ITS007-V212 (see Table 7, and Figure 20).

The observation that a flanking lysine also could support agonist activity of a grafted peptide antibody to a type 1 GPCR was corroborated with the subsequent isolation of glucagon HCAb agonists generated *de novo* from V(D)J peptide grafted substrates that contained a lysine flanking the histidine of the grafted glucagon peptide and oxyntomodulin peptide (ITS011-V066 and ITS011-V074, see Table 6 above).

It should be appreciated that the presence of a lysine or arginine N-terminally to the histidine of peptides to either the GLP-1 receptor or Glucagon receptor is not sufficient to impart agonist activity. In addition to non-agonist peptide-grafted HCAbs being identified with either a lysine or arginine in this position, when the Exendin peptide in another HCAb GLP1 agonist, ITS007-V126 (see Table 7, Figure 20; SEQ ID NOs:53 and 54), was replaced with the glucagon peptide sequence, no glucagon activity was observed implying that the conformational context is important in positioning the charged amino acid so that it can contribute to generating agonist activity from the grafted peptide.

### EXAMPLE 8: CDR3 Optimization of HCAb Agonists

In order to improve the potency of the GLP-1 receptor agonists described above, V(D)J protein optimization as described in International Patent Application PCT/CA2013/050203 (WO 2013/134880) was applied to these HCAbs. Briefly, V(D)J substrates (a total of 15) were designed to target every other amino acid in CDR3 of the HCAb GLP1 agonists, V125, V126 and V130 (see Table 7, and Figure 20), and used to generate membrane-bound HCAb variant libraries. Cells were selected with increased affinity to the amino-terminus of the GLP-1 receptor (Figure 23A).

Several agonists with increased potency with mutations that clustered in the carboxy terminal region of the grafted peptide were identified. Three of these clones (ITS007-V206, -V209 and -V211; see Table 7 and Figure 24) were shown to have approximately a 3-4 fold increase in agonist activity to the receptor (Figure 23B), demonstrating that sequences outside of the amino terminus of the peptide derived sequences can modulate agonist activity. Interestingly, although each of the three sequences (V206, V209 and V211) was unique, they all shared a common amino acid change (see Figure 24). Two of the sequences (ITS007-V209 and ITS007-V211) shared two amino acid changes.

ITS007-V206, -V209 and -V211 were all generated in the context of the V125 amino terminal sequences. In a further experiment, these three different carboxy mutations were paired with V126 and V130 amino terminal sequences to provide to provide hybrid molecules ITS007-V241-V246 (Table 7, and Figure 28). All six of these peptide-grafted HCAbs were shown to have agonist activity.

In a separate experiment, V(D)J mutagenesis was applied and agonist activity selected for directly. In order to be able to identify antibodies with increased potency, these V(D)J substrates were engineered with a tetracycline inducible TK promoter driving HCAb expression. This allowed the HCAb to be expressed at low levels and modulated with the addition of Tetracycline (ITS007-V152 (tet); see Figure 27). In conditions with no tetracycline added to the culture, the agonist activity of cells constitutively expressing the V125 HCAb was below detection.

A V(D)J *de novo* generated library of approximately 10⁸ CDR3 variants targeting every other amino acid in CDR3 was magnetically enriched for CD19 positive cells and subsequently FACS sorted. Initial frequencies of CD19 positive cells were below detection (less than one in hundred thousand). Following magnetic enrichment via the CD19 surface marker of GLP-1 receptor activity, frequencies of approximately 0.5% were observed. Subsequent FACS sorts increased the frequency of CD19 positive cells to 14% representing cells expressing variants of ITS007-V125 with increased potency (Figure 25A).

**Table 7: Variant HCAb GLP1 Agonists**

| **ID** | **VH Gene** | **Sequence*** | **JH Gene** | **SEQ ID NO** |
|---|---|---|---|---|
| Original clone in HcAb format | | | | |
| ITS007-V125 | 3-9 | | 3 | 82 |

| V-exendin junction mutants | | | | |
|---|---|---|---|---|
| ITS007-V126 | 3-9 | | 3 | 83 |
| ITS007-V127 | 3-9 | | 3 | 84 |
| ITS007-V128 | 3-9 | | 3 | 85 |
| ITS007-V130 | 3-9 | | 3 | 86 |
| | | | | |
| ITS007-V212 | 3-9 | | 3 | 87 |

| C-terminal exendin mutants | | | | |
|---|---|---|---|---|
| ITS007-V206 | 3-9 | | 3 | 88 |
| ITS007-V209 | 3-9 | | 3 | 89 |
| ITS007-V211 | 3-9 | | 3 | 90 |

| Hybrid mutants^{†} | | | | |
|---|---|---|---|---|
| ITS007-V241 | 3-9 | | 3 | 91 |
| ITS007-V242 | 3-9 | | 3 | 92 |
| ITS007-V243 | 3-9 | | 3 | 93 |
| ITS007-V244 | 3-9 | | 3 | 94 |
| ITS007-V245 | 3-9 | | 3 | 95 |
| ITS007-V246 | 3-9 | | 3 | 96 |

| | | | | |
|---|---|---|---|---|
| * Peptide sequence in bold; charged ammo acid flanking terminal Histidine of peptide sequence in underlined bold italics; carboxy flanking sequences found between peptide and JH gene segment in italics; additional mutations of interest underlined. † based on: V241: ITS007-V126 / ITS007-V206; V242: ITS007-V126 / ITS007-V209; V243: ITS007-V126 / ITS007-V211; V244: ITS007-V130 / ITS007-V206; V245: ITS007-V130 / ITS007-V209; V246: ITS007-V130 / ITS007-V211. | | | | |

### EXAMPLE 9: CDR1 and CDR2 Optimization of HCAb Agonists

A V(D)J *de novo* generated library of approximately 10⁸ CDR1 and CDR2 variants was also generated. Initial frequencies of CD19 positive cells were again below detection (less than one in hundred thousand). Following magnetic enrichment via the CD19 surface marker (expressed following activation of GLP-1 receptor), frequencies of approximately 0.3% were observed. Subsequent FACS sorts increased the frequency of CD19 positive cells to approximately 4% for the CDR1 and CDR2 variant pools (Figure 25B). This latter result demonstrates the potential to increase the potency of HCAb agonists by mutagenesis outside of the peptide sequences and of CDR3.

### EXAMPLE 10: Increasing Potency of HCAbs by Addition of a VH Domain

In order to investigate whether the potency of the GLP-1 agonist HCAbs described above could be increased, a construct that placed the GLP-1R agonist V130 VH domain downstream of the CH3 sequences of the V130 HCAb was prepared (ITS007- V248, see Figure 29). The ITS007-V248 construct was generated by using PCR to amplify the VH domain and place it downstream of the CH3 domain of the ITS007-V130 HCAb. A schematic of this molecule, which contains a total of 4 VH agonist domains, is shown in Figure 22B. This format of HCAb is referred to herein as "VH²."

Transient transfections were performed and supernatant was generated and quantitated for IgG. A titration of the supernatants was performed assaying for agonist activity, and activity was compared to supernatants generated from the original V130 HCAb. As can be seen in Figure 26, not only is the VH² active but it appears to be approximately 4 times more potent than the original V130 HCAb. This increase represents more than just a doubling, which would be expected from the presence of the two additional VH domains.

This example not only confirms that the VH domain is modular, but also provides a method to enhance the potency of an existing HCAb or to generate molecules with multiple functionality. For example, placing a GLP-1 receptor agonist VH domain on a glucagon agonist HCAb could generate a dual agonist HCAb. Likewise, placing a VH domain downstream of a full antibody would allow the generation of a novel bi-specific antibody that could potentially target GLP-1 agonist activity if desired.

## Claims

1. A peptide-grafted antibody or antibody fragment comprising at least one peptide grafted into at least one CDR of the antibody or antibody fragment, the peptide comprising the sequence of exendin, GLP1, glucagon or oxyntomodulin peptide or a functional fragment thereof, wherein the peptide-grafted antibody has agonist activity against the GLP1 receptor, the glucagon receptor, or both, wherein the grafted peptide comprises a basic amino acid upstream and adjacent to the natural N-terminal histidine of the exendin, GLP1, glucagon or oxyntomodulin peptide, and wherein the basic amino acid is an arginine or a lysine.

2. The peptide-grafted antibody or antibody fragment according to claim 1, wherein the basic amino acid is within a heterologous flanking sequence of between one and about 20 amino acids at the N-terminus of the grafted peptide.

3. The peptide-grafted antibody or antibody fragment according to claim 1, wherein the basic amino acid is part of the antibody sequence N-terminal to the grafted peptide.

4. The peptide-grafted antibody or antibody fragment according to any one of claims 1 to 3, wherein the at least one CDR comprises a heavy chain CDR.

5. The peptide-grafted antibody or antibody fragment according to any one of claims 1 to 4, wherein the at least one CDR comprises a light chain CDR.

6. The peptide-grafted antibody or antibody fragment according to any one of claims 1 to 5, wherein the at least one CDR is a CDR3.

7. The peptide-grafted antibody or antibody fragment according to any one of claims 1 to 6, further comprising a heterologous flanking sequence of between one and about 20 amino acids at the C-terminus of the grafted peptide.

8. The peptide-grafted antibody or antibody fragment according to any one of claims 1 to 4, 6 and 7, wherein the antibody is a heavy-chain only antibody or the antibody fragment is a single VH domain antibody.

9. The peptide-grafted antibody according to claim 8, further comprising a peptide-grafted VH domain attached to a CH3 region of the peptide-grafted antibody, wherein the peptide comprised by the peptide-grafted VH domain comprises the sequence of exendin, GLP1, glucagon or oxyntomodulin peptide or a functional fragment thereof.

10. The peptide-grafted antibody according to claim 9, wherein the peptide comprised by the peptide-grafted VH domain and the peptide comprised by the peptide-grafted antibody target the same receptor or different receptors.

11. The peptide-grafted antibody or antibody fragment according to any one of claims 1 to 10, wherein the peptide comprised by the peptide-grafted antibody comprises the sequence of exendin or GLP1 peptide, or a functional fragment thereof, and the peptide-grafted antibody has agonist activity against GLP1 receptor.

12. The peptide-grafted antibody or antibody fragment according to claim 11, wherein the peptide-grafted antibody comprises the sequence as set forth in any one of SEQ ID NOs: 24, 26, 32, 33 51, 54, 56, 58, 60, 62, 64, 66, 68, 71, 73, 75, 77, 79, 81 and 82 to 96.

13. The peptide-grafted antibody or antibody fragment according to any one of claims 1 to 10, wherein the peptide comprised by the peptide-grafted antibody comprises the sequence of glucagon or oxyntomodulin peptide, or a functional fragment thereof, and the peptide-grafted antibody has agonist activity against glucagon receptor.

14. The peptide-grafted antibody or antibody fragment according to claim 13, wherein the peptide-grafted antibody comprises the sequence as set forth in any one of SEQ ID NOs: 37 to 42, 98, 100, 102, 104, 106, 108, 110 and 112 to 114.

15. A polynucleotide encoding the peptide-grafted antibody or antibody fragment according to any one of claims 1 to 14.

## Patentansprüche

1. Ein Peptid-transplantierter Antikörper oder Antikörper-Fragment, umfassend mindestens ein Peptid, transplantiert in mindestens eine CDR des Antikörpers oder Antikörperfragments, wobei das Peptid die Sequenz von Exendin, GLP1, Glucagon oder Oxyntomodulin-Peptid oder ein funktionelles Fragment davon umfasst, wobei der Peptid-transplantierte Antikörper agonistische Aktivität am GLP1-Rezeptor, am Glucagon-Rezeptor oder beiden besitzt, wobei das transplantierte Peptid eine basische Aminosäure oberhalb oder neben dem natürlichen N-terminalen Histidin von Exendin, GLP1, Glucagon oder Oxyntomodulin-Peptid umfasst und wobei die basische Aminosäure ein Arginin oder ein Lysin ist.

2. Der Peptid-transplantierte Antikörper oder das Antikörper-Fragment gemäß Anspruch 1, wobei die basische Aminosäure sich innerhalb einer heterologen flankierenden Sequenz mit zwischen einer und etwa 20 Aminosäuren am N-Terminus des transplantierten Peptids befindet.

3. Der Peptid-transplantierte Antikörper oder das Antikörper-Fragment gemäß Anspruch 1, wobei die basische Aminosäure Teil der Antikörper-Sequenz N-terminal vom transplantierten Peptid ist.

4. Der Peptid-transplantierte Antikörper oder das Antikörper-Fragment gemäß einem der Ansprüche 1 bis 3, wobei die mindestens eine CDR eine CDR der schweren Kette umfasst.

5. Der Peptid-transplantierte Antikörper oder das Antikörper-Fragment gemäß einem der Ansprüche 1 bis 4, wobei die mindestens eine CDR eine CDR der leichten Kette umfasst.

6. Der Peptid-transplantierte Antikörper oder das Antikörper-Fragment gemäß einem der Ansprüche 1 bis 5, wobei die mindestens eine CDR eine CDR3 ist.

7. Der Peptid-transplantierte Antikörper oder das Antikörper-Fragment gemäß einem der Ansprüche 1 bis 6, außerdem umfassend eine heterologe flankierende Sequenz mit zwischen einer und etwa 20 Aminosäuren am C-Terminus des transplantierten Peptids.

8. Der Peptid-transplantierte Antikörper oder das Antikörper-Fragment gemäß einem der Ansprüche 1 bis 4, 6 und 7, wobei der Antikörper ein Schwere-Kette-Antikörper ist oder das Antikörper-Fragment ein Einzel-VH-Domänen-Antikörper ist.

9. Der Peptid-transplantierte Antikörper gemäß Anspruch 8, außerdem umfassend eine Peptid-transplantierte VH-Domäne, angehängt an eine CH3-Region des Peptid-transplantierten Antikörpers, wobei das Peptid, das in der Peptid-transplantierten VH-Domäne enthalten ist, die Sequenz von Exendin, GLP1, Glucagon oder Oxyntomodulin-Peptid oder ein funktionelles Fragment davon umfasst.

10. Der Peptid-transplantierte Antikörper gemäß Anspruch 9, wobei das Peptid, das in der Peptid-transplantierten VH-Domäne enthalten ist, und das Peptid, das im Peptid-transplantierten Antikörper enthalten ist, den gleichen Rezeptor oder verschiedenen Rezeptoren targeten.

11. Der Peptid-transplantierte Antikörper oder das Antikörper-Fragment gemäß einem der Ansprüche 1 bis 10, wobei das Peptid, das in dem Peptid-transplantierten Antikörper enthalten ist, die Sequenz von Exendin oder GLP1-Peptid, oder einem funktionellen Fragment davon, umfasst und der Peptid-transplantierte Antikörper agonistische Aktivität am GLP1-Rezeptor besitzt.

12. Der Peptid-transplantierte Antikörper oder das Antikörper-Fragment gemäß Anspruch 11, wobei der Peptid-transplantierte Antikörper die Sequenz umfasst, wie sie in einem der SEQ ID NOs: 24, 26, 32, 33, 51, 54, 56, 58, 60, 62, 64, 66, 68, 71, 73, 75, 77, 79, 81 und 82 bis 96 dargestellt ist.

13. Der Peptid-transplantierte Antikörper oder das Antikörper-Fragment gemäß einem der Ansprüche 1 bis 10, wobei das Peptid, das in dem Peptid-transplantierten Antikörper enthalten ist, die Sequenz von Glucagon oder Oxyntomodulin-Peptid, oder einem funktionellen Fragment davon, umfasst und der Peptid-transplantierte Antikörper agonistische Aktivität am Glucagon-Rezeptor besitzt.

14. Der Peptid-transplantierte Antikörper oder das Antikörper-Fragment gemäß Anspruch 13, wobei der Peptid-transplantierte Antikörper die Sequenz umfasst, wie sie in einem der SEQ ID NOs: 37 bis 42, 98, 100, 102, 104, 106, 108, 110 und 112 bis 114 dargestellt ist.

15. Ein Polynukleotid, das den Peptid-transplantierten Antikörper oder das Antikörper-Fragment gemäß einem der Ansprüche 1 bis 14 kodiert.

## Revendications

1. Anticorps ou fragment d'anticorps greffé à un peptide comprenant au moins un peptide greffé dans au moins une CDR de l'anticorps ou du fragment d'anticorps, le peptide comprenant la séquence d'un peptide exendine, GLP1, glucagon ou oxyntomoduline ou un fragment fonctionnel de celui-ci, dans lequel l'anticorps greffé à un peptide présente une activité agoniste contre le récepteur de GLP1, le récepteur du glucagon, ou les deux, dans lequel le peptide greffé comprend un acide aminé basique en amont et adjacent à l'histidine N-terminale naturelle du peptide exendine, GLP1, glucagon ou oxyntomoduline, et dans lequel l'acide aminé basique est une arginine ou une lysine.

2. Anticorps ou fragment d'anticorps greffé à un peptide selon la revendication 1, dans lequel l'acide aminé basique est à l'intérieur d'une séquence flanquante hétérologue d'un à environ 20 acides aminés à l'extrémité N-terminale du peptide greffé.

3. Anticorps ou fragment d'anticorps greffé à un peptide selon la revendication 1, dans lequel l'acide aminé basique fait partie de la séquence d'anticorps N-terminale par rapport au peptide greffé.

4. Anticorps ou fragment d'anticorps greffé à un peptide selon l'une quelconque des revendications 1 à 3, dans lequel l'au moins une CDR comprend une CDR de chaîne lourde.

5. Anticorps ou fragment d'anticorps greffé à un peptide selon l'une quelconque des revendications 1 à 4, dans lequel l'au moins une CDR comprend une CDR de chaîne légère.

6. Anticorps ou fragment d'anticorps greffé à un peptide selon l'une quelconque des revendications 1 à 5, dans lequel l'au moins une CDR est une CDR3.

7. Anticorps ou fragment d'anticorps greffé à un peptide selon l'une quelconque des revendications 1 à 6, comprenant en outre une séquence flanquante hétérologue d'un à environ 20 acides aminés à l'extrémité C-terminale du peptide greffé.

8. Anticorps ou fragment d'anticorps greffé à un peptide selon l'une quelconque des revendications 1 à 4, 6 et 7, dans lequel l'anticorps est un anticorps uniquement à chaîne lourde ou le fragment d'anticorps est un anticorps à domaine VH unique.

9. Anticorps greffé à un peptide selon la revendication 8, comprenant en outre un domaine VH greffé à un peptide fixé à une région CH3 de l'anticorps greffé à un peptide, dans lequel le peptide compris par le domaine VH greffé à un peptide comprend la séquence de peptide exendine, GLP1, glucagon ou oxyntomoduline ou un fragment fonctionnel de celui-ci.

10. Anticorps greffé à un peptide selon la revendication 9, dans lequel le peptide compris par le domaine VH greffé à un peptide et le peptide compris par l'anticorps greffé à un peptide ciblent le même récepteur ou des récepteurs différents.

11. Anticorps ou fragment d'anticorps greffé à un peptide selon l'une quelconque des revendications 1 à 10, dans lequel le peptide compris par l'anticorps greffé à un peptide comprend la séquence de peptide exendine ou GLP1, ou un fragment fonctionnel de celui-ci, et l'anticorps greffé à un peptide présente une activité agoniste contre le récepteur de GLP1.

12. Anticorps ou fragment d'anticorps greffé à un peptide selon la revendication 11, dans lequel l'anticorps greffé à un peptide comprend la séquence telle que représentée par l'une quelconque parmi SEQ ID NO : 24, 26, 32, 33, 51, 54, 56, 58, 60, 62, 64, 66, 68, 71, 73, 75, 77, 79, 81 et 82 à 96.

13. Anticorps ou fragment d'anticorps greffé à un peptide selon l'une quelconque des revendications 1 à 10, dans lequel le peptide compris par l'anticorps greffé à un peptide comprend la séquence de peptide glucagon ou oxyntomoduline, ou un fragment fonctionnel de celui-ci, et l'anticorps greffé à un peptide présente une activité agoniste contre le récepteur du glucagon.

14. Anticorps ou fragment d'anticorps greffé à un peptide selon la revendication 13, dans lequel l'anticorps greffé à un peptide comprend la séquence telle que représentée par l'une quelconque parmi SEQ ID NO : 37 à 42, 98, 100, 102, 104, 106, 108, 110 et 112 à 114.

15. Polynucléotide codant pour l'anticorps ou fragment d'anticorps greffé à un peptide selon l'une quelconque des revendications 1 à 14.
